# EUROPEAN PATENT APPLICATION

(11) **EP 2 583 678 A2**
(43) Date of publication of application: **24.04.2013**
(21) Application number: 10179796.7
(22) Date of filing: 24.06.2005
(51) Int. Cl.: A61K 31/404, A61K 31/4184, A61K 31/47, A61K 31/517, A61P 31/12, A61P 35/00, A61P 37/08, A61P 11/06, A61K 31/34, A61K 31/341, A61K 31/7056, A61K 31/7076, A61K 45/06

(54) **Small molecule immunopotentiators and assays for their detection**

(30) Priority: 24.06.2004 US 582654 P; 22.07.2004 US 590459 P; 05.08.2004 US 599592 P; 05.08.2004 US 599717 P; 11.08.2004 US 600850 P; 19.08.2004 US 603001 P; 23.08.2004 US 603867 P; 21.09.2004 US 612070 P; 21.09.2004 US 614963 P
(62) Divisional of application: 05857800.6
(71) Applicant: Novartis Vaccines and Diagnostics, Inc., Emeryville, CA 94608-2916 (US)
(72) Inventor: Valiante, Nicholas, Emeryville, CA 94608-2916 (US); Xu, Feng, Emeryville, CA 94608-2916 (US); Jansen, Johanna, Emeryville, CA 94608-2916 (US); Kaufman, Susan, Emeryville, CA 94608-2916 (US)
(74) Representative: Marshall, Cameron John

(57) **Abstract**

The invention provides immunostimulatory compositions comprising a small molecule immuno-potentiator (SMIP) compound and methods of administration thereof. Also provided are methods of administering a SMIP compound in an effective amount to enhance the immune response of a subject to an antigen. Further provided are novel compositions and methods of administering SMIP compounds alone or in combination with another agent for the treatment of cancer, infectious diseases and/or allergies/asthma. In a further aspect, the invention relates generally to methods of screening for small molecule immuno-modulatory compositions.

## Description

### FIELD OF THE INVENTION

In one embodiment, this invention relates generally to compounds capable of stimulating or modulating an immune response in a subject. More particularly the invention pertains to novel combinations of antigens with small molecules to be used in vaccine therapies. The compounds in one embodiment can be used as adjuvants for prophylactic and therapeutic vaccines for infectious diseases and cancer. In another embodiment they can be used as immunotherapeutics for cancer, infectious diseases and/or allergy/asthma either alone or in combination with existing therapies. In a further embodiment, the invention pertains generally to methods of identifying small molecule immunomodulators, and, more particularly, to high throughput assays for detection of immunopotentiators and immunosuppressants by measurement of immunological markers, such as cytokines, chemokines, and/or growth factors.

### BACKGROUND OF THE INVENTION

Immune response to certain antigens that are otherwise weakly antigenic can be enhanced through the use of vaccine adjuvants. Such adjuvants potentiate the immune response to specific antigens and are therefore the subject of considerable interest and study within the medical community.

Research has permitted development of vaccines possessing antigenic epitopes that were previously impossible to produce. For example, currently available vaccine candidates include synthetic peptides mimicking streptococcal, gonococcal, and malarial antigens. These purified antigens are generally weak antigens, however, that require adjuvants in order to evoke protective immunity. However, conventional vaccine adjuvants possess a number of drawbacks that limit their overall use and effectiveness.
Again, this is fine for vaccines but not other uses.

Substances that stimulate immune cells *in vitro* exhibit similar immunostimulatory effects *in vivo.* These compounds, such as recombinant cytokines, pathogen products (e.g. toxins, lipids, proteins/peptides, carbohydrates and nucleic acids) and other mammalian-derived immunostimulatory molecules (e.g. heat shock proteins, complement, immune complexes and proteoglycans) all induce a measurable pro-inflammatory response both *in vitro* and *in vivo.*

Historically, the classic adjuvants have been Freund's complete or incomplete (i.e., without mycobacteria) adjuvants. Edmund Coley described the potential of Coley's toxin for cancer immuno-therapy. Other materials, such as mineral oil and aluminum hydroxide, have also been used as adjuvants, but they invariably suffer from disadvantages. For example, mineral oil is known to produce tissue irritation and to be potentially oncogenic. Alum, the only approved adjuvant in the United States, can induce granulomas at the inoculation site and does not effectively induce cell-mediated immunity. Moreover, many of the adjuvants currently available have limited utility because they contain components, which are not metabolizable in humans. Additionally, most adjuvants are difficult to prepare in that they may require time consuming procedures and the use, in some cases, of elaborate and expensive equipment to formulate a vaccine and adjuvant system.

Immunological adjuvants are described in "Current Status of Immunological Adjuvants", Ann. Rev. Immunol., 1986, 4, pp. 369-388, and "Recent Advances in Vaccine Adjuvants and Delivery Systems" by Derek T O'Hagan and Nicholas M. Valiente. See also U.S. Pat. Nos. 4,806,352; 5,026,543; and 5,026,546 for disclosures of various vaccine adjuvants appearing in the patent literature.

Compounds are described in issued U.S. Patent Nos. 4,547,511 and 4,738,971 with the general structure (a): 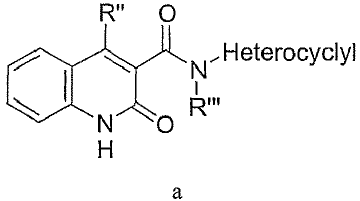 for the treatment of disorders responsive to agents that enhance cell-mediated immunity. An essential component of the molecule as described in the cited patents is the amide substituent as shown in structure (a). The invention did not contemplate combinations with antigens.

Immunostimulatory oligonucleotides and polynucleotides are described in PCT WO 98/55495 and PCT WO 98/16247. U.S. Patent Application No. 2002/0164341 describes adjuvants including an unmethylated CpG dinucleotide (CpG ODN) and a non-nucleic acid adjuvant. U.S. Patent Application No. 2002/0197269 describes compositions comprising an antigen, an antigenic CpG-ODN and a polycationic polymer.

Additionally, issued U.S. Patent Nos. 4,689,338, 5,389,640, 5,268,376, 4,929,624, 5,266,575, 5,352,784, 5,494,916, 5,482,936, 5,346,905, 5,395,937, 5,238,944, 5,525,612, WO99/29693 and U.S. Ser. No. 09/361,544 disclose compounds of the general structure (b): 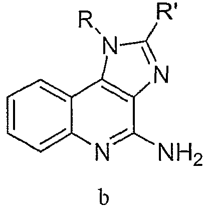 for the use as "immune response modifiers."

There has been an effort to find new immune modulators for use as adjuvants for vaccines and immunotherapies that would overcome the drawbacks and deficiencies of conventional immune modulators. In particular, an adjuvant formulation that elicits potent cell-mediated and humoral immune responses to a wide range of antigens in humans and domestic animals, but lacking the side effects of conventional adjuvants and other immune modulators, would be highly desirable. This need could be met by small molecule immune potentiators (SMIPs) because the small molecule platform provides diverse compounds for the selective manipulation of the immune responses.

Furthermore, it would be desirable to provide novel compounds with a varied capacity to alter levels and/or profiles of cytokine production in human immune cells. Compounds with structural disparities will often times elicit a desired response through a different mechanism of action, or with greater specificity to a target, such as a dendritic cell, modulating potency and lowering side effects when administered to a patient.

The immunosuppressive effect of cytostatic substances has rendered them useful in the therapy of autoimmune diseases such as multiple sclerosis, psoriasis and certain rheumatic diseases. Even here their beneficial effect has to be weighed against the serious side effects that necessitate too low dosages and/or interruption of the treatment.

To date immune suppressants have also been of great interest due to their ability to decrease immune responses in surgical operations and transplants, when a full-blown immune attack or rejection of a transplant organ could prove fatal.

It is one object of the present invention to provide a combination of active substances that results in a significantly improved cytostatic or cytotoxic effect as compared to conventional cytostatics given alone, e.g. vincristin, methotrexate, cisplatin etc. Thereby, chemotherapies may be offered that combine increasing efficiency with a large reduction of side effects and therapeutic doses. Thus, the therapeutic efficiency of known cytostatic drugs is increased. Also, certain cell lines that are insensitive to chemotherapeutic treatment may become susceptible to chemotherapy by applying the combination of active substances.

Therapeutics that could serve to augment natural host defenses against viral and bacterial infections, or against tumor induction and progression, with reduced cytotoxicity would be very beneficial. The present invention provides such therapeutic agents, and further provides other related advantages.

The small molecule platform provides diverse compounds for the selective manipulation of the immune response, necessary for increasing the therapeutic index of a drug. Compounds with structural disparities will often times elicit a desired response through a different mechanism of action, or with greater specificity to a target, such as a dendritic cell, modulating potency and lowering side effects when administered to a patient.

It would therefore be beneficial to provide an assay for rapidly screening large quantities of small molecule compounds, which may have immunomodulatory activity. It is an object of the instant invention to provide a high throughput screen for detecting small molecules capable of modulating an immune response. It is also an object of the invention to provide methods of producing large libraries of compounds, through solid support resins, capable of being screened in the high throughput assays of the invention.

All of the aforementioned documents are incorporated by reference as if fully set forth herein.

### BRIEF DESCRIPTION OF THE TABLES

Table 1 lists *in vitro* inhibitory activity of TNF- *α* for 1*H*-imidazoquinolines

Table 2 identifies potent SMIS compounds and analogs thereof, which have been shown to be SMIPs.

Table 3 lists SMIS analogs, which are SMIPs.

Table 4 lists preferred SMIS compounds and their corresponding references, which are capable of modulating an immune response in a patient and being modified to elicit an immune response in a patient.

Table 5 lists chemokine species which may serve as immunological markers for identification of immunomodulation.

Table 6 lists interleukin species which may serve as immunological markers for identification of immunomodulation.

### SUMMARY OF THE INVENTION

The instant invention provides novel immune potentiators, immunogenic compositions, novel compounds and pharmaceutical compositions, and novel methods of administering a vaccine, by administering small molecule immune potentiators in combination with antigens. The invention further provides compositions, novel compounds and pharmaceutical compositions, for use in the treatment of cancer, infectious diseases, allergies, and asthma.

The SMIP compounds used in the methods and compositions of the invention are inexpensive to produce and easy to administer. They have potential for finer specificity compared to existing immunostimulants, thus providing improved efficacy and safety profiles.

As adjuvants, the SMIP compounds are combined with numerous antigens and delivery systems to form a final vaccine product.

As immuno-therapeutics, the SMIP compounds are used alone or in combination with other therapies (e.g. anti-virals, anti-bacterials, other immune modulators or in therapeutic vaccine antigens) for treatment of chronic infections such as HIV, HCV, HBV, HSV, and H. pylori, as well as medicaments for the reduction of tumor growth.

As immunotherapeutics, the SMIP compounds also may be used for the treatment of cancer either alone or in combination with other anti-cancer therapies (e.g. chemotherapeutic agents, mAbs or other immune potentiators). In addition, certain SMIPs with the capacity to induce Type 1 cytokines (e.g. IL-12, TNF or IFN's) could be useful for the treatment of allergies or asthma due to their capacity to steer the immune response towards more benign sequelae. The SMIP compounds may be used for example for the treatment of BCG, cholera, plague, typhoid, hepatitis B infection, influenza, inactivated polio, rabies, measles, mumps, rubella, oral polio, yellow fever, tetanus, diphtheria, hemophilus influenzae b, meningococcus infection, and pneumococcus infection. The SMIP compounds may be used in an anti cell proliferative effective amount for the treatment of cancer. The SMIP compounds may also be used in anti-Th2/Type2 cytokine amount for the deviation of allergic/asthmatic immune responses.

In another embodiment methods of treating cancer are provided wherein known anticancer agents are combined with SMIP compounds to reduce tumor, growth in a subject. A number of suitable anticancer agents are contemplated for use in the methods of the present invention and are described more thoroughly in the detailed description.

In accordance with the present invention, there is provided a method of inhibiting tumor cell growth in a subject, which method comprises administering to said subject an effective dose of a combination containing at least a SMIP and a MAb, wherein said combination is more effective to inhibit such cell growth than when said MAb is administered individually. Further provided are methods of treating cancer with said combination comprising an additional SMIP compound or MAb, as well as methods of administration to a subject in need thereof.

Additional embodiments, methods and compositions contemplated to be useful in the instant invention are disclosed in USSN 10/814,480, 10/762,873, and 10/748,071 which are incorporated by reference as if set forth fully herein.

In a further embodiment, the invention provides a high throughput assay for identifying small molecule immunomodulators, said assay comprising:
a) contacting a plurality of test compounds with cells to form one or more test solution(s);
b) incubating said test solution for at least 30 minutes;
c) measuring for an increased level of one or more immunological markers in said test solution;
wherein immunomodulation by one or more test compounds present in said plurality of test compounds causes an increase in the amount of said immunological markers in said test solution.

Another embodiment of the invention provides a high throughput assay for identifying small molecule immunomodulators, said assay comprising:
a) contacting a plurality of test compounds with cells to form one or more test solution(s);
b) adding a known immunopotentiating agent to said test solution and to a control solution devoid of said test compounds;
c) incubating said test solution and said control solution for at least 1 hour;
d) measuring levels of one or more immunological markers in said test solution and said control solution;
e) comparing said levels of one or more immunological markers in said test solution with corresponding levels of one or more immunological markers in said control solution to detect a difference in the levels of one or more
immunological markers between said test solution and said control solution; wherein a decrease in the level of one or more of the immunological markers in the test solution indicates immunosuppression by one or more of the test compounds and an increase in the level of one or more of the immunological markers in the test solution indicates immunopotentiation by one or more of the test compounds.

Another embodiment of the invention provides a high throughput assay for identifying a small molecule immunomodulator, said method comprising:
a) providing a mixture of test compounds divided into an archive portion and a screening portion from a combinatorial library, wherein (i) said mixture comprises a plurality of resin support beads having test compounds attached thereto, and (ii) each said bead has only one discrete test compound at a concentration greater than 1 nmol attached thereto;
b) contacting said screening portion with cells;
c) measuring for increased levels of immunological markers in said screening portion, wherein immunomodulation by one or more test compounds present in said screening portion causes an increase in the amount of said immunological markers;
d) individually distributing beads from the archive portion in step (a) into a plurality of reaction vessels such that each vessel contains a single bead;
e) cleaving the test compounds from the beads and separating said beads from the cleaved test compounds, thereby providing discrete samples of individual test compounds;
f) screening each cleaved test compound for identification of a small molecule immunomodulator;
g) performing a mass spectrometric analysis on said small molecule immunomodulator; and
h) deriving the chemical identity of said small molecule immunomodulator from said reserved portion using said mass spectrometric analysis.

In another embodiment of the invention, a method is provided for screening components of a combinatorial library for immunological activity, and then performing a nonsynthetic deconvolution to identify and characterize specific components from the library.

Further embodiments of the invention include those described in the detailed description.

### DETAILED DESCRIPTION

Applicants have discovered compounds capable of stimulating cytokine activity in cells and immunotherapeutics and/or vaccine adjuvants in combination with an antigen(s), that will provide effective treatments for disorders described herein and those apparent to one skilled in the art.

For each of the following embodiments, a method of eliciting an immune response in a patient can be replaced with: use of a SMIP in the manufacture of a medicament for eliciting an immune response in a patient. In other embodiments the SMIPs are used in the manufacture of a medicament for treating an infectious disease, autoimmune disease, allergies, or cancer. In other embodiments the SMIP s are used in the manufacture of a medicament for use as an adjuvant. SMIPs of the present invention include those described in formula I-L.

One embodiment of the invention provides a method of eliciting an immune response in a patient comprising administering a SMIP of formula (I): 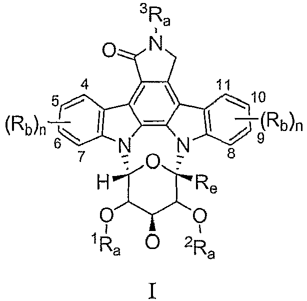 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstihited aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstitute heteroaralkenyl, substituted and unsubstituted heterocyclylalkenyl, and substituted and unsubstituted carbocyclylalkenyl; R_{c} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
n is 1, 2, or 3.

In a more particular embodiment of formula I, Rₐ at position 1 is H. In a more particular embodiment of formula I, Rₐ at position 2 is substituted or unsubstituted alkyl. In a more particular embodiment of formula I, R_{b} is H. In a more particular embodiment of formula 1 wherein Rₐ at position 3 is H. In a more particular embodiment of formula I, Rₑ is substituted or unsubstituted alkyl.

Another embodiment of the invention provides a method of eliciting an immune response in a patient comprising administering a SMIP of formula (II): 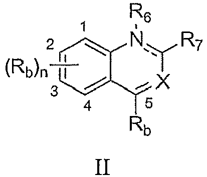 wherein,
a dotted line represents an optional placement of an additional bond;
R₆ is H, substituted alkyl or unsubstituted alkyl; R₇ is =O; and the dotted line is absent; or
R₆ is absent; R₇ is selected from the group consisting of H, hydroxy, amino, nitro, cyano, carboxylic acid, substituted and unsubstituted alkyl, substituted and unsubstituted alkenyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, and substituted and unsubstituted carbocyclylalkyl; and the dotted line represents the placement of an additional bond;
X is =N- or =C(R₈)-, wherein R₈ is selected from the group consisting of H, hydroxy, amino, nitro, cyano, carboxylic acid, substituted and unsubstituted alkyl, substituted and unsubstituted alkenyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, and substituted and unsubstituted carbocyclylalkyl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and n is 1, 2, or 3.

In another embodiment, if R₇ is =O and R₈ is a heterocyclyl, it is not a substituted benzimidazole. In a more particular embodiment of formula II, Examples 42, 43, and 44 are excluded.

In a more particular embodiment of formula II, R_{b} is H, halogen, or unsubstituted alkoxy. In a more particular embodiment of formula II, R₆ is H; R₇ is =O, and the dotted line is absent. In a more particular embodiment still, R₆ is H; R₇ is =O, the dotted line is absent; X is CR₈ and R₈ is further defined as a structure of formula (R₈ₐ): 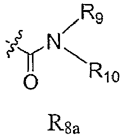 wherein, R₉ and R₁₀ are independently selected from the group consisting of H, substituted and unsubstituted alkyl, substituted and unsubstituted aryl, substituted and unsubstituted heterocyclyl and substituted and unsubstitute heteroaryl.

In a more particular embodiment of formula II, R₆ is absent, R₇ is selected from the group consisting of H, hydroxy, amino, nitro, cyano, carboxylic acid, substituted and unsubstituted alkenyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, and substituted and unsubstituted carbocyclylalkyl; and the dotted line is present. In a more particular embodiment thereof, R₇ is selected from the group consisting of substituted and unsubstituted heterocyclyl, substituted and unsubstituted aryl, substituted and unsubstituted heteroaryl, substituted and unsubstituted aralkyl, substituted and unsubstituted heteroaralkyl, and substituted and unsubstituted heterocyclylalkyl. In a still more particular embodiment of formula said aryl group of R₇ is more particularly defined as a fused arylaryl or unfused arylaryl and said heteroaryl group of R₇ is more particularly defined as a fused heteroarylaryl, fused heteroarylheteroaryl, unfused heteroarylaryl, or unfused heteroarylheteroaryl. In a more particular embodiment X is N and R_{b} at position 5 is a substituted arylamino, group.

Another embodiment of the invention provides a method of eliciting an immune response in a patient comprising administering a SMIP of formula (III): 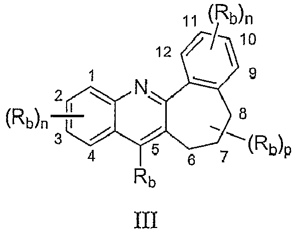 wherein,
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
n is independently 1, 2, or 3; and
p is 1 or 2.

Another embodiment of the invention provides a method of eliciting an immune response in a patient comprising administering a SMIP of formula (IV): 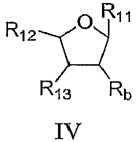 wherein,
R₁₁ is selected from the group consisting of hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted hydroxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted arylamino, substituted and unsubstituted heteroalylamino, substituted and unsubstituted heterocyclylamino, and substituted and unsubstituted carbocyclylamino;
R₁₂ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
R₁₃ is selected from the group consisting of H, hydroxyl, alkoxy, substituted and unsubstituted alkyl, substituted and unsubstituted aryl, substituted and unsubstituted heterocyclyl and substituted and unsubstituted heteroaryl; or R₁₂ and R₁₃ are bound together to form a substituted or unsubstituted heterocyclyl group; and
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula IV, R₁₂ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl; R₁₃ is selected from the group consisting of H, substituted and unsubstituted alkyl, substituted and unsubstituted aryl, substituted and unsubstituted heterocyclyl and substituted and unsubstituted heteroaryl; and R₁₁ is further defined as structure of formula (R₁₁ₐ): 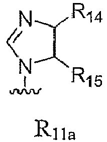 wherein,
R₁₄ and R₁₅ are independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; or R₁₄ and R₁₅ are taken together to form a substituted or unsubstituted heteroaryl group.

In a more particular embodiment of formula R₁₁ₐ, R₁₄ and R₁₅ are taken together to form a substituted heteroaryl group such that R₁₁ₐ is a DNA base. In a more particular embodiment said DNA base is adenine. In a more particular embodiment of formula R₁₁ₐ, R₁₄ is aminocarbonyl. In a more particular embodiment of formula R₁₁ₐ, R₁₄ is hydroxy.

In a more particular embodiment of formula IV, R₁₂ and R₁₃ are bound together to form a heterocyclyl group as shown in Figure (IVₐ): 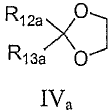 wherein,
R₁₂ₐ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
R₁₃ₐ is selected from the group consisting of H, substituted and unsubstituted alkyl, substituted and unsubstituted aryl, substituted and unsubstituted heterocyclyl and substituted and unsubstituted heteroaryl.

Another embodiment of the invention provides a method of eliciting an immune response in a patient comprising administering a SMIP of formula (V): 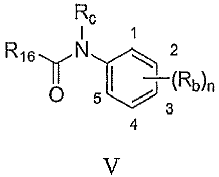 wherein,
R₁₆ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{c} is selected from the group consisting of H, substituted and unsubstituted alkyl, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstitute heterocyclylalkenyl, and substituted and unsubstituted carbocyclylalkenyl; and n is 1, 2, or 3.

In a more particular embodiment of formula V, Example 57 is excluded.

In a more particular embodiment of formula V, n is 1 and R_{b} is triflouromethyl.

In a more particular embodiment of formula V, n is 1 and R_{b} is cyano.

In a more particular embodiment of formula V, R₁₆ is further defined as a structure of formula R₁₆ₐ: 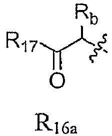 wherein,
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstittited aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R₁₇ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

Another embodiment of the invention provides a method of eliciting an immune response in a patient comprising administering a SMIP of formula (VI): 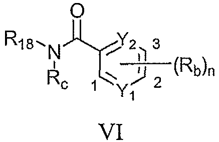 wherein,
Y₁ and Y₂ are independently N or CR_{b};
R₁₈ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{c} is selected from the group consisting of H, substituted and unsubstituted alkyl, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and n is 1, 2, or 3.

In a more particular embodiment of formula VI, Examples 2-11 and 59 are excluded.

In a more particular embodiment of formula VI, at least one of Y₁ and Y₂ is N.

In a more particular embodiment of formula VI, Y₁ and Y₂ are both N.

In a more particular embodiment of formula VI, R₁₈ is selected from the group consisting of substituted and unsubstituted aryl, substituted and unsubstituted heteroaryl, substituted and unsubstituted carbocyclyl, and substituted and unsubstituted heterocyclyl.

Another embodiment of the invention provides a method of eliciting an immune response in a patient comprising administering a steroid SMIP to a patient in need thereof. In a more particular embodiment, said steroid SMIP is more particularly defined as a structure of formula (VII): 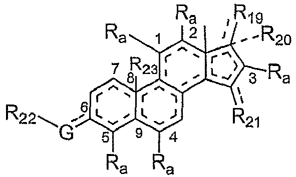 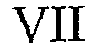 wherein,
a dotted line represents an optional placement of an additional bond with the proviso that two contiguous bonds are not double bonds;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R₁₉ is connected by a single bond and is selected from the group consisting of H, halogen, hydroxy, amino, nitro, cyano, carboxylic acid, substituted and unsubstituted alkyl, substituted and unsubstituted alkylthiol, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R₂₀ is selected from the group consisting of H, hydroxy, cyano nitro, carboxylic acid, halogen, amino, substituted and unsubstituted alkyl, substituted and unsubstituted alkylthiol, substituted and unsubstituted alkylamino, substituted and unsubstituted alkylcarbonyl, and substituted and unsubstituted alkoxy; or
R₁₉ is =O and R₂₀ is absent;
R₂₁ is connected by a single bond and is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted, and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; or
R₂₁ is =O;
G is O- and R₈ is selected from the group consisting of H, substituted and unsubstituted alkyl, substituted and unsubstituted aryl, and substituted and unsubstituted heteroaryl; or
G is O= and R₈ is absent; and
R₂₃ is H or substituted or unsubstituted alkyl; or
a double bond connects position 8 and 9 and R₂₃ is absent.

In another embodiment, Rₐ at position 3 is not hydroxy. In a more particular embodiment of formula VII, Example 62 is excluded.

In a more particular embodiment said steroid SMIP is defined as a structure of formula (VIII): 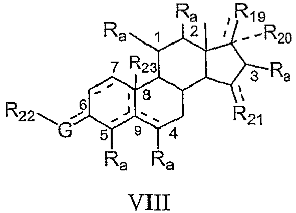 wherein,
a dotted line represents an optional placement of an additional bond with the proviso that two contiguous bonds are not double bonds;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R₁₉ is connected by a single bond and is selected from the group consisting of H, halogen, hydroxy, amino, nitro, cyano, carboxylic acid, substituted and unsubstituted alkyl, substituted and unsubstituted alkylthiol, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R₂₀ is selected from the group consisting of H, hydroxy, cyano nitro, carboxylic acid, halogen, amino, substituted and unsubstituted alkyl, substituted and unsubstituted alkylthiol, substituted and unsubstituted alhylamino, substituted and unsubstituted alkylcarbonyl, and substituted and unsubstituted alkoxy; or
R₁₉ is =O and R₂₀ is absent;
R₂₁ is connected by a single bond and is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; or R₂₁ is =O;
G is O- and R₈ is selected from the group consisting of H, substituted and unsubstituted alkyl, substituted and unsubstituted aryl, and substituted and unsubstituted heteroaryl; or
G is O= and R₂₂ is absent; and
R₂₃ is H or substituted or unsubstituted alkyl; or
a double bond connects position 8 and 9 and R₂₃ is absent.

In another embodiment, Rₐ at position 3 is not hydroxy. In a more particular embodiment of formula VIII, Example 62 is excluded.

Another embodiment of the invention provides a method of eliciting an immune response in a patient comprising administering a SMIP of formula (IX): 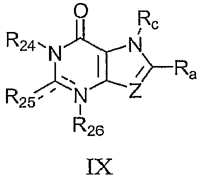 wherein,
a dotted line represents an optional placement of an additional bond with the proviso that two contiguous bonds are not double bonds;
Z is CR_{b} or N;
R₂₄ is selected from the group consisting of H, substituted and unsubstituted alkyl, aryl, substituted and unsubstituted heterocyclyl, substituted and unsubstituted heteroaryl, and substituted and unsubstituted alkyl-amino-alkyl; R₂₅ is O= or bound by a single bond and selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl;
R₂₆ is absent or selected from the group consisting of H, carboxylic acid, substituted and unsubstituted alkyl, substituted and unsubstituted alkenyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl;
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl, with the proviso that Rₐ at position 3 is not hydroxy;
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carb onyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R_{c} is selected from the group consisting of H, substituted and unsubstituted alkyl, substituted and unsubstituted aryl, substituted and unsubstituted heterocyclyl, and substituted and unsubstituted heteroaryl.

Another embodiment of the invention provides a method of eliciting an immune response in a patient comprising administering a SMIP of formula (XI): 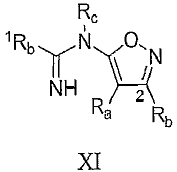 wherein,
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and R_{c} is selected from the group consisting of H, substituted and unsubstituted alkyl, substituted and unsubstituted aryl, substituted and unsubstituted heterocyclyl, and substituted and unsubstituted heteroaryl.

In a more particular embodiment of formula XI, R_{b} at position 1 is a nitrogen-containing heterocyclyl group selected from the group consisting of piperizine, morpholine, homomorpholine, piperidine, and pyrrolidine.

In a more particular embodiment of formula XI, R_{b} at position 2 is selected from the group consisting of substituted and unsubstituted heterocyclyl, substituted and unsubstituted aryl, substituted and unsubstituted heteroaryl, substituted and unsubstituted aralkyl, substituted and unsubstituted heteroaralkyl, and substituted and unsubstituted heterocyclylalkyl. In a preferred embodiment thereof said aryl group is more particularly defined as a fused arylaryl or unfused arylaryl and said heteroaryl group is more particularly defined as a fused heteroarylaryl, fused heteroarylheteroaryl, unfused heteroarylaryl, or unfused heteroarylheteroaryl.

Another embodiment of the invention provides a method of eliciting an immune response comprising administering a macrocycle SMIP to a patient in need thereof. In a more particular embodiment said macrocycle SMIP is an analog of cyclosporine. In a more particular embodiment, said analog of cyclosporine has a structure of formula (XII): 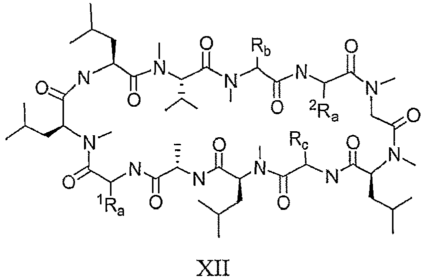 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{c} is selected from the group consisting of H, substituted and unsubstituted alkyl, substituted and unsubstituted aryl, substituted and unsubstituted heterocyclyl, and substituted and unsubstituted heteroaryl; or the stereoisomers thereof.

In a more particular embodiment said macrocycle SMIP has a structure of formula (XIII): 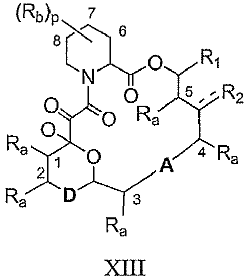 wherein,
a dotted line represents an optional placement of an additional bond;
A is selected from the group consisting of substituted C₁-C₁₆ alkyl, unsubstituted C₁-C₁₆ alkyl, substituted C₁-C₁₆ alkenyl, unsubstituted C₁-C₁₆ alkenyl, substituted C₁-C₁₆ alkoxy, or unsubstituted C₁-C₁₆ alkoxy;
D is absent, thereby forming a covalent bond or selected from the group consisting of -CH(alkoxy)-, -CH(alkoxyalkyl)-, -CH(alkylamino)-, -CH(aminoalkyl)-, -CH(alkylaminoalkyl)-, -C(=O)-, -CH(alkyl)-, and -CH₂-; R₁ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substitlited and unsubstituted aralkenyl, substituted and unsubstituted heteroaralkenyl, substituted and unsubstituted heterocyclylalkenyl, and substituted and unsubstituted carbocyclylalkenyl;
R₂ is selected from the group consisting of H, halogen, hydroxy, =O, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and p is 1 or 2;
or the stereoisomers thereof.

In a more particular embodiment of formula XIII, R₂ is substituted carbocyclylalkenyl.

In a more particular embodiment of formula XIII, A is further defined as a structure of group A₁: 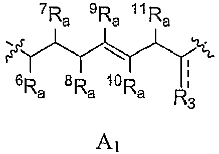 wherein,
a dotted line represents an optional placement of an additional bond;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R₃ is =O or bound by a single bond and selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl groups.

In a more particular embodiment of formula A₁, R₁ is substituted carbocyclylalkenyl, R₃ is =O and D is -CH(alkoxy)-.

In a more particular embodiment of formula XIII, A is further defined as a structure of group A₂: 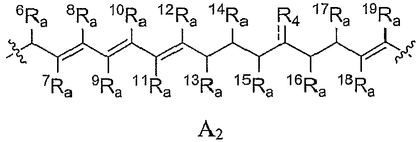 wherein,
a dotted line represents an optional placement of an additional bond;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R₄ is =O or bound by a single bond and selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl groups.

In a more particular embodiment of formula A₂, R₁ is substituted carbocyclylalkenyl, R₄ is =O and D is -CH(alkoxy)-.

The method as in any of formula's XIII, A₁, or A₂, R₁ is further defined as a substituent of formula (R₁ₐ): 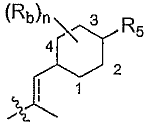 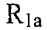 wherein,
a dotted line represents an optional placement of an additional bond;
R₅ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, substituted and unsubstituted carbocyclylalkenyl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
n is 1, 2, or 3.

In a more particular embodiment said macrocycle SMIP has a structure of formula (XIV): 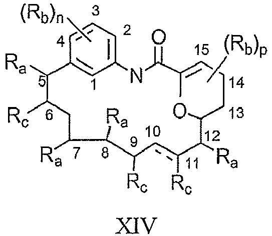 wherein,
a dotted line represents an optional placement of an additional bond;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{c} is independently selected from the group consisting of H, substituted and unsubstituted alkyl, substituted and unsubstituted aryl, substituted and unsubstituted heterocyclyl, and substituted and unsubstituted heteroaryl;
n is 1, 2, or 3; and
p is 1 or 2;
or the stereoisomers thereof.

In a more particular embodiment of formula XIV, R_{b} is H.

In a more particular embodiment of formula XIV, R_{c} is methoxy.

In a more particular embodiment said macrocycle SMIP has a structure of formula (XV): 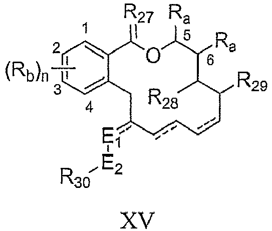 wherein,
a dotted line represents an optional placement of an additional bond;
E₁ is -N=, -(Rₐ)C=, -(R_{c})N-, or -(R_{c})CH-;
E₂ is absent forming a covalent bond, -O- or -CHR_{c}-;
R₂₇ is O= or bound by a single bond selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R₂₈ is selected from the group consisting of H, halogen, amino, hydroxy, substituted and unsubstituted alkyl, substituted and unsubstituted alkoxy, substituted and unsubstituted aryl, substituted and unsubstitute heterocyclyl, and substituted and unsubstituted heteroaryl; and
R₂₉ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; or
R₂₈ and R₂₉ are bound together by an oxygen atom, forming an epoxide;
R₃₀ is selected from the group consisting of H, carboxylic acid, substituted and unsubstituted alkyl, substituted and unsubstituted alkenyl, substituted and unsubstituted alkynyl, substituted and unsubstituted carbonyloxy, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{c} is independently selected from the group consisting of H, substituted and unsubstituted alkyl, substituted and unsubstituted aryl, substituted and unsubstituted heterocyclyl, and substituted and unsubstituted heteroaryl; and n is 1, 2, or 3;
or the stereoisomers thereof.

In a more particular embodiment of formula XV, E₁ is -N=, E₂ is -O-, and R₂₇ is 0=.

In a more particular embodiment said macrocycle SMIP has a structure of formula (XVI): 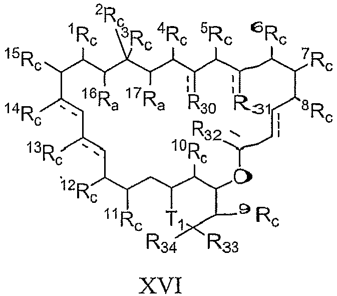 wherein,
a dotted line represents an optional placement of an additional bond;
T₁ is -CH(R_{c})-, -O-, or -N(R_{c})-;
R₃₀, R₃₁, and R₃₂ are independently O=, or selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R₃₃ is selected from the group consisting of H, halogen, amino, hydroxy substituted and unsubstituted alkyl, substituted and unsubstituted alkoxy, substituted and unsubstituted aryl, substituted and unsubstituted heterocyclyl, and substituted and unsubstituted heteroaryl; and
R₃₄ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; or
R₃₃ and R₃₄ are bound together forming a substituted carbocycle unsubstituted carbocycle, substituted heterocycle or unsubstituted heterocycle;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{c} is independently selected from the group consisting of H, substituted and unsubstituted alkyl, substituted and unsubstituted aryl, substituted and unsubstituted heterocyclyl, and substituted and unsubstituted heteroaryl; or the stereoisomers thereof.

In a more particular embodiment of formula XVI R₃₃ and R₃₄ are bound together forming a structure as shown in formula R₃₃ₐ: 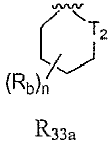 wherein,
T₂ is -CH(R_{c})-, -O-, or -N(R_{c})-;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is 1, 2, or 3.

Another embodiment of the invention provides a method of eliciting an immune response in a patient comprising administering a SMIP of formula (XVII): 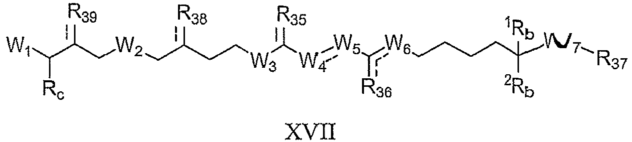 wherein,
a dotted line represents an optional placement of an additional bond with the proviso that two contiguous bonds are not double bonds;
W₁, W₂, and W₃ are independently -CH(Rₐ)- or -N(R_{c})-;
W₄ is -O-, -CH(Rₐ)- or -C(Rₐ)=,
W₅ is -O-, -CH(Rₐ)- or =C(Rₐ)-, with the proviso that both W₄ and W₅ are not -O-;
W₆ is -CH(Rₐ)-, =C(Rₐ)-, or NR_{c};
W₇ is -O- or -CH(Rₐ)-;
R₃₅, R₃₆, R₃₈ and R₃₉ are independently O= or selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R₃₇ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted aminocarbonylamino, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and R_{c} is selected from the group consisting of H, substituted and unsubstituted alkyl, substituted and unsubstituted aryl, substituted and unsubstituted heterocyclyl, and substituted and unsubstituted heteroaryl.

In a more particular embodiment of formula XVII, W₁, W₂, and W₃ are NH; W₇ is -CH(Rₐ)-; and R₃₇ is aminocarbonylamino.

In a more particular embodiment of XVII, wherein W₁, W₂, and W₃ are - CH(Rₐ)-; W₄ is -CH(Rₐ)- or -C(Rₐ)=; W₅ is -CH(Rₐ)- or =C(Rₐ)-; W₆ is =C(Rₐ)-;W₇ is -O-; and R₃₇ is H.

Another embodiment of the invention provides a method of eliciting an immune response in a patient comprising administering a SMIP of formula (XVIII) ; 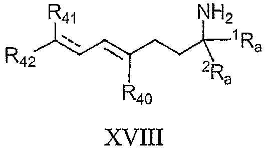 wherein,
R₄₀ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and
R₄, is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; or
R₄₀ and R₄₁ are bound together forming a substituted aryl, unsubstituted aryl, substituted heteroaryl, or unsubstituted heteroaryl group;
R₄₂ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl.

In a more particular embodiment of fonnula XVIII R₄₀ and R₄₁ are bound together forming an optionally substituted benzyl group. In a more particular embodiment of formula XVIII, wherein R₄₀ and R₄₁ are bound together forming an optionally substituted benzyl group, R₄₂ is unsubstituted alkyl.

Another embodiment of the invention provides a method of eliciting an immune response in a patient comprising administering a SMIP of formula (XIX): 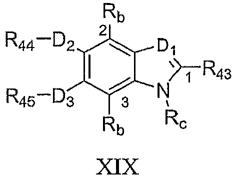 D₁ is -N= or -C(R₄₆)=;
D₂ and D₃ are independently selected from the group consisting of a covalent bond, -O-, -S-, -NH-, and NR_{d}-;
R₄₃ is selected from the group consisting of H, halogen, hydroxy, amino, nitro., cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted aralkylsulfonyl, substituted and unsubstituted heteroaralkylsulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkemyl; R₄₄ and R₄₅ are independently selected from the group consisting of H, halogen, cyano, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkellyl, substituted and unsubstituted heteroaralkenyl, substituted and unsubstituted heterocyclylalkenyl, and substituted and unsubstituted carbocyclylalkenyl groups;
R₄₆ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, -CH=N-N-CO-Rₐ, -CH₂CHRₐCOR_{b}, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl. substituted and unsubstituted heteroaralkenyl, substituted and unsubstituted heterocyclylalkenyl, and substituted and unsubstituted carbocyclylalkenyl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstitttted aminocarbonyl, substituted and unsubstittited carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{c} is selected from the group consisting of H, substituted and unsubstituted alkyl, substituted and unsubstituted aryl, substituted and unsubstituted heterocyclyl, and substituted and unsubstituted heteroaryl;
R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is 1, 2, or 3;
with the proviso that if R₄₃ is amino, alkylamino, carbonylamino, or alkylcarbonylamino, then both D₂ and D₃ are covalent bonds.

In a more particular embodiment of formula XIX, D₁ is CR₄₆, wherein R₄₆ is -CH=N-N-CO-Rₐ or -CH₂CHRₐCOR_{b}. In a more particular embodiment of formula XIX, wherein D₁ is CR₄₆, wherein R₄₆ is -CH=N-N-CO-Rₐ or -CH₂CHRₐCOR_{b}, R₄₃ is H.

In a more particular embodiment of formula XIX, R₄₃ is substituted or unsubstituted heteroaralkylsufonyl.

Another embodiment of the invention provides a method of eliciting an immune response in a patient comprising administering a SMIP of formula (XX): 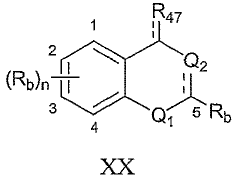 wherein,
a dotted line represents an optional placement of an additional bond;
Q₁ is -N(R_{d})- or -CH(R_{b})-;
Q₂ is -N(R_{c})-, =N-, or =C(R_{b})-;
R₄₇ is absent, =NR_{c} or -CH(R_{b})-;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{c} is independently selected from the group consisting of H, substituted and unsubstituted alkyl, substituted and unsubstituted aryl, substituted and unsubstituted heterocyclyl, and substituted and unsubstituted heteroaryl;
R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted alyl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is 1, 2, or 3.

In another embodiment, if n is 2 then R_{b} at positions 2 and 3 are not methoxy. In a more particular embodiment of formula XX, Examples 22 and 23 are excluded.

In a more particular embodiment of formula XX, Q₁ is -N(R_{d})-, Q₂ is - C(R_{b})= and R₄₇ is =NR_{c}.

In a more particular embodiment of formula XX, Q₁ is -CH(R_{b})-, Q₂ is - N(R_{c})-, and R₇ is -CH(R_{b})-. In a more particular embodiment of formula XX wherein Q₁ is -CH(R_{b})-, Q₂ is -N(R_{c})-, and R₄₇ is -CH(R_{b})-, R_{c} of Q₂ is substituted or unsubstituted heteroaryl.

In a more particular embodiment of formula XX, R₇ is absent and Q₁ is - N(R_{d})- and Q₂ is =N-.

Another embodiment of the invention provides a method of eliciting an immune response in a patient comprising administering a SMIP of formula (XXI): 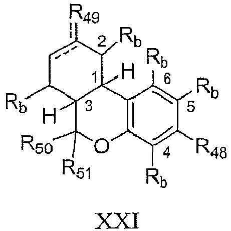 wherein,
a dotted line represents an optional placement of an additional bond with the proviso that two contiguous bonds are not double bonds;
R₄₈ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R₄₉ is =CH₂, =O or bound by a single bond and selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and Unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R₅₀ and R₅₁ are independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{b} is independently selected from the group consisting of H, halogen, hydroxy, amino, nitro, cyano, carboxylic acid, substituted and unsubstitute alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula XXI, R₄₈ is:

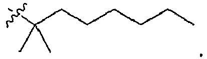

In a more particular embodiment of formula XXI, positions 1 and 3 are in an R configuration.

Another embodiment of the invention provides a method of eliciting an immune response in a patient comprising administering a SMIP of formula (XXII): 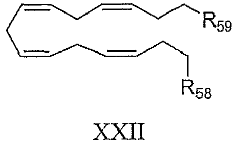 wherein,
R₅₈ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and
R₅₉ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted aminocarbonylalkyl, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula XXII, R₅₈ is unsubstituted alkyl such as propyl.

In a more particular embodiment of formula XXII, R₅₈ is substituted aminocarbonylalkyl, such as:

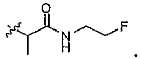

Further embodiments of the invention include a SMIP of structure XXI or XXII or the more particular embodiments thereof, wherein upon administration of said SMIP, cannabinoid receptors are inhibited in said patient.

Another embodiment of the invention provides a method of eliciting an immune response in a patient comprising administering a SMIP of formula (XXII): 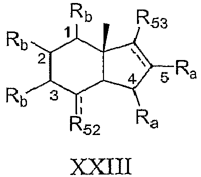 wherein,
a dotted line represents an optional placement of an additional bond;
R₅₂ is bound by a double bond and selected from the group consisting of =O, substituted and unsubstituted alkenyl, substituted and unsubstituted aralkenyl, substituted and unsubstituted heteroaralkenyl, substituted and unsubstituted heterocyclylalkenyl, substituted and unsubstituted carbocyclylalkenyl, substituted and unsubstituted imine, or bound by a single bond and selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, sub stituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl groups;
R₅₃ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylaminca, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl groups;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substitute d and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
or the stereoisomers thereof.

In a more particular embodiment of formula XXIII, R₅₂ is a substituted carbocyclylalkenyl as shown in figure R₅₂ₐ: 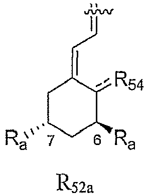 wherein,
a dotted line represents an optional placement of an additional bond;
R₅₄ is =CH₂, =O, or bound by a single bond and selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, substituted and unsub stituted carbocyclylalkenyl; and
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl.

In a more particular embodiment of formula R₅₂ₐ, R₅₄ is =CH₂ and Rₐ at positions 6 and 7 is hydroxy.

In a more particular embodiment of formula XXIII, R₅₃ is selected from the group consisting of substituted and unsubstituted alkyl, substituted and unsubstituted alkenyl, and substituted and unsubstituted alkoxyalkyl.

Another embodiment of the invention provides a method of eliciting an immune response in a patient comprising administering a SMIP of formula (XXIII): 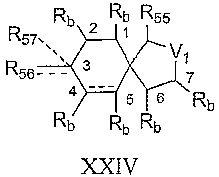 wherein,
a dotted line represents an optional placement of an additional bond, with the proviso that R₅₇ is absent if R₅₆ is bound by a double bond;
V₁ is CR_{b} or NR_{d};
R₅₅ is selected from the group consisting of H, halogen, hydroxy, amino, nitro, cyano, carboxylic acid, substituted and unsubstituted alkyl, substituted and unsubstituted alkenyl, substituted and unsubstituted alkynyl, sub stituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, substituted and unsubstituted and substituted and unsubstituted carbocyclylalkenyl;
R₅₆ is =O and R₅₇ is absent; or
R₅₆ is bound to position 3 by a single bond and selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R₅₇ is bound to position 3 by a single bond and selected from the group consisting of H, halogen, amino, hydroxy, substituted and unsubstituted alkyl, substituted and unsubstituted alkoxy, substituted and unsubstituted alkoxyalkyl, and substituted and unsubstituted carbonyl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclalkenyl.

In a more particular embodiment of formula XXIV, V, is N-alkyl-amino or N-alkyl-heterocyclyl. In a more particular embodiment of formula XXIV, wherein V₁ is N-alkyl-amino or N-alkyl-heterocyclyl, R₅₆ and R₅₇ are independently substituted or unsubstituted alkyl and bound to position 3 by a single bond. In a more particular embodiment thereof, R₅₆ is =O, R₅₇ is absent and V₁ is CR_{b}, R_{b} being preferably alkenyl.

Another embodiment of the invention provides a method of eliciting an immune response in a patient comprising administering a SMIP of formula (XXV): 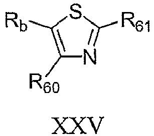 wherein,
R₆₀ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R₆₁ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula XXV, R_{b} is heteroarylsulfonyl.

In a more particular embodiment of formula XXV, R₆₀ is an aryl or aralkyl group wherein said aryl group alone or appended to an alkyl group is more particularly defined as an unfused arylaryl group. In preferred embodiments of any of the embodiments formula XXV; XXV where R_{b} is heteroarylsulfonyl; or XXV where R₆₀ is an optionally substituted aryl or aralkyl group wherein said aryl group alone or appended to an alkyl group is more particularly defined as an unfused arylaryl group, R₆ is amino, substituted alkylamino, or unsubstituted alkylamino.

Another embodiment of the invention provides a method of eliciting an immune response in a patient comprising administering a SMIP of formula (XXVI): 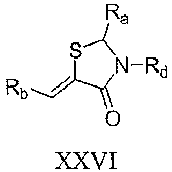 wherein,
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula XXVI, R_{b} is a substituted benzyl group.

Another embodiment of the invention provides a method of eliciting an immune response in a patient comprising administering a SMIP of formula (XXVII): 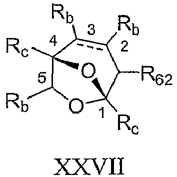 wherein,
a dotted line represents an optional placement of an additional bond;
R₆₂ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted arylcarbonyloxy, substituted and unsubstituted heteroarylcarbonyloxy, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and R_{c} is independently selected from the group consisting of H, substituted and unsubstituted alkyl, substituted and unsubstituted aryl, substituted and unsubstituted heterocyclyl, and substituted and unsubstituted heteroaryl.

In a more particular embodiment of formula XXVII, R₆₂ is substituted or unsubstituted heteroarylcarbonyloxy.

In a more particular embodiment of formula XXVII, the dotted line represents the placement of an additional bond.

Another embodiment of the invention provides a method of eliciting an immune response in a patient comprising administering a SMIP of formula (XXVIII): 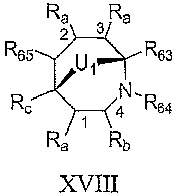 wherein,
U₁ is -CH(Rₐ)- or -O-;
R₆₃ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and
R₆₄ is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, sub stituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, substituted and unsubstituted and substituted and unsubstituted carbocyclylalkenyl; or
R₆₃ and R₆₄ are taken together to form an optionally substituted heterocyclyl group;
R₆₅ is selected from the group consisting of H, halogen, amino, hydroxy, sulfonyl, -O-PO₃, substituted and unsubstituted alkyl, substituted and unsubstituted alkoxy, substituted and unsubstituted aryl, substituted and unsubstituted heterocyclyl, and substituted and unsubstituted heteroaryl;
Rₐ is independently selected from the group consisting of H, halogen, amino, hydroxy, substituted and unsubstituted alkyl, sub stituted and unsubstituted alkoxy, substituted and unsubstituted aryl, substituted and unsubstituted heterocyclyl, and substituted and unsubstituted heteroaryl;
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsub stituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R_{c} is selected from the group consisting of H, substituted and unsubstituted alkyl, substituted and unsubstituted aryl, substituted and unsubstituted heterocyclyl, and substituted and unsubstituted heteroaryl.

In a more particular embodiment of formula XXVIII, R₆₃ and R₆₄ are taken together to form an optionally substituted heterocyclyl group of formula R₆₃ₐ: 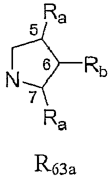 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula XXVIII, wherein R₆₃ and R₆₄ are taken together to form an optionally substituted heterocyclyl group of formula R₆₃ₐ, U₁ is -CH₂- and R_{b} of formula R₆₃ₐ is alkylamino.

In a more particular embodiment of formula XXVIII, R_{b} at position 4 is para-methoxybenzyl.

In a more particular embodiment of formula XXVIII, R₆₅ is -O-PO₃.

Another embodiment of the invention provides a method of eliciting an immune response in a patient comprising administering a SMIP of formula (XXIX): 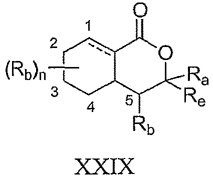 wherein,
a dotted line represents an optional placement of an additional bond;
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted hetero cyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; Rₑ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
nis 1, 2, o r3.

In a more particular embodiment of formula XXIX, R_{b} at positions 1,3, and 4 is hydroxy.

In a more particular embodiment of formula XXIX, the dotted line represents the placement of an additional bond and R_{b} at position 5 is amino.

Another embodiment of the invention provides a method of eliciting an immune response in a patient comprising administering a SMIP of formula (XXX): 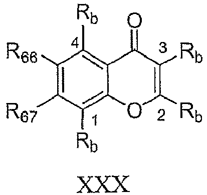 wherein,
R₆₆ and R₆₇ are independently H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted aminosulfonyl, substituted and unsubstituted alkylaminosulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted aryloxy, substituted and unsubstituted heteroaryloxy, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula XXX, R₆₇ is substituted aminosulfonyl, unsubstituted aminosulfonyl, substituted alkylaminosulfonyl, or unsubstituted aminosulfonyl.

In a more particular embodiment of formula XXX, R₆₆ is substituted aryloxy, unsubstituted aryloxy, substituted heteroaryloxy, or unsubstituted heteroaryloxy.

In a more particular embodiment of formula XXX, R_{b} at position 3 is substituted or unsubstituted carbonylamino.

Another embodiment of the invention provides a method of eliciting an immune response in a patient comprising administering a SMIP of formula (XXXI): 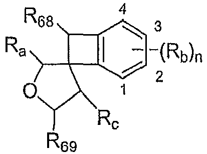 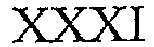 wherein,
R₆₈ and R₆₉ are independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{c} is selected from the group consisting of H, substituted and unsubstituted alkyl, substituted and unsubstituted aryl, substituted and unsubstituted heterocyclyl, and substituted and unsubstituted heteroaryl; and
n is 1, 2, or 3.

In a more particular embodiment of formula XXXI, R₆₈ is a substituted or unsubstituted aryl group.

In a more particular embodiment of formula XXXI, R₆₉ is optionally substituted 4H-pyran-4-one.

Another embodiment of the invention provides a method of eliciting an immune response in a patient comprising administering a SMIP of formula (XXXII): 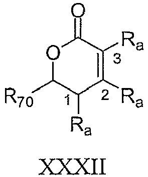 wherein,
R₇₀ is selected from the group consisting of H, halogen, amino, hydroxy, substituted and unsubstituted alkyl, substituted and unsubstituted alkylamino, substituted and unsubstituted alkoxy, substituted and unsubstituted aryl, substituted and unsubstituted heterocyclyl, and substituted and unsubstituted heteroaryl; and
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl.

In a more particular embodiment of formula XXXII, R₇₀ is further defined as R₇₀ₐ: 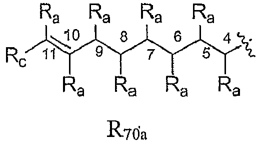 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{c} is selected from the group consisting of H, substituted and unsubstituted alkyl, substituted and unsubstituted aryl, substituted and unsubstituted heterocyclyl, and substituted and unsubstituted heteroaryl.

In a more particular embodiment R₇₀ₐ is defined as:

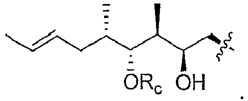

Another embodiment of the invention provides a method of eliciting an immune response in a patient comprising administering a SMIP of formula (XXXIII): 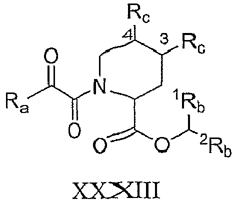 wherein,
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted amino carbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and R_{c} is independently selected from the group consisting of H, substituted and unsubstituted alkyl, substituted and unsubstituted aryl, substituted and unsubstituted heterocyclyl, and substituted and unsubstituted heteroaryl.

In a more particular embodiment of formula XXXIII, R_{b} at positions 1 and 2 are independently selected from the group consisting of substituted or unsubstituted aralkyl and heteroaralkyl.

In a more particular embodiment of formula XXXIV, Rₐ is trimethoxyphenyl.

Another embodiment of the invention provides a method of treating a patient comprising administering a terpene SMIP to a patient in need thereof, wherein upon administration an immune response is elicited in said patient.

In a more particular embodiment said terpene SMIP is a structure of formula (XXXIV): 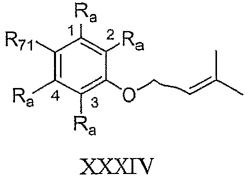 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R₇ is selected from the group consisting; of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstittited carbocyclylalkenyl;

In a more particular embodiment of formula XXXIV, R₇₁ is a substituted or unsubstituted aryl group more particularly defined as a substituted or unsubstituted unfused arylaryl group.

In a more particular embodiment of formula XXXIV, wherein R₇₁ is a substituted or unsubstituted unfused arylaryl group, said substituted or unsubstituted arylaryl group is defined as R₇₁ₐ: 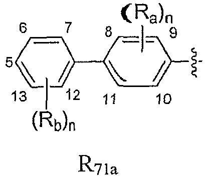 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstitute alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstitlited carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is independently 1, 2, or 3.

In a more particular embodiment, said terpene SMIP is a structure of formula (XXXV): 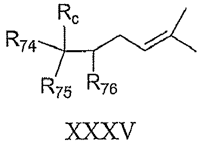 wherein,
R₇₄ is selected from the group consisting substituted and unsubstituted alky 1, substituted and unsubstituted aryl, substituted and unsubstituted heterocyclyl and substituted and unsubstituted heteroaryl;
R₇₅ and R₇₆ are independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; or R₇₅ and R₇₆ are bound together to form an epoxide; and,
R_{c} is selected from the group consisting of H, substituted and unsubstituted. alkyl, substituted and unsubstituted aryl, substituted and unsubstituted heterocyclyl, and substituted and unsubstituted heteroaryl.

In a more particular embodiment of formula XXXV, R₇₄ is further defined as a structure of formula R₇₄ₐ: 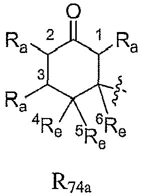 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and Rₑ is independently selected from the group consisting of H, halogen, hydroxy, amino, nitro, cyano, carboxylic acid, substituted and unsubstituted alkyl, substituted and unsubstituted alkenyl, substituted and unsubstituted alkynyL, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl.

Another embodiment of the invention provides a method of eliciting an immune response in a patient comprising administering a SMIP of formula (XXXVI): 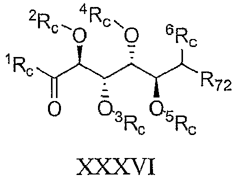 wherein,
R₇₂ is selected from the group consisting of H, halogen, amino, hydroxy, sulfonyl, -O-PO₃, substituted and unsubstituted alkyl, substituted and unsubstituted alkoxy, substituted and unsubstituted aryl, substituted and unsubstituted heterocyclyl, and substituted and unsubstituted heteroaryl; and R_{c} is independently selected from the group consisting of H, substituted and unsubstituted alkyl, substituted and unsubstituted aryl, substituted and unsubstituted heterocyclyl, and substituted and unsubstituted heteroaryl.

In a more particular embodiment of formula XXXVI, R_{c} is H.

In a more particular embodiment of formula XXXVI, R₇₂ is -O-PO₃.

Another embodiment of the invention provides a method of eliciting an immune response in a patient comprising administering a SMIP of formula (XXXVII): 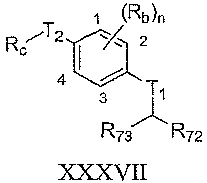 wherein,
T₁ and T₂ are independently O, S, CHR_{c}, or NR_{c};
R₇₂ and R₇₃ are independently selected from the group consisting of H, halogen, amino, hydroxy, sulfonyl, -PO₃, substituted and unsubstituted alkyl,, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{c} is selected from the group consisting of H, substituted and unsubstituted alkyl, substituted and unsubstituted aryl, substituted and unsubstituted heterocyclyl, and substituted and unsubstituted heteroaryl; and
n is 1, 2, or 3; or
the pharmaceutically acceptable salts thereof.

In a more particular embodiment of formula XXXVII, Examples 2, 6, 9, 11, 20, and 21 are excluded.

Furthermore, R_{b} of formula XXXVII may be H and said pharmaceutically acceptable salt may be sodium.

In a more particular embodiment of formula XXXVII, T₁ and T₂ are both S. Furthermore, R_{b} may be H and said pharmaceutically acceptable salt may be sodium.

In a more particular embodiment of formula XXXVII, R₇₂ and R₇₃ are - PO₃. Furthermore, R_{b} may be H and said pharmaceutically acceptable salt may be sodium.

Another embodiment of the invention provides a method of treating a patient comprising administering a ruthenium complex SMIP to a patient in need thereof, wherein upon administration an immune response is elicited in said patient. In a more particular embodiment said ruthenium complex SMIP consists of at least 2 nitrogen-containing heteroaryl groups, such as imidazole. In an even more particular embodiment, said ruthenium complex SMIP consists of 6 nitrogen-containing heteroaryl groups, such as imidazole.

In a more particular embodiment, said ruthenium complex SMIP is a structure of formula (XXXVIII): 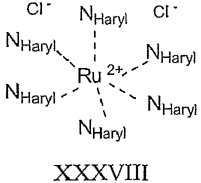 wherein,
N_{Haryl} is a substituted or unsubstituted nitrogen containing heteroaryl group.

In a more particular embodiment of formula XXXVIII, said nitrogen containing heteroaryl group is selected from the group consisting of acridine, carbazole, β-carboline, cinnoline, imidazole, indazole, indole, indolizine, isoindole, isoquinoline, isothiazole, oxazole, isoxazole, naphthyridine, perimidine, phenanthridine, phenanthroline, phenazine, phthalazine, pteridine, purine, pyrazine, pyrazole, pyridazine, pyridine, pyrimidine, pyrrole, pyrrolizine, quinazoline, quinoline, quinolizine, quinoxaline, and thiazole.

Another embodiment of the invention provides a method of eliciting an immune response in a patient comprising administering a SMIP of formula (XXXIX): 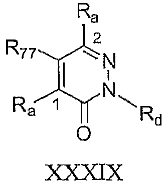 wherein,
R₇₇ is selected from the group consisting of H, halo, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkylamino, substituted and unsubstituted heteroaralkylamino, substituted and unsubstituted heterocyclylalkylamino, substituted and unsubstituted carbocyclylalkylamino, substituted and unsubstituted aralkenyl, substituted and unsubstituted heteroaralkenyl, substituted and unsubstituted heterocyclylalkenyl, and substituted and unsubstituted carbocyclylalkenyl;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiment of formula XXXIX, R₇₇ is selected from the group consisting of substituted and unsubstituted aralkylamino and substituted and unsubstituted heteroaralkylamino.

Another embodiment of the invention provides a method of eliciting an immune response in a patient comprising administering a SMIP of formula (XL): 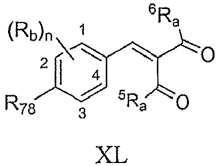 wherein,
R₇₈ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted heteroaralkoxy, substituted and unsubstituted aralkoxy, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl; and R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and n is 1, 2, or 3.

In a more particular embodiment of formula XL, R₇₈ is substituted or unsubstituted aralkoxy.

Another embodiment of the invention provides a method of eliciting an immune response in a patient comprising administering a SMIP of formula (XLI): 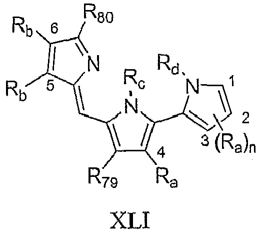 wherein,
R₈₀ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{c} is selected from the group consisting of H, substituted and unsubstituted alkyl, substituted and unsubstituted aryl, substituted and unsubstituted heterocyclyl, and substituted and unsubstituted heteroaryl;
R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
nis 1, 2, or 3.

In a more particular embodiment of formula XLI, R₈₀ is undecanyl.

Another embodiment of the invention provides a method of eliciting an immune response in a patient comprising administering a SMIP of formula (XLII): 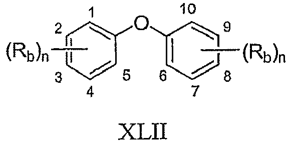 wherein,
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is 1, 2, or 3.

In a more particular embodiment of formula XLII, R_{b} at position 1 is a substituted alkyl group further defined as carbonylalkyl or -alkyl-COOH.

Another embodiment of the invention provides a method of eliciting an immune response in a patient comprising administering a SMIP of formula (XLIII): 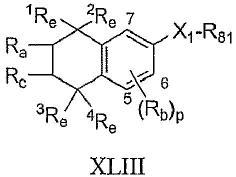 wherein,
X₁ is selected from the group consisting of -CO-, -O-, -S-, -NH-, sulfonyl, substituted and unsubstituted alkyl, substituted and unsubstituted alkenyl, substituted and unsubstituted alkynyl, substituted and unsubstituted carbonylalkyl, carbonyl, substituted and unsubstituted alkoxycarbonyl, substituted and unsubstituted alkylamino, substituted and unsubstituted carbonyloxy, aminocarbonyl, carbonylamino, substituted and unsubstituted alkoxy, substituted and unsubstituted alkoxyalkyl, sulfonamide, imine, and thiourea;
R₈₁ is selected from the group consisting of H, substituted and unsubstituted aryl, substituted and unsubstituted heterocyclyl, substituted and unsubstituted carbocyclyl, and substituted and unsubstituted heteroaryl;
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{c} is selected from the group consisting of H, substituted and unsubstituted alkyl, substituted and unsubstituted aryl, substituted and unsubstituted heterocyclyl, and substituted and unsubstituted heteroaryl;
Rₑ is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
p is 1 or 2.

In a more particular embodiment of formula XLIII, X₁ is substituted or unsubstituted alkenyl. In a more particular embodiment of formula XLIII, wherein X₁ is substituted or unsubstituted alkenyl, R₈₁ is substituted or unsubstituted aryl. In a preferred embodiment thereof, R₈₁ is an aryl substituted with a sulfonyl or alkylsulfonyl group.

Another embodiment of the invention provides a method of eliciting an immune response in a patient comprising administering a SMIP of formula (XLIV): 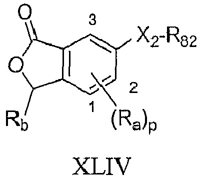 wherein,
X₂ is selected from the group consisting of -CO-, -O-, -S-, -NH-, sulfonyl, substituted and unsubstituted alkyl, substituted and unsubstituted alkenyl, substituted and unsubstituted alkynyl, substituted and unsubstituted carbonylalkyl, carbonyl, substituted and unsubstituted alkoxycarbonyl, substituted and unsubstituted alkylamino, substituted and unsubstituted carbonyloxy, substituted and unsubstituted aminocarbonyl, substituted and unsubstituted carbonylamino, substituted and unsubstituted alkoxy, substituted and unsubstituted alkoxyalkyl, sulfonamide, imine, and thiourea;
R₈₂ is selected from the group consisting of H, substituted and unsubstituted aryl, substituted and unsubstituted heterocyclyl, substituted and unsubstituted carbocyclyl, and substituted and unsubstituted heteroaryl;
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
p is 1 or 2.

In a more particular embodiment of formula XLIV, X₂ is a substituted alkenyl that is further defined as an optionally substituted -alkenyl-carbonyl-oxy-. In a more particular embodiment thereof, R₈₂ is a sub stituted or unsubstituted heterocyclyl, such as morpholine.

Another embodiment of the invention provides a method of eliciting an immune response in a patient comprising administering a SMIP of formula (XLV): 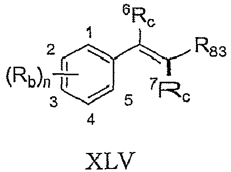 wherein,
R₈₃ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted carbocyclylamino, substituted and unsubstituted heterocyclylamino, substituted and unsubstituted arylamino, substituted and unsubstituted heteroarylamino, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{c} is independently selected from the group consisting of H, substituted and unsubstituted alkyl, substituted and unsubstituted aryl, substituted and unsubstituted heterocyclyl, and substituted and unsubstituted heteroaryl; and
n is 1, 2, or 3.

In another embodiment of the invention, if both R_{c} groups and R_{b} are all H, then R₈₃ is not an alkyl group substituted with a piperizinyl group. In another embodiment of formula XLV, Example 15 is excluded.

In a more particular embodiment of formula XLV, R_{b} at position 3 may be substituted or unsubstituted carbocyclyl.

In a more particular embodiment of formula XLV, R₈₃ is substituted or unsubstituted carbocyclylamino. Furthermore, R_{b} at position 3 may be substituted or unsubstituted carbocyclyl.

Another embodiment of the invention provides a method of eliciting an immune response in a patient comprising administering a SMIP of formula (XLVI): 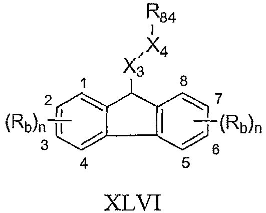 wherein,
X₃ is a substituted or unsubstituted alkyl group;
X₄ is a covalent bond or selected from the group consisting of -CO-, -O-CO-N(Rₐ)-, -O-, -S-, -NH-, sulfonyl, substituted and unsubstituted alkyl, substituted and unsubstituted alkenyl, substituted and unsubstituted alkynyl, substituted and unsubstituted carbonylalkyl, carbonyl, substituted and unsubstituted alkoxycarbonyl, substituted and unsubstituted alkylamino, substituted and unsubstituted carbonyloxy, substituted and unsubstituted aminocarbonyl, substituted and unsubstituted carbonylamino, substituted and unsubstituted alkoxy, substituted and unsubstituted alkoxyalkyl, sulfonamide, imine, and thiourea;
R₈₄ is selected from the group consisting of H, substituted and unsubstituted carbocyclyl, substituted and unsubstituted heterocyclyl, substituted and unsubstituted aryl, and substituted and unsubstituted heteroaryl groups;
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and nis 1, 2, or 3.

In a more particular embodiment of formula XLVI, X₃ is ethyl and X₄ is -O-CO-NH-.

In a more particular embodiment of formula XLVI, R_{b} is H. Furthermore, X₃ may be ethyl and X₄, -O-CO-NH-.

Another embodiment of the invention provides a method of eliciting an immune response in a patient comprising administering a SMIP of formula (XLVII): 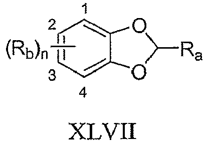 wherein,
Rₐ is selected from the group consisting of H, halogen, amino, hydroxy, substituted and unsubstituted alkyl, substituted and unsubstituted alkoxy, substituted and unsubstituted aryl, substituted and unsubstituted hetero cyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and n is 1, 2, or 3.

In a more particular embodiment of formula XLVII, R_{b} at position 2 is substituted or unsubstituted alkylcarbonylamino.

In a more particular embodiment of formula XLVII, Rₐ is H.

Another embodiment of the invention provides a method of eliciting an immune response in a patient comprising administering a SMIP of formula (XLVIII): 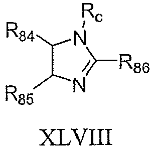 wherein,
R₈₄, R₈₅, and R₈₆ are independently selected from the group consisting of substituted and unsubstituted carbocyclyl, substituted and unsubstituted heterocyclyl, substituted and unsubstituted aryl, and substituted and unsubstituted heteroaryl groups; and
R_{c} is selected from the group consisting of H, substituted and unsubstituted alkyl, substituted and unsubstituted aryl, substituted and unsubstituted heterocyclyl, and substituted and unsubstituted heteroaryl.

In a more particular embodiment of formula XLVIII, R₈₄, R₈₅, and R₈₆ are independently substituted or unsubstituted aryl.

In a more particular embodiment of formula XLVIII, R₈₄, R₈₅, and R₈₆ are independently substituted or unsubstituted phenyl.

Another embodiment of the invention provides a method of eliciting an immune response in a patient comprising administering a SMIP of formula (XLIX): 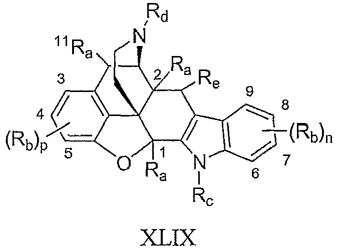 wherein,
Rₐ is independently selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted hetero cyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; R_{c} is selected from the group consisting of H, substituted and unsubstituted alkyl, substituted and unsubstituted aryl, substituted and unsubstituted heterocyclyl, and substituted and unsubstituted heteroaryl;
R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; Rₑ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl;
n is 1, 2, or 3; and
p is 1 or 2.

In a more particular embodiment of formula XLIX, R_{d} is substituted or unsubstituted carbocyclylalkyl.

In a more particular embodiment of formula XLIX, Rₐ at position 1 is substituted or unsubstituted alkyl.

In a more particular embodiment of formula XLIX, Rₐ at position 2 is substituted or unsubstituted alkoxy.

Another embodiment of the invention provides a method of eliciting an immune response in a patient comprising administering a SMIP of formula (L): 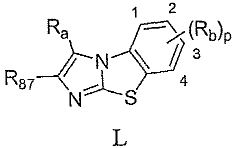 wherein,
R₈₇ is selected from the group consisting of H, substituted and unsubstituted alkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted arylamino, substituted and unsubstituted heteroarylamino, substituted and unsubstituted heterocyclylamino, and substituted and unsubstituted carbocyclylamino;
Rₐ is selected from the group consisting of H, halogen, amino, hydroxy, substituted and unsubstituted alkyl, substituted and unsubstituted alkoxy, substituted and unsubstituted aryl, substituted and unsubstituted heterocyclyl, and substituted and unsubstituted heteroaryl;
R_{b} is independently selected from the group consisting of H, halogen, hydroxy, amino, nitro, cyano, carboxylic acid, substituted and unsubstituted alkyl, substituted and un substituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substitute and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is 1, 2, or 3.

In a more particular embodiment of formula L, R_{b} is H.

In a more particular embodiment of formula L, R₈₇ is a substituted aryl or heteroaryl group. Furthermore, R_{b} may be H. In a more particular embodiment of formula L, wherein R₈₇ is a sub stituted aryl or heteroaryl group, said aryl or heteroaryl group of R₈₇ may be substituted with a malonate. Furthermore, R_{b} may be H.

Another embodiment of the invention provides a method of eliciting an immune response in a patient comprising administering a SMIP of formula (LI): 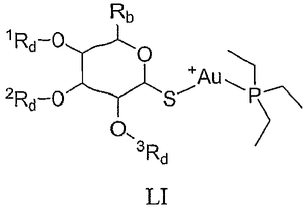 wherein,
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsub stituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted carbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl.

In a more particular embodiments of formula LI, R_{d} is substituted or unsubstituted carbonyl.

In a more particular embodiment of formula LI, R_{d} is methylcarbonyl.

In one embodiment of the invention compounds of Table 3 (Examples 2-67) are excluded from any one of the embodiments disclosed herein. In another embodiment of the invention any of the compounds disclosed in US Application Number 10/814480 are excluded from any one of the embodiments disclosed herein. Preferably specific embodiments for which the aforementioned disclaimers apply are those of Formula I-LI.

Another embodiment of the invention provides a method of identifying a SMIP comprising:
a. identifying a core scaffold of a SMIS;
b. altering or adding at least one variable substituent of said core scaffold of said SMIS with a substituent selected from the group consisting of substituted or unsubstituted alkyl, alkoxy, alkenyl, alkynyl, amino, alkylamino, dialkylamino, aryl, aralkyl, heteroaryl, heteroaralkyl, heterocyclyl, heterocyclylalkyl, carbonyl, carbonyloxy, sulfonyl, carbonylamino, aminocarbonyl, guanidinyl, and alkoxycarbonyl, to form a SMIS analog;
c. contacting said SMIS analog with cells to form a reaction solution;
d. monitoring said reaction solution for an immune response to said SMIS analog; and
e. identifying said immune response to said SMIS analog by detecting increased levels of cytokines or TNF-α, wherein a SMIS analog capable of eliciting cytokine or TNF-α production is a SMIP.

In a more particular embodinent, said cells are human peripheral blood mononuclear cells. In another embodiment said cells are human monocyte-derived cells, specifically THP-1. In another embodiment said cells are mouse monocyte-derived cell, such as, Raw 264.7. In another more particular embodiment said reaction solution is incubated at about 37 °C.

In another more particular embodiment said reaction solution is incubated for at least about 2 hours, or at least about 5 hours, or at least about 10 hours, or at least about 15 hours, or at least about 18 hours, or about 18 hours.

In another more particular embodiment, wherein said cells are human peripheral blood mononuclear cells; and said reaction solution is incubated for at least about 2 hours, or at least about 5 hours, or at least about 10 hours, or at least about 15 hours, or at least about 18 hours, or about 18 hours, said reaction solution is incubated at about 37°C.

In another more particular embodiment said step of detecting increased levels of cytokines or TNF-α is done by using a primary plate bound antibody for capture followed by a secondary biotinylated anti-TNF antibody. In a more particular embodiment said secondary biotinylated anti-TNF antibody is detected using streptavidin-Europium. In a more particular embodiment thereof, increased levels of cytokines or TNF-α in said reaction solution are identified by an increased europium count. In a still more particular embodiment said increased europium count is identified by time resolved fluorescence.

Further embodiments of the invention include a SMIP of structure I-L or the more particular embodiments thereof, wherein said SMIP is co-administered with another agent, such as a vaccine.

Other embodiments provide the use of a SMIP of formula I-L and another agent for simultaneous separate or sequential administration. In a more particular embodiment the other agent is an antigen. In another more particular embodiment the use is for eliciting an immune response in a patient. In another embodiment the use is for treating an infectious disease, autoimmune disease, allergies, or cancer. In another embodiment the use is as an adjuvant.

Other embodiments provide a pharmaceutical preparation or system, comprising (a) a first pharmaceutical agent, which comprises a SMIP of formula I-L; and (b) a second pharmaceutical agent, wherein said first and second agents are either in admixture or are separate compositions. In a more particular embodiment the second agent is an antigen. More specifically, the agents are for simultaneous separate or sequential administration. In another more particular embodiment the use is for eliciting an immune response in a patient. In another embodiment the use is for treating an infectious disease, autoimmune disease, allergies, or cancer. In another embodiment the use is as an adjuvant.

A kit comprising (a) a first pharmaceutical agent, which comprises a SMIP of formula I-L; and (b) a second pharmaceutical agent. In a more particular embodiment the second agent is an antigen. In another more particular embodiment the use is for eliciting an immune response in a patient. In another embodiment the use is for treating an infectious disease, autoimmune disease, allergies, or cancer. In another embodiment the use is as an adjuvant.

Another embodiment provides the use of a SMIP of formula I-L and another agent in the manufacture of a combination medicament. In a more particular embodiment the other agent is an antigen. In another more particular embodiment the use is for eliciting an immune response in a patient. In another embodiment the use is for treating an infectious disease, autoimmune disease, allergies, or cancer. In another embodiment the use is as an adjuvant.

Another embodiment provides the use of a SMIP of formula I-L in the manufacture of a medicament, wherein the medicament is co-administered with another agent. In a more particular embodiment the second agent is an antigen. In another more particular embodiment the use is for eliciting an immune response in a patient. In another embodiment the use is for treating an infectious disease, autoimmune disease, allergies, or cancer. In another embodiment the use is as an adjuvant.

Another embodiment provides the use of an antigen in the manufacture of a medicament, wherein the medicament is co-administered with a SMIP of formula I-L.

The two agents are preferably administered within 4 hours of each other.

Another embodiment provides the use of a SMIP of formula I-L in the manufacture of a medicament, wherein the medicament is for administration to a patient who has been pre-treated with another agent. In a more particular embodiment the second agent is an antigen. In another more particular embodiment the use is for eliciting an immune response in a patient. In another embodiment the use is for treating an infectious disease, autoimmune disease, allergies, or cancer. In another embodiment the use is as an adjuvant.

Another embodiment provides the use of an antigen in the manufacture of a medicament, wherein the me dicament is for administration to a patient who has been pre-treated with a SMIP of formula I-L. The pre-treatment may be recent (e.g. within the 24 hours preceding administration of said medicament), intermediate (e.g. more than 24 hours previous, but no longer than 4 weeks), more distant (e.g. at least 4 weeks previous), or very distant (e.g. at least 6 months previous), with these time periods referring to the most recent pre treatment dose. The patient may be refractory to treatment by the pharmaceutical agent that was administered in the pre-treatment. In another more particular embodiment the use is for eliciting an immune response in a patient. In another embodiment the use is for treating an infectious disease, autoimmune disease, allergies, or cancer. In another embodiment the use is as an adjuvant.

Another embodiment provides, the use of a SMIP of formula I-L in the manufacture of a medicament, wherein the medicament is for administration to a patient who has a tumor or infection that is resistant to treatment with another agent.

Further embodiments of the invention include a SMIP of structure I-L or the more particular embodiments thereof, wherein said SMIP is administered in a dose capable of increasing TNF-α levels.

Further embodiments of the invention include a SMIP of structure I-L or the more particular embodiments thereof, wherein said SMIP modulates activity of at least one target selected from the group consisting of glucocortocoid receptors, DNA alkylation, calcineurin, JNK, p38 kinase, cyclin kinase cascade, PDE IV, IMPDH, DHOD, 1ck, and thymidylate synthase.

Further embodiments of the invention include a SMIP of structure I-L or the more particular embodiments thereof, wherein said immune response involves production of cytokines.

Further embodiments of the invention include a SMIP of structure I-L or the more particular embodiments thereof, wherein said immune response involves increased production of TNF-α.

Further embodiments of the invention include a SMIP of structure I-L or the more particular embodiments thereof, wherein the patient is suffering from a viral infection, such as HCV. In a preferred embodiment said SMIP is co-administered with another agent, such as a vaccine.

Further embodiments of the invention include a SMIP of structure I-L or the more particular embodiments thereof, wherein said patient is suffering from increased cellular proliferation or cancer. In a preferred embodiment said SMIP is co-administered with another agent, such as a vaccine.

Further embodiments of the invention include a SMIP of structure I-L or the more particular embodiments thereof, wherein said patient is suffering from allergic diseases. In a preferred embodiment said SMIP is co-administered with another agent, such as a vaccine.

Further embodiments of the invention include a SMIP of structure I-L or the more particular embodiments thereof, wherein said patient is suffering from asthma. In a preferred embodiment said SMIP is co-administered with another agent, such as a vaccine.

Further embodiments of the invention include a SMIP of structure I-L or the more particular embodiments thereof, wherein said SMIP, present at a concentration less than 20uM, induces production of TNF-α.

Candidate small molecule immuno-potentiators can be identified *in vitro.* Compounds are screened *in vitro* for their ability to activate immune cells. One marker of such activation is the induction of cytokine production, for example TNF-α production. Apoptosis inducing small molecules may be identified having this activity. These small molecule immuno-potentiators have potential utility as adjuvants and immuno-therapeutics.

Another embodiment of the invention provides a method of identifying a SMIP comprising:
a) contacting a compound with cells to form a cell culture;
b) incubating said cell culture for at least 1 hour;
c) monitoring for increased TNF-α levels in said cell culture;
d) identifying said SMIP by the increased TNF-α levels in said cell culture.

In a more particular embodiment, said cells are human peripheral blood mononuclear cells. In another more particular embodiment said cell culture is incubated at about 37 °C.

In another more particular embodiment said cell culture is incubated for at least about 2 hours, or at least about 5 hours, or at least about 10 hours, or at least about 15 hours, or at least about 18 hours, or about 18 hours.

In another more particular embodiment, wherein said cells are human peripheral blood mononuclear cells; and said cell culture is incubated for at least about 2 hours, or at least about 5 hours, or at least about 10 hours, or at least about 15 hours, or at least about 18 hours, or about 18 hours, said cell culture is incubated at about 37 °C.

In another more particular embodiment said step of monitoring for increased TNF-α levels in said cell culture is done by using a primary plate bound antibody for capture followed by a secondary biotinylated anti-TNF antibody. In a more particular embodiment said secondary biotinylated anti-TNF antibody is detected using streptavidin-Europium. In a more particular embodiment thereof, increased TNF-α levels in said cell culture are identified by an increased europium count. In a still more particular embodiment said increased europium count is identified by time resolved fluorescence.

Another embodiment of the invention provides a method of identifying a potency of a SMIP comprising:
a) contacting a compound, and separately LPS, with cells to form a compound cell culture and an LPS cell culture;
b) incubating said compound cell culture and said LPS cell culture for at least 1 hour;
c) monitoring for increased TNF-α levels in said compound cell culture and said LPS cell culture;
d) comparing the increased TNF-α levels in said compound cell culture with the increased TNF-α levels in said LPS cell culture;
e) identifying the potency of said SMIP by the increase in TNF-α levels in said compound cell culture with respect to the increased TNF-α levels in said LPS cell culture.

In a more particular embodiment said cells are human peripheral blood mononuclear cells. In another more particular embodiment said compound cell culture and said LPS cell culture are incubated at about 37 °C.

In another more particular embodiment said compound cell culture and said LPS cell culture are incubated for at least about 2 hours, or at least about 5 hours, or at least about 10 hours, or at least about 15 hours, or at least about 18 hours, or about 18 hours.

In another more particular embodiment, wherein said cells are human peripheral blood mononuclear cells; and said compound cell culture and said LPS cell culture are incubated for at least about 2 hours, or at least about 5 hours, or at least about 10 hours, or at least about 15 hours, or at least about 18 hours, or about 18 hours, said cell culture is incubated at about 37 °C.

In another more particular embodiment said step of monitoring for increased TNF-α levels in said compound cell culture and said LPS cell culture is done by using a primary plate bound antibody for capture followed by a secondary biotinylated anti-TNF antibody. In a more particular embodiment said secondary biotinylated anti-TNF antibody is detected using streptavidin-Europium. In a more particular embodiment thereof increased TNF-α levels in said compound cell culture and said LPS cell culture are identified by an increased europium count. In still a more particular embodiment said increased europium count is identified by time resolved fluorescence.

One embodiment of the invention provides a high throughput assay for identifying small molecule immunomodulators, said assay comprising:
a) contacting a plurality of test compounds with cells to form one or more test solution(s);
b) incubating said test solution for at least 30 minutes;
c) measuring for an increased level of one or more immunological markers in said test solution;
wherein immunomodulation by one or more test compounds present in said plurality of test compounds causes an increase in the amount of said immunological markers in said test solution.

In a more particular embodiment, said small molecule immunomodulators are SMIPs and said immunomodulation is immunopotentiation. A more particular embodiment provides the high throughput assay further comprising the step of comparing said amount of immunological markers in said test solution with an unstimulated solution, devoid of any test compounds. More particularly, said unstimulated solution is run in parallel with said test solution.

In a more particular embodiment, the high throughput assay further comprises the step of comparing said amount of immunological markers in said test solution with a stimulated solution containing a known immunopotentiating agent. Further still, said stimulated solution is run in parallel with said test solution. In another more particular embodiment, said immunopotentiating agent is selected from the group consisting of LPS, CpG, resiquimod, Poly I:C (dsRNA), Pam3-Cys, MPL, and anti-CD3.

In another more particular embodiment said immunological markers are cytokines. In another more particular embodiment said immunological markers are chemokines. In another more particular embodiment said immunological markers are growth factors. In another more particular embodiment said immunological markers are both cytokines and chemokines. In another more particular embodiment said immunological markers are both cytokines and growth factors. In another more particular embodiment said immunological markers are both chemokines and growth factors. In another more particular embodiment said immunological markers are cytokines, chemokines and growth factors.

In another more particular embodiment said immunological marker is TGF-beta, said small molecule immunomodulators are SMIS, and said immunomodulation is immunosuppression.

In another embodiment of the high throughput assay, an initial step of providing said plurality of test compounds bound to one or more support resins is provided. In a more particular embodiment said plurality of test compounds are cleaved from their support resins before the step of contacting them with cells. In another more particular embodiment said support resins are beads and said beads are distributed such that a single bead is distributed in separate wells of a multi-well plate. In another more particular embodiment each bead contains between 20 and 100 test compounds. In another more particular embodiment, the high throughput screen further comprises the step of comparing said amount of immunological markers in said test solution with a stimulated solution containing a known immunopotentiating agent. In another more particular embodiment said stimulated solution is run in parallel with said test solution. In another more particular embodiment said immunopotentiating agent is selected from the group consisting of LPS, CpG, resiquimod, Poly I:C (dsRNA), Pam3-Cys, MPL, and anti-CD3. In another more particular embodiment the high throughput assay further provides the step of comparing said amount of immunological markers in said test solution with an unstimulated solution, devoid of any test compounds. In another more particular embodiment said unstimulated solution is run in parallel with said test solution. In another more particular embodiment said immunological markers are cytokines. In another more particular embodiment said immunological markers are chemokines. In another more particular embodiment said immunological markers are growth factors. In another more particular embodiment said immunological markers are both cytokines and chemokines. In another more particular embodiment said immunological markers are both cytokines and growth factors. In another more particular embodiment said immunological markers are both chemokines and growth factors. In another more particular embodiment said immunological markers are cytokines, chemokines and growth factors.

In another a more particular embodiment said test solution is incubated for at least 1 hour, or 2 hours, or 3 hours, or 4 hours, or 5 hours, or 6 hours, or 10 hours, or 15 hours, or 18 hours.

In another a more particular embodiment said test compounds have a molecular weight of less than 700 g/mol.

In another a more particular embodiment said immunologi calmarkers are detected by fluorescent dyes bound to capture antibodies.

In another a more particular embodiment said cytokines are selected from the group consisting of IL 1-26, TNF-alpha, TNF-beta, IFN-alpha, IFN-beta and IFN-gamma.

In another a more particular embodiment said cytokines are selected from the group consisting of IL-1b, IL-2, IL-4, IL-5, IL-6, IL-10, IL-12, and IL-13.

In another a more particular embodiment said chemokines are selected from the group consisting of CXCL 1-16, XCL 1-2, CX₃CL 1, and CCL 1-28.

In another a more particular embodiment said growth factors are selected from the group consisting of GM-CSF, G-CSF and M-CSF.

In another a more particular embodiment said plurality of test compounds are distributed in a multi-well plate such that one species of test compound is distributed per well.

In another more particular embodiment said plurality of test compounds are distributed in a multi-well plate such that more than one species of test compound is distributed per well. Alternatively, said plurality of test compounds are added to one or more vessels such that more than one species of test compound exists in each vessel.

In another a more particular embodiment identification of said small molecule immunomodulator is performed by successively screening an incrementally smaller subset of said plurality of test compounds contained within wells showing immunomodulatory activity until the small molecule immunomodulator is isolated.

In another a more particular embodiment said plurality of test compounds refers to at least 20 distinct test compound species.

In another a more particular embodiment said cells are human PBM cells or mu-splenocytes.

Another embodiment of the invention provides a high throughput assay for identifying small molecule immunomodulators, said assay comprising:
a) contacting a plurality of test compounds with cells to form one or more test solution(s);
b) adding a known immunopotentiating agent to said test solution and to a control solution devoid of said test compounds;
c) incubating said test solution and said control solution for at least 1 hour;
d) measuring levels of one or more immunological markers in said test solution and said control solution;
e) comparing said levels of one or more immunological markers in said test solution with corresponding levels of one or more immunological markers in said control solution to detect a difference in the levels of one or more immunological markers between said test solution and said control solution;
wherein a decrease in the level of one or more of the immunological markers in the test solution indicates immunosuppression by one or more of the test compounds and an increase in the level of one or more of the immunological markers in the test solution indicates immunopotentiation by one or more of the test compounds.

In another more particular embodiment said immunopotentiating agent is added in a sub-optimal concentration, such that said immunological markers are only partially stimulated.

Another embodiment provides the high throughout screen further comprising the step of comparing said amount of immunological markers in said test solution with an unstimulated solution, devoid of any test compounds or immunopotentiating agents. Further still, said unstimulated solution is run in parallel with said test solution. In another embodiment said immunopotentiating agent is selected from the group consisting of LPS, CpG, resiquimod, Poly I:C (dsRNA), Pam3-Cys, MPL, and anti-CD3.

In another embodiment, said immunological markers are cytokines. In another more particular embodiment said immunological markers are chemokines. In another more particular embodiment said immunological markers are growth factors. In another more particular embodiment said immunological markers are both cytokines and chemokines. In another more particular embodiment said immunological markers are both cytokines and growth factors. In another more particular embodiment said immunological markers are both chemokines and growth factors. In another more particular embodiment said immunological markers are cytokines, chemokines and growth factors.

In another a more particular embodiment said test solution is incubated for at least 1 hour, or 2 hours, or 3 hours, or 4 hours, or 5 hours, or 6 hours, or 10 hours, or 15 hours, or 18 hours.

In another a more particular embodiment said test compounds have a molecular weight of less than 700 g/mol.

In another a more particular embodiment said immunological markers are detected by fluorescent dyes bound to capture antibodies.

In another a more particular embodiment said cytokines are selected from the group consisting of IL 1-26, TNF-alpha, TNF-beta, IFN-alpha, IFN-beta and IFN-gamma.

In another a more particular embodiment said cytokines are selected from the group consisting of IL-1b, IL-2, IL-4, IL-5, IL-6, IL-10, IL-12, and IL-13.

In another a more particular embodiment said chemokines are selected from the group consisting of CXCL 1-16, XCL 1-2, CX₃CL 1, and CCL 1-28.

In another a more particular embodiment said growth factors are selected from the group consisting of GM-CSF, G-CSF and M-CSF.

In another a more particular embodiment said plurality of test compounds are distributed in a multi-well plate such that one species of test compound is distributed per well.

In another more particular embodiment said plurality of test compounds are distributed in a multi-well plate such that more than one species of test compound is distributed per well. Alternatively, said plurality of test compounds are added to one or more vessels such that more than one species of test compound exists in each vessel.

In another a more particular embodiment identification of said small molecule immunomodulator is performed by successively screening an incrementally smaller subset of said plurality of test compounds contained within wells showing immunomodulatory activity until the small molecule immunomodulator is isolated.

In another a more particular embodiment said plurality of test compounds refers to at least 20 distinct test compound species.

In another a more particular embodiment said cells are human PBM cells or mu-splenocytes.

In another embodiment of the high throughput assay, an initial step of providing said plurality of test compounds bound to one or more support resin(s) is provided. In a more particular embodiment said plurality of test compounds are cleaved from their support resins before the step of contacting them with cells. In another more particular embodiment said support resins are beads and said beads are distributed such that a single bead is distributed in each well. In another more particular embodiment each bead contains between 20 and 100 test compounds. In another more particular embodiment, the high throughput screen further comprises the step of comparing said amount of immunological markers in said test solution with a stimulated solution containing a known immunopotentiating agent. In another more particular embodiment said stimulated solution is run in parallel with said test solution. In another more particular embodiment said immunopotentiating agent is selected from the group consisting of LPS, CpG, resiquimod, Poly I:C (dsRNA), Pam3-Cys, MPL, and anti-CD3. In another more particular embodiment the high throughput assay further provides the step of comparing said amount of immunological markers in said test solution with an unstimulated solution, devoid of any test compounds. In another more particular embodiment said unstimulated solution is run in parallel with said test solution. In another more particular embodiment said immunological markers are cytokines. In another more particular embodiment said immunological markers are chemokines. In another more particular embodiment said immunological markers are growth factors. In another more particular embodiment said immunological markers are both cytokines and chemokines. In another more particular embodiment said immunological markers are both cytokines and growth factors. In another more particular embodiment said immunological markers are both chemokines and growth factors. In another more particular embodiment said immunological markers are cytokines, chemokines and growth factors.

Another embodiment of the invention provides a high throughput assay for identifying a small molecule immunomodulator, said method comprising:
a) providing a mixture of test compounds divided into an archive portion and a screening portion from a combinatorial library, wherein (i) said mixture comprises a plurality of resin support beads having test compounds attached thereto, and (ii) each said bead has only one discrete test compound at a concentration greater than 1 nmol attached thereto;
b) contacting said screening portion with cells;
c) measuring for increased levels of immunological markers in said screening portion, wherein immunomodulation by one or more test compounds present in said screening portion causes an increase in the amount of said immunological markers;
d) individually distributing beads from the archive portion in step (a) into a plurality of reaction vessels such that each vessel contains a single bead;
e) cleaving the test compounds from the beads and separating said beads from the cleaved test compounds, thereby providing discrete samples of individual test compounds;
f) screening each cleaved test compound for identification of a small molecule immunomodulator;
g) performing a mass spectrometric analysis on said small molecule immunomodulator; and
h) deriving the chemical identity of said small molecule immunomodulator from said reserved portion using said mass spectrometric analysis.

In another a more particular embodiment said screening portion is incubated for at least 2 hours.

In a more particular embodiment said small molecule immunomodulator is a SMIP and said immunomodulation is immunopotentiation. In another embodiment, the high throughput assay, further comprising the step of comparing said amount of immunological markers in said screening portion with an unstimulated solution, devoid of any test compounds. In another more particular embodiment said unstimulated solution is run in parallel with said screening portion.

Another embodiment provides the high throughput assay, further comprising the step of comparing said amount of immunological markers in said screening portion with a stimulated solution containing a known immunopotentiating agent. In a more particular embodiment said stimulated solution is run in parallel with said screening portion. In a more particular embodiment said immunopotentiating agent is selected from the group consisting of LPS, CpG, resiquimod, Poly I:C (dsRNA), Pam3-Cys, MPL, and anti-CD3.

In a more particular embodiment said immunological markers are cytokines. In another a more particular embodiment said immunological markers are chemokines. In another a more particular embodiment said immunological markers are growth factors. In another a more particular embodiment said immunological markers are both cytokines and chemokines. In another a more particular embodiment said immunological markers are both cytokines and growth factors. In another a more particular embodiment said immunological markers are both chemokines and growth factors. In another a more particular embodiment said immunological markers are cytokines, chemokines and growth factors.

In another a more particular embodiment said immunological marker is TGF-beta, said small molecule immunomodulators are SMIS, and said immunomodulation is immunosuppression.

In another a more particular embodiment said cells are human PBM cells or mu-splenocytes.

In another a more particular embodiment said test solution is incubated for at least 1 hour, or 2 hours, or 3 hours, or 4 hours, or 5 hours, or 6 hours, or 10 hours, or 15 hours, or 18 hours.

In another a more particular embodiment said test compounds have a molecular weight of less than 700 g/mol.

In another a more particular embodiment said immunological markers are detected by fluorescent dyes bound to capture antibodies.

In another a more particular embodiment said cytokines are selected from the group consisting of IL 1-26, TNF-alpha, TNF-beta, IFN-alpha, IFN-beta and IFN-gamma.

In another a more particular embodiment said cytokines are selected from the group consisting of IL-1b, IL-2, IL-4, IL-5, IL-6, IL-10, IL-12, and IL-13.

In another a more particular embodiment said chemokines are selected from the group consisting of CXCL 1-16, XCL 1-2, CX₃CL 1, and CCL 1-28.

In another a more particular embodiment said growth factors are selected from the group consisting of GM-CSF, G-CSF and M-CSF.

In another a more particular embodiment said plurality of test compounds are distributed in a multi-well plate such that one species of test compound is distributed per well.

In another more particular embodiment said plurality of test compounds are distributed in a multi-well plate such that more than one species of test compound is distributed per well. Alternatively, said plurality of test compounds are added to one or more vessels such that more than one species of test compound is distributed per vessel.

In another a more particular embodiment identification of said small molecule immunomodulator is performed by successively screening an incrementally smaller subset of said plurality of test compounds contained within wells showing immunomodulatory activity until the small molecule immunomodulator is isolated.

In another a more particular embodiment said plurality of test compounds refers to at least 20 distinct test compound species.

It should be understood that the organic compounds described herein may exhibit the phenomenon of tautomerism. It should be understood that the invention encompasses any tautomeric form of the drawn structure. The compounds comprise asymmetrically substituted carbon atoms. Such asymmetrically substituted carbon atoms can result in the compounds comprising mixtures of stereoisomers at a particular asymmetrically substituted carbon atom or a single stereoisomer. As a result, racemic mixtures, mixtures of diastereomers, as well as single diastereomers of the compounds are included in the present invention. The terms "S" and "R" configuration, are as defined by the IUPAC 1974 Recommendations for Section E, Fundamental Stereochemistry, Pure Appl. Chem. (1976) 45, 13-30. The terms α and β are employed for ring positions of cyclic compounds. The α-side of the reference plane is that side on which the preferred substituent lies at the lowered numbered position. Those substituents lying on the opposite side of the reference plane are assigned β descriptor. It should be noted that this usage differs from that for cyclic stereoparents, in which "α" means "below the plane" and denotes absolute configuration. The terms α and β configuration,, are as defined by the Chemical Abstracts Index Guide-Appendix IV (1987) paragraph 203.

The SMIP compounds can be used with or without an antigen in therapeutic applications, for example to treat cancer or infectious diseases. The SMIP compounds also may be used in combination with other therapeutic agents, such as anti-virals and monoclonal antibodies in different therapeutic applications.

One preferred embodiment of the method of inducing an immunostimulatory effect in a patient is directed to administering an immunogenic composition comprising a vaccine in an amount effective to stimulate an immune response such as a cell-mediated immune response and, as a vaccine adjuvant, a SMIP compound, in an amount effective to potentiate the immune response such as the cell-mediated immune response to the vaccine.

It is contemplated that a vast number of disorders can be treated with the SMIP compounds and compositions of the present invention. Preferred methods of the invention include SMIP compounds as single agents or in combination with (an)other agent(s), to treat diseases including bacterial diseases, mycotic diseases, viral diseases, malignant tumors, hyperlipemias, and ischemic heart diseases; for example, digestive diseases, circulatory organs' diseases, urinary/genital organs' diseases, immune diseases, cranial nerve diseases, eye diseases, skin diseases, and diseases of nose, ear and throat.

Examples of such diseases susceptive to the SMIP compounds and combinations are bacterial diseases such as bacterial corneal ulcer, bacterial conjunctivitis, bacterial food poisoning, septic shock, endotoxin shock, bacterial endocarditis, bacterial meningitis, bacterial pneumonia, bacterial aneurysm, and bacterial cerebral aneurysm; viral diseases such as fungal meningitis, fungal corneal ulcer, fungal skin diseases, candidiasis, and tinea; viral diseases such as viral gastroenterocolitis, viral hepatitis, viral bronchitis, viral colon inflammatory, viral myocarditis, viral meningitis, viral enterocolitis, viral encephalitis, viral pneumonia, and AIDS; massive malignant tumors such as renal cell carcinoma, mycosis fungoides, and chronic granuloma; blood malignant tumors such as colonic cancer, rectal cancer, carcinoma of the colon and rectum, gastric cancer, thyroid carcinoma, cancer of the tongue, bladder carcinoma, cilium carcinoma, hepatoma, prostatic cancer, carcinoma uteri, cancer of pharynx, lung cancer, breast cancer, malignant melanoma, Kaposi's sarcoma, brain tumor, neuroblastoma, ovarian tumor, testicular tumor, pancreatic tumor, renal cancer, hypernephroma, hemangioendothelioma, adult T-cell leukemia (ATL), chronic myelogenous leukemia (CML), and malignant lymphoma; autoimmune-, allergic- and viral-diseases such as active chronic hepatitis, atrophic gastritis, autoimmune hemolytic anemia, Basedow disease, Behcet's syndrome, Crohn's disease, CRST syndrome, cold agglutinin hemolytic anemia, idiopathic ulcerative colitis, Goodpasture's syndrome, hyperthyroidism, chronic thyroiditis, inflammation of pulmonary alveoli, glomerulonephritis, idiopathic thrombocytopenic purpura, juvenile diabetes mellitus, insulin dependent diabetes mellitus, leukopenia, multi sclerosis, myasthenia gravis, paroxysmal cold hemoglobinuria, pernicious anemia, polyarteritis nodosa, polymyositis, primary biliary cirrhosis, rheumatic fever, rheumatoid arthritis, Sjögren's syndrome, sympathetic ophthalmia, progressive systemic sclerosis, Wegener granulomatosis, asthma, atopic dermatitis, bronchial asthma, graft-versus-host disease, allergic rhinitis, pollinosis. and allergy for bee's toxic; hepatic diseases such as alcoholic hepatitis, toxic hepatitis, viral cirrhosis, alcoholic cirrhosis, toxic cirrhosis, biliary cirrhosis, fatty liver, hepatic tumor, and hepatic vascular disorder; gallbladder/biliary tract diseases such as cholangitis, cholecystitis, primary clerosing cholangitis, gallbladder tumor, and cancer of the bile duct; pancreatic diseases such as acute insufficiency, .pancreatic tumor, and pancreatic cysts; circulatory organs' diseases such as ischemia, ischemic heart disease, cerebral ischemia, basilar artery migraine, abnormal vasculamet at the brain base, cerebral apoplexy, aneurysm of the brain bas e, arteriosclerosis, vascular endothelial disorder, noninsulin-dependent diabetes mellitus, occlusion of the mesenteric vessel, and superior mesenteric artery syndrome; nerve diseases such as Parkinson's disease, spinal atrophy, amyotrophic lateral sclerosis, Alzheimer's disease, dementia, cerebrovascular dementia, AIDS dementia, and Meningitis; digestive diseases such as peptic ulcer, peptic esophagus ulcer, intestinal polyp, intestinal adhesion, intestinal rigidity, and gastric ulcer; sleep disturbances caused by the incidence of mental diseases, central nervous system depressants, habitual alcohols, and the disorder of respiratory system; and other diseases induced by side effects accompanied by the administration of hypnotics.

Agents combined with the SMIP compounds, contemplated to be useful in treating the aformentioned diseases include those well known in the art, such as, anesthetics, hypnotic sedatives, anti-anxieties, antiepileptics, antipyretic antiphlogistics, stimulants, wake amines, anti-parkinson drugs, agents for psychoneuroses, agents for central nervous system, skeletal muscle relaxants, agents for autonomic nervous system, antispastic agents, cytotoxic agents, monoclonal antibodies, drugs for eye, drugs for nose and ear, anti-vertiginous drugs, cardiotonics, antiarrithythmic drugs, diuretics, pressure reduction drugs, vasoconstrictors, coronary vaso-dilators, peripheral vasodilating drugs, hyper-lipemia drugs, breath stimulants, antitussive and expectorant drugs, bronchodilators, drugs for allergy, antidiarrheal drugs, drugs for intestinal disorders, peptic ulcer drugs, stomachic digestants, antacids, cholagogouses, pituitary hormone drugs, salivary gland hormones, thyroid hormone drugs, antithyroid drugs, anabolic steroids, corticosteroids, androgen drugs, estrogen drugs, corpus luteum hormone drugs, mixed hormones, urinary/genital organ drugs, anus drugs, surgical sterilizations/antiseptics, wound protectives, externals for purulent diseases, analgesics, antipruritics, astringents, antiphlogistics, externals for parasite skin diseases, skin-softening drugs, caustics, dental/oral drugs, vitamins, inorganic preparations, supplemental liquids, hemostatics, anticoagulation drugs, drugs for liver diseases, antidotes, habitual intoxication drugs, drugs for treatment of gout, enzyme preparations, diabetic drugs, antioncotics, antihistaminics, drugs for stimulation treatment, antibiotics, chemotherapeutics, biological preparations, anthelmintics, anti-Protozoas, drugs for preparations, X-ray contrast media, and diagnostic drugs.

Further methods of the invention are provided wherein compositions described herein are used for the treatment of cancer and reduction of tumor growth. In one aspect a SMIP compound of the invention is combined with a known MAb for the treatment of cancer. In a presently preferred aspect of this embodiment of the present invention, an antibody and a SMIP compound are administered. It may be particularly preferred that said antibody, individually, has an inhibiting effect upon tumor cell growth and that the SMIP compound induces the production of cytokines.

In accordance with another embodiment of the present invention, there is provided a therapeutic composition for inhibiting tumor cell growth in a subject, which composition comprises an effective amount of a combination of at least a SMIP compound and a MAb and a pharmaceutically acceptable carrier, wherein said combination is more effective to inhibit growth of certain mammalian tumor cells than are either of the agents when administered individually.

In another embodiment methods of treating cancer are provided wherein known anticancer agents are combined with SMIP compounds to reduce tumor growth in a subject. A number of suitable anticancer agents are contemplated for use in the methods of the present invention. Indeed, the present invention contemplates, but is not limited to, administration of numerous anticancer agents such as: agents that induce apoptosis; polynucleotides (e.g., ribozymes); polypeptides (e.g., enzymes); drugs; biological mimetics; 25 alkaloids; alkylating agents; antitumor antibiotics; antimetabolites; hormones; platinum compounds; monoclonal antibodies conjugated with anticancer drugs, toxins, and/or radionuclides; biological response modifiers (e.g. interferons [e.g. IFN-a, etc.] and interleukins [e.g. IL-2, etc.], etc.); adoptive immunotherapy agents; hematopoietic growth factors; agents that induce tumor cell differentiation (e.g. all-trans-retinoic acid, etc.); gene 30 therapy reagents; antisense therapy reagents and nucleotides; tumor vaccines; and inhibitors of angiogenesis, and the like. Numerous other examples of chemotherapeutic compounds and anticancer therapies suitable for coadministration with the disclosed SMIP compounds are known to those skilled in the art.

In preferred embodiments, anticancer agents comprise agents that induce or stimulate apoptosis. Agents that induce apoptosis include, but are not limited to, radiation (e.g., W); kinase inhibitors (e.g., Epidermal Growth Factor Receptor [EGFR] kinase; inhibitor, Vascular Growth Factor Receptor [VGFR] kinase inhibitor, Fibroblast Growth 5 Factor Receptor [FGFR] kinase inhibitor, Platelet-derived Growth Factor Receptor [PGFR] I kinase inhibitor, and Bcr-Abl kinase inhibitors such as STI-571, Gleevec, and Glivec]); antisense molecules; antibodies [e.g., Herceptin and Rituxan]; anti-estrogens [e.g., raloxifene and tamoxifen]; antiandrogens [e.g., flutamide, bicalutamide, finasteride, aminoglutethamide, ketoconazole, and corticosteroids]; cyclooxygenase 2 (COX-2) inhibitors [e.g., Celecoxib, meloxicam, NS-398, and non-steroidal
antiinflammatory drugs I (NSAIDs)]; and cancer chemotherapeutic drugs [e.g.,
irinotecan (Camptosar), CPT-11, fludarabine (Fludara), dacarbazine (DTIC), dexamethasone, mitoxantrone, Mylotarg, VP-; 16, cisplatinum, 5-FU,
Doxrubicin, Taxotere or taxol]; cellular signaling molecules; ceramides and cytokines; and staurosprine, and the like.

In another embodiment methods of treating allergies are provided comprising administering a SMIP compound alone or in combination with at one other agent known to be effective against allergies, wherein said combination is more effective in treating an allergic condition than the know agent(s) are without the addition of said SMIP compound. In a more preferred embodiment the known agent is antihistamine and/or leukotriene inhibitor. In another preferred embodiment, the allergic condition is asthma. In another preferred embodiment, the allergic condition is selected from the group consisting of allergic rhinitis, dermatosis, and urticaria. In an even more preferred embodiment the combination is administered to a subject enterally, parenterally, intranasally, subcutaneously, or intraarterially.

In another embodiment, the present invention provides methods of screening a SMIP compound and a test compound comprising: providing a SMIP compound; a test compound; a first group of cells; and contacting the first group of cells with the SMIP compound and the test compound; and observing the effects of contacting the first group of cells with the SMIP compound and the test compound. In some of these embodiments, the present invention further provides the additional step of comparing the effects observed in the first cells against a second group of the cells contacted with the SMIP compound alone, or with the test compound alone. Effects that may be observed include, but are not limited to, changes in cell proliferation, changes in TNF alpha levels, changes in infected viral content of a cell, changes in bacterial infection levels in a cell, changes in histamine levels of a cell, changes in apoptotic stats, and changes in the expression of Bcl-2 family proteins, and the like.

In another embodiment methods of manufacturing compounds and compositions described herein are provided and contemplated to fall within the scope of the invention.

Qualitative and quantitative measurement of the immune response of a compound or composition can be implemented using methods known in the art, such as measuring antigen specific antibody production, activation of specific populations of lymphocytes such as CD4⁺ , CD8+ T cells or NK cells, and/or production of cytokines such as IFN, IL-2, IL-4 or IL-12. Methods for measuring specific antibody responses include enzyme-linked immunosorbent assay (ELISA) as known in the art. Measurement of numbers of specific types of lymphocytes such as CD4⁺ T cells can be achieved, for example, with fluorescence-activated cell sorting (FACS). Cytotoxicity assays can be performed, e.g., as described in Raz et al., (1994) Proc. Natl. Acad. Sci. USA 91:9519-9523. Serum concentrations of cytokines can be measured, for example, by ELISA. Such assays are described, e.g., in Selected Methods in Cellular Immunology (1980) Mishell and Shiigi, eds., W.H. Freeman and Co.

In one embodiment, a compound or composition, such as a SMIP compound, is considered effective to elicit an immune response if a concentration of 20 µM (or alternatively 100 µM, or 200µM, or 300µM) of the SMIP compound causes the production of TNF-α in an in vitro cell based assay of human peripheral blood mononuclear cells, wherein the concentration of the human peripheral blood mononuclear cells is about 500,000/mL, and wherein the cells are exposed to the compound for about 18-24 hours, e.g., about 24 hours.

The above method of stimulating a local immune response for example in selected cells or tissues of a patient includes the stimulation of a local immune response wherein the selected cells or tissues are infected or cancerous. In one embodiment the selected cells or tissues are infected with a fungus or bacterium. In another embodiment the selected tissues are inflamed with an allergen, for example in an asthmatic condition. In another embodiment the selected cells are infected with a virus or bacteria. In still a more particular embodiment the infectious agent is HCV, HIV, HBV, HSV, H. pylori, HSV Type 1 or 2, or Human Papilloma Virus.

The methods and compounds disclosed herein can be used generally for the treatment of asthma by steering the immune response away from Type 2 cytokine secretion and effector mechanisms (e.g. IgE production and/or mast cell/basophil activation).

The immunogenic compositions of the invention can contain further pharmaceutically acceptable ingredients, excipients, carriers, and the like well known to those skilled in the art.

The vaccine composition may include an additional adjuvant. Preferred adjuvants to enhance effectiveness of the composition include, but are not limited to:
(1) aluminum salts (alum), such as aluminum hydroxide aluminum phosphate, aluminum sulfate, etc; (2) oil-in-water emulsion formulations (with or without specific immunostimulating agents such as muramyl peptides or bacterial cell wall components), such as, for example (a) MF59™ (WO90/14837), containing 5% squalene, 0.5% Tween 80, and 0.5% Span 85 (optionally containing MTP-PE) formulated into submicron particles using a microfluidizer, (b) SAF, containing 5% squalene, 0.5% Tween 80, 5% pluronic-blocked polymer L121, and thr-MDP either microfluidized into a submicron emulsion or vortexed to generate a larger particle size emulsion, and (c) Ribi ™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox ™); (3) saponin adjuvants, such as QS21 or Stimulon™ (Cambridge Bioscience, Worcester, MA) may be used or particles generated therefrom such as ISCOMs (immunostimulating complexes), which ISCOMs may be devoid of additional detergent e.g. WO00/07621; (4) Complete Freund's Adjuvant (CFA) and Incomplete Freund's Adjuvant (IFA); (5) cytokines, such as interleukins (e.g. IL-1, IL-2, IL-4, IL-5, IL-6, IL-7, IL-12 (WO99/44636), etc.), interferons (e.g. gamma interferon), macrophage colony stimulating factor (M-CSF), tumor necrosis factor (TNF), etc.; (6) momophosphoryl lipid A (MPL) or 3-O-deacylated MPL (3dMPL), optionally in the substantial absence of alum when used with pneumococcal saccharides e.g. WO00/56358; and RC529 (7) combinations of 3dMPL with, for example, QS21 and /or oil-in-water emulsions e.g. EP-A-0835318; (8) oligonucleotides comprising CpG motifs, i.e. containing at least one CG dinucleotide, with 5-methylcytosine optionally being used in place of cytosine; (9) a polyoxyethylene ether or a polyoxyethylene ester e.g. WO99/52549; (10) a polyoxyethylene sorbitan ester surfactant in combination with an octoxynol (WO0121207) or a polyoxyethylene alkyl ether or ester surfactant in combination with at least one additional non-ionic surfactant such as an octoxynol (WO01/21152); (11) a saponin and an immunostimulatory oligonucleotide (e.g. a CpG oligonucleotide) (WO00/62800); (12) an immunostimulant and a particle of metal salt e.g WO00/23105; (13) a saponin and an oil-in-water emulsion e.g. WO99/11241; (14) a saponin (e.g. QS21) + 3dMPL +IL-12 (optionally + a sterol) e.g. WO98/57659; (14) other substances that act as immunostimulating agents to enhance the effectiveness of the composition. In one particular embodiment, Alum (especially aluminium phospate and/or hydroxide) and MF59 are preferred for use with saccharide antigens.

The invention is also directed to administering the immunogenic composition. The vaccine is administered in an amount effective to stimulate an immune response. The amount that constitutes an effective amount depends, inter *alia,* on the particular vaccine used, the particular adjuvant compound being administered and the amount thereof, the immune response that is to be enhanced (humoral or cell mediated), the state of the immune system (e.g., suppressed, compromised, stimulated), and the desired therapeutic result. Accordingly it is not practical to set forth generally the amount that constitutes an effective amount of the vaccine. Those of ordinary skill in the art, however, can readily determine the appropriate amount with due consideration of such factors.

The immunogenic compositions of the invention can be administered to animals, e.g., mammals human and non-human, including, for example, pocket pets, fowl, and the like according to conventional methods well known to those skilled in the art (e.g., orally, subcutaneously, nasally, topically).

Suitable vaccines include, but are not limited to, any material that raises either humoral or cell mediated immune response, or both. Suitable vaccines include live viral and bacterial antigens and inactivated viral, tumor-derived, protozoal, organism-derived, fungal, and bacterial antigens, toxoids, toxins, polysaccharides, proteins, glycoproteins, peptides, and the like. Conventional vaccines, such as those used in connection with BCG (live bacteria), cholera, plague, and typhoid (killed bacteria), hepatitis B, influenza, inactivated polio, and rabies (inactivated virus), measles, mumps, rubella, oral polio, and yellow fever (live virus), tetanus and diphtheria (toxoids), hemophilus influenzae b, meningococcal, and pneumococcal (bacterial polysaccharides) also can be used. Any antigen known in the art or disclosed herein may be used.

Furthermore, it is contemplated that certain currently experimental vaccines, especially materials such as recombinant proteins, glycoproteins, and peptides that do not raise a strong immune response, will also find use in connection with the SMIP compound. Exemplary experimental subunit antigens include those related to viral disease such as adenovirus, AIDS, chicken pox, cytomegalovirus, dengue, feline leukemia, fowl plague, hepatitis A, hepatitis B, hepatitis C, HSV-1, HSV-2, hog cholera, influenza A, influenza B, Japanese encephalitis, measles, parainfluenza, rabies, respiratory syncytial virus, rotavirus, wart, and yellow fever.

Specific antigens for use with the invention include, but are not limited to, those listed below. The number(s) in parenthesis indicate representative resources of the antigen. The resource list follows the antigen list and each resource is incorporated by reference in its entirety.

Specific antigens include: a protein antigen from *N. meningitides* serogroup B (1-7); an outer-membrane vesicle (OMV) preparation from *N*. *meningitides* serogroup B. (8, 9, 10, 11); a saccharide antigen from *N. meningitides* serogroup A, C W135 and/or Y, such as the oligosaccharide (12) from serogroup C (13); a saccharide antigen from *Streptocaccus pneumoniae* (14, 15, 16); an antigen from *N. gonorrhoeae* (1, 2, 3); an antigen from *Chlamydia pneumoniae* (17, 18, 19, 20, 21, 22, 23); an antigen from *Chlamydia trachomatis* (24); an antigen from hepatitis A virus, such as inactivated virus (25, 26); an antigen from hepatitis B virus, such as the surface and/or core antigens (e.g. 26, 27); an antigen from hepatitis C virus (28); an antigen from *Bordetella pertussis,* such as petussis holotoxin (PT) and filamentous haemagglutinin (FHA) from *B. pertussis,* optionally also combination with pertactin and/or agglutinogens 2 and 3 (29, 30); a diphtheria antigen, such as a diphtheria toxoid (31:chapter 3) e.g. the CRM₁₉₇ mutant (32); a tetanus antigen, such as a tetanus toxoid (31 :chapter 4); a protein antigen from Helicobacter pylori such as CagA (33), VacA (33), NAP (34), HopX (5), HopY (35) and/or urease; a saccharide antigen from Haemophilus influenzae B (13); an antigen from Porphyromonas gingivalis (36); polio antigen(s) (37, 38) such as IPV or OPV; rabies antigen(s) (39) such lyophilized inactivated virus (40, RabAvert™); measles, mumps and/or rubella antigens (31: chapters 9, 10, & 11); influenza antigen(s) (31:chapter 19), such as the haemagglutinin and/or neuraminidase surface proteins; an antigen from Moraxella catarrhalis (41); an antigen from Streptococcus agalactiae (group B streptococcus) (42, 43); an antigen from Streptococcus pyogenes (group A streptococcus) (43, 44, 45); and an antigen from Staphylococcus aureus (46).

The composition may comprise one or more of the above antigens.

Where a saccharide or carbohydrate antigen is used, it is preferably conjugated to a carrier protein in order to enhance antigenicity (47-56). Preferred carrier proteins are bacterial toxine or toxoids, such as diphtheria or tetanus toxoids. The CRM₁₉₇ diphtheria toxoid is particularly preferred. Other suitable carrier proteins include the *N. meningitides* outer membrane protein (57), synthetic peptides (58, 59), heat shock proteins (60), pertussis proteins (61, 62), protein D from *H. influenzae* (63), toxin A or B from C. *difficile* (64) etc. Where a mixture comprises capsular saccharides from both serogroups A and C, it is preferred that the ratio (w/w) of MenA saccharide:MenC saccharide is greater than 1 (e.g. 2:1, 3:1, 4:4, 5:1, 10:1 or higher). Saccharides from different serogroups of N. meningitides may be conjugated to the same or different carrier proteins.

Any suitable conjugation reaction can be used, with any suitable linker where necessary. Toxic protein antigens may be detoxified where necessary (e.g. detoxification of pertussis toxin by chemical and/or genetic means (30)). Where a diphtheria antigen is included in the composition it is preferred also to include tetanus antigens and pertussis antigens. Similar, where a tetanus antigen is included it is preferred also to include diphtheria and pertussis antigens. Similar, where pertussis antigen is included it is preferred also to include diphtheria and tetanus antigens.

The pharmaceutical compositions containing the compounds described herein can include additives such as excipients. Suitable pharmaceutically acceptable excipients include processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-β-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991), incorporated herein by reference.

Pharmaceutical compositions containing the compounds of the invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, and the like, as well as mixtures of two or more there of. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols. Suitable oils include, for example, soybean oil, coconut oil, olive oil, safflower oil, cottonseed oil, and the like. For parenteral administration, the carrier can also be an oily ester such as ethyl oleate, isopropyl myristate, and the like. Compositions of the present invention may also be in the form of microparticles, microcapsules, and the like, as well as combinations of any two or more thereof.

The compounds and combinations of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N.W., p. 33 et seq (1976).

Other additives include immunostimulatory agents known in the art. Immunostimulatory oligonucleotides and polynucleotides are described in PCT WO 98/55495 and PCT WO 98/16247. U.S. Patent Application No. 2002/0 164341 describes adjuvants including an unmethylated CpG dinucleotide (CpG ODN) and a non-nucleic acid adjuvant. U.S. Patent Application No. 2002/0197269 describes compositions comprising an antigen, an antigenic CpG-ODN and a polycationic polymer. Other immunostimulatory additives described in the art may be used, for example, as described in U.S. Patent No. 5,026,546; U.S. Patent No. 4,806,352; and U.S. Patent No. 5,026,543. Additionally, SMIP compounds as described in USSN 60/458,888, which is incorporated herein by reference, are contemplated as effective co-administration agents or combination with the compositions of the instant invention. The SMIPs may serve as vaccine adjuvants as well.

A controlled release delivery system may be used, such as a diffusion controlled matrix system or an erodible system, as described for example in: Lee, "Diffusion-Controlled Matrix Systems", pp. 155-198 and Ron and Langer, "Erodible Systems", pp. 199-224, in "Treatise on Controlled Drug Delivery", A. Kydonieus Ed., Marcel Dekker, Inc., New York 1992. The matrix may be, for example, a biodegradable material that can degrade spontaneously *in situ* and *in vivo* for, example, by hydrolysis or enzymatic cleavage, e.g., by proteases. The delivery system may be, for example, a naturally occurring or synthetic polymer or copolymer, for example in the form of a hydrogel. Exemplary polymers with cleavable linkages include polyesters, polyorthoesters, polyanhydiides, polysaccharides, poly(phosphoesters), polyamides, polyurethanes, poly(imidocarbonates) and poly(phosphazenes).

The compounds of the invention may be administered enterally, orally, parenterally, sublingually, by inhalation spray, rectally, or topically in dosage unit formulations containing conventional nontoxic pharmaceutically acceptable carriers, adjuvants, and vehicles as desired. For example, suitable modes of administration include oral, subcutaneous, transdennal, transmucosal, iontophoretic, intravenous, intramuscular, intraperitoneal, intranasal, subdermal, rectal, and the like. Topical administration may also involve the use of transdermal administration such as transdermal patches or ionophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oil s are conventionally employed as a solvent or suspending medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols that are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

Effective amounts of the compounds of the invention generally include any amount sufficient to detectably treat the disorders described herein.

Successful treatment of a subject in accordance with the invention may result in the inducement of a reduction or alleviation of symptoms in a subject afflicted with a medical or biological disorder to, for example, halt the further progression of the disorder, or the prevention of the disorder.

The amount of active ingredient that may be combined with the carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy. The therapeutically effective amount for a given situation can be readily determined by routine experimentation and is within the skill and judgment of the ordinary clinician.

The compounds can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-napthalenesulfonate, oxalate, pamoate, pectinate, persulfate, 3-phenylproionate, picrate, pivalate, propionate, succinate, tartrate, thiocyanate, p-toluenesulfonate and undecanoate. Also, the basic nitrogen-containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.
Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Basic addition salts can be prepared *in situ* during the final isolation and purification of the compounds of formula (I), or separately by reacting carboxylic acid moieties with a suitable base such as the hydroxide, carbonate or bicarbonate of a pharmaceutical acceptable metal cation or with ammonia, or an organic primary, secondary or tertiary amine. Pharmaceutical acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

Other additives include immunostimulatory agents known in the art. Immunostimulatory oligonucleotides and polynucleotides are described in PCT WO 98/55495 and PCT WO 98/16247. U.S. Patent Application No. 2002/0164341 describes adjuvants including an unmethylated CpG dinucleotide (CpG ODN) and a non-nucleic acid adjuvant. U.S. Patent Application No. 2002/0197269 describes compositions comprising an antigen, an antigenic CpG-ODN and a polycationic polymer. Other immunostimulatory additives described in the art may be used, for example, as described in U.S. Patent No. 5,026,546; U.S. Patent No. 4,806,352; and U.S. Patent No. 5,026,543.

It is contemplated that the invention encompasses all possible combinations of the embodiments described herein.

### DEFINITIONS :

As used above and elsewhere herein the following terms and abbreviations have the meanings defined below:
- ATP:: Adenosine triphosphate
- BCG: Mycobacterium bovis bacillus Calmette-Guerin
- BSA:: Bovine Serum Albumin
- FHA: Filamentous haemaglutinin
- GCMS: Gas Chromatography / Mass Spectroscopy
- H. Pylori: Helicobacter Pylori
- HAV: Hepatitis A Virus
- HBV: Hepatitis B Virus
- HCV: Hepatitis C Virus
- HIV: Human Immunodeficiency Virus
- HPLC: High Performance Liquid Chromatography
- HSV: Herpes Simplex Virus
- IC₅₀ value:: The concentration of an inhibitor that causes a 50 % reduction in a measured activity.
- IFN: Interferon
- IL: Interleukin
- IMS: Immunomagnetic separation
- IPV: Inactivated polio virus
- LCMS: Liquid Chromatography / Mass Spectroscopy
- LPS: Lipopolysaccharide
- Men A: Neisseria Meningitidis Type A
- Men C: Neisseria Meningitidis Type C
- Men B: Neisseria Meningitidis Type B
- Men W: Neisseria Meningitidis Type W
- Men Y: Neisseria Meningitidis Type Y
- MeOH:: Methanol
- NANB: Non-A, non-B hepatitis
- NMR: Nuclear magnetic resonance
- OMV: Outer membrane vesicle
- PBMC: Peripheral blood mononuclear cells
- PT: Petussis holotoxin
- Rt: Room temperature (25°C)
- SMIP: Small Molecule Immune Potentiator
- SMIS: Small Molecule Immune Suppressant (or Small Molecule Immunosuppressant)
- TLC: Thin-layer chromatography
- TNF-α: Tumour necrosis factor-a

The methods of the invention are useful in treating "allergic diseases," that is accomplished in the same way as other immunotherapeutic methods described herein.

An "allergen" refers to a substance (antigen) that can induce an allergic or asthmatic response in a susceptible subject. The list of allergens is enormous and can include pollens, insect venoms, animal dander, dust, fungal spores, and drugs (e.g. penicillin).

"Asthma" refers to a disorder of the respiratory system characterized by inflammation, narrowing of the airways and increased reactivity of the airways to inhaled agents. Asthma is frequently, although not exclusively associated with atopic or allergic symptoms.

The term "leukotriene inhibitor" includes any agent or compound that inhibits, restrains, retards or otherwise interacts with the action or activity of leukotrienes, such as, but not limited to, 5-lipoxygenase ("5-LO") inhibitors, 5-lipoxygenase activating protein ("FLAP") antagonists, and leukotriene D4 ("LTD4 ") antagonists.

"Immune-stimulation" or "immune potentiation" refers to activation of the immune system, including humoral or cellular activation, for example, activation of a cell, such as a killer (T or NK) or dendritic cell of the immune system, for example, causing the increase in cytokine production from a dendritic cell leading to an overall enhancement of host defense (immune response).

An "immunogenic composition" refers to a composition capable of modulating the production of cytokines in a subject thereby effecting immune potentiation in the subject.

An "immune-stimulatory effective amount" is an amount effective for activation of the immune system, for example, causing the increase in cytokine production from a dendritic cell leading to an overall enhancement of host defense (immune response).

"Enhancing the immune response to an antigen" by a compound refers to enhancement of the immune response in comparison to that in the absence of the compound. An enhanced immune-response eliciting composition is a composition generally comprising an antigen and a small molecule immune potentiator compound that elicits an immune response greater that a composition comprising an antigen and not containing one or more small molecule immune potentiator compounds. In this embodiment, the compound acts as an adjuvant, for example for use in vaccine compositions and methods.

The term "small molecule immunomodulator" or "small molecule immuno-modulatory composition" refers to small molecule compounds below about MW 800 g/mol, capable of stimulating or suppressing an immune response in a patient.

"Modulating" refers to inducing or suppressing.

"Immune suppression" or "immunosuppression" refers to deactivation of the immune system, for example, preventing or lessening cytokine production from a dendritic cell leading to an overall attenuation of host defense (immune response).

Reference to a "sub-optimal concentration," indicates a less than maximum effect of an agent on a system due to a decreased quantity of the agent in the system, whereby an increased amount of the agent or addition of another agent capable of stimulating a response would lead to further production of immunological markers, such as cytokines, chemokines, and/or growth factors.

Reference to a "sub-optimal concentration, such that said immunological markers are only partially stimulated" indicates a concentration of an agent that renders an immunological response less than the maximum, whereby an increased amount of the agent or addition of another agent capable of stimulating a response would lead to further production of immunological markers, such as cytokines, chemokines, and/or growth factors.

Reference to a "compound" is meant to indicate a small molecule, unless otherwise specified, wherein a small molecule has a molecular weight less than 800 g/mol and preferably less than 700 g/mol.

A "test compound" has the same meaning as compound, wherein the compound is being tested for activity, such as immunomodulation, immunosuppression, or immunopotentiation.

The term "plurality of compounds" refers to more than one compound. More preferably it refers to at least ten compounds, or even more preferably, 20-100 compounds.

A "high throughput assay" is meant to indicate an assay capable of identifying a certain attribute, specifically immunosuppression and/or immunopotentiation, in a number of compounds simultaneously.

A "capture antibody" is an antibody that is able to bind a particular immunological marker, such as a particular chemokine, cytokine, or growth factor. The capture antibodies are labeled, preferably with fluorescent dyes, such that they can be recognized and quantified. See Luminex Technologies, US 6,268,222 B1.

Reference to "wells" or "a well," as will be apparent to one skilled in the art, is meant to indicate a vessel for holding a reaction mixture for an assay, more specifically, a vessel for holding a test solution, control solution, stimulated solution, or unstimulated solution, as further described herein.

An "unstimulated solution" refers to a reaction mixture in which no agent capable of modulating an immune response was added.

A "stimulated solution" refers to a reaction mixture in which a known immune stimulant or immunopotentiating agent was added.

An "immunopotentiating agent" includes, among others, LPS, CpG, resiquimod (or R848), an adjuvant or SMIP disclosed in any reference cited herein, Poly I:C (dsRNA), Pam3-Cys, MPL, and stimulatory antibodies, specifically anti-CD3.

Reference to "immunological markers" indicates substances produced as a result of immunomodulation. If not otherwise specified, immunological markers could be present as a result of immunopotentiation or immunosuppression. Preferred immunological markers include cytokines, chemokines and growth factors.

Preferred "cytokines" include IL 1-30 (as described in Table 6) as well as TNF-alpha, TNF-beta, IFN-alpha (family), IFN-beta. and IFN-gamma.

Reference to "IL 1-30" indicates interleukin cytokines selected from the group consisting of ILIA, IL1B, IL1F5, IL1F6, IL1 F7, IL1F8, IL1F9, IL1F10, IL1R1, IL1R2, IL1RAP, IL1RAPL1, IL1RAPL2, IL1RL1, IL1RL2, IL1RN, IL2, IL2RA, IL2RB, IL2RG, IL3, IL3RA, IL4, IL4R, IL5, IL5RA, IL6, IL6R, IL6RL1, IL6ST, IL6ST2, IL6STP, IL7, IL7R, IL8, IL8RA, IL8RB, IL8RBP, IL9, IL9R, IL9RP1, IL9RP2, IL9RP3, IL9RP4, IL10, IL10RA, IL10RB, IL11, IL11RA, IL11RB, IL12A, IL12B, IL12RB1, IL12RB2, IL13, IL13RA1, DL13RA2, IL14, IL15, IL15RA, IL15RB, IL16, IL17, IL17B, IL17C, IL17D, IL17E, IL 17F, IL17R, IL17RB, IL17RC, IL17RD, IL17RE, IL18, IL18BP, IL18R1, IL18RAP, IL19, IL20, IL20RA, IL20RB, IL21, IL21R, IL22, IL22RA1, IL22RA2, IL23A, IL24, IL26, IL28A, IL28B, IL28RA, IL29, and IL30. More preferred interleukins include IL-1b, IL-2, IL-4, IL-5, IL-6, IL-10, IL-12, and IL-13. The accession ID numbers for each of IL 1-30 are listed in Table 6.

Reference to "chemokines" indicates: CXC chemokines including CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, and CXCL16; C chemokines including XCL1, and XCL2; CX₃C chemokines including CX₃CL1; and CC chemokines including CCL1, CCL2, CCL3, CCL3L1, CCL4, CCL5, CCL6, CCL7, CCL8, CCL9/CCL10, CCL11, CCL12, CCL13, CCL14, CCL15, CCL16, CCL17, CCL18, CCL19, CCL20, CCL21, CCL22, CC123, CCL24, CCL25, CCL26, CCL27, and CCL28 for which greater description is given in Table 5.

"Growth factors" that may serve as immunological markers include, among others, GM-CSF, G-CSF, M-CSF, VEGF, EGF, HGF, and FGF.

The term "providing" as it pertains to compounds bound to support resins, encompasses synthesizing and/or purchasing.

The terms "solid phase," "resin support bead," and "bead," intend any solid support or substrate on which screening and/or the reaction steps of chemical syntheses involving a sequence of reaction steps can be carried out. Thus, the term includes particulate substrates such as polystyrene resins which have traditionally been employed in standard Fmoc chemical syntheses.

The term "library" or "combinatorial library" includes, *inter alia,* a collection of sublibraries each containing 2-500 components or compounds, and more preferably about 10-100 components or compounds. The components or compounds of such sublibraries are diverse synthesized molecules which have been prepared using standard combinatorial chemistries (see, e.g., Furka et al., In t.J. Peptide Protein Res. 37:487-493 (1991); and Lam et al., Nature 354:82-84 (1991)).

The term "effective amount" is an amount necessary or sufficient to realize a desired biological effect. For example, an effective amount of a compound to treat an infectious disorder may be an amount necessary to cause an antigen specific immune response upon exposure to an infectious agent. The effective amount may vary, depending, for example, upon the condition treated, weight of the subject and severity of the disease. One of skill in the art can readily determine the effective amount empirically without undue experimentation.

As used herein "an effective amount for treatment" refers to an amount sufficient to palliate, ameliorate, stabilize, reverse, slow or delay progression of a condition such as a disease state.

A "subject" or "patient" is meant to describe a human or vertebrate animal including a dog, cat, pocket pet, marmoset, horse, cow, pig, sheep, goat, elephant, giraffe, chicken, lion, monkey, owl, rat, squirrel, slender loris, and mouse.

A "pocket pet" refers to a group of vertebrate animals capable of fitting into a commodious coat pocket such as, for example, hamsters, chinchillas, ferrets, rats, guinea pigs, gerbils, rabbits and sugar gliders.

As used herein, the term "pharmaceutically acceptable ester" refers to esters, which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Representative examples of particular esters include, but are not limited to, formates, acetates, propionates, butyrates, acrylates and ethylsuccinates.

The compounds of the present invention can be used in the form of salts as in "pharmaceutically acceptable salts" derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-napthalenesulfonate, oxalate, pamoate, pectinate, sulfate, 3-phenylpropionate, picrate, pivalate, propionate, succinate, tartrate, thiocyanate, p-toluenesulfonate and undecanoate. Also, the basic nitrogen-containing groups can be quaternized with such agents as lower alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

The term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transfonned *in vivo* to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference. Prodrugs as described in U.S. Patent No. 6,284,772 for example may be used.

The term "incubating" refers to maintaining a system under specific conditions in order to promote a particular reaction.

The term "preparing" in reference to a solution or combination of elements includes mixing, adding, or combining the elements in any order as well as procuring the solution or combination of elements in its final form.

The term "SMIP" refers to small molecule immuno-potentiating compounds, including small molecule compounds below about MW 800 g/mol, capable of stimulating or modulating a pro-inflammatory response in a patient. In an embodiment, the SMIP compounds are able to stimulate human peripheral blood mononuclear cells to produce cytokines, chemokines, and/or growth factors.

The term "SMIS" refers to small molecule immunosuppressant compounds, including small molecule compounds below about MW 800 g/mol, capable of suppressing or modulating an immune response in a patient. In an embodiment, the SMIS compounds are able to inhibit human peripheral blood mononuclear cell's ability to produce cytokines, chemokines, and/or growth factors. In another embodiment, the SMIS compounds are able to stimulate TGF-beta, thereby suppressing an immune response. In some embodiments, compounds of interest in the instant invention are analogs thereof, or "SMIS analogs," which are meant to describe a derivative of a compound known generally in the art to suppress the immune system. Preferred examples include compounds and analogs thereof described in the following US patents, US patent application publications, and PCT publications, which are hereby incorporated by reference as if set forth fully herein: US 4810692, US 4617315, US 4988680, US 5087619, US 5001124, US 4987139, US 5023264, US 5023263, US 4975372, US 4981792, US 5064835, US 5120842, US 5120727, US 5120725, US 5118678, US 5118677, US 5130307, US 5100883, US 5102876, US 5093338, US 5143918, US 5091389, US 5120726, US 5233036, US 5227467, US 5221740, US 5247076, US 5208241, US 5262423, US 5260300, US 5258389, US 5247119, US 5376663, US 5373014, US 5371225, US 5362735, US 5360794, US 5352783, US 5310903, US 5302584, US 5444072, US 5387680, US 5389639, US 6121257, US 6071947, US 6150373, US 6051590, US 6015809, US 5252732, US 5874411, US 5877184, US 5252732, US 5247076, US 5344925, US 5777105, US 5739169, US 5696156, US 5679705, US 5631282, US 5561228, US 5563145, US 5525610, US 5523408, US 5512687, US 5463048, US 5451604, US 5461054, US 5302584, US 5162334, US 5284877, US 5284840, US 5256790, US 5252579, US 5262533, US 2002016460, US 2002061905, US 6440991, US 6399626, US 6399773, US 6331547, US 6110922, US 5759550, US 5192773, US 5194447, US 5189042, US 5162333, US 5162334, US 5149701, US 5147877, US 4885276, US 4314061, US 4895872, US 4847299, US 4525299, US 5679640, US 5385910, US 5385908, US 5385909, US 5378836, US 5362718, US 5470878, US 5346912, US 5359073, US 6100259, US 5550214, US 5455230, US 6136817, US 5385910, US 5730979, US 5817311, US 5189039, US 5171864, US 5169963, US 5183906, US 5006520, US 5091381, US 5905090, US 5665772, US 5260323, US 5091381, US 4675382, US 5321009, US 5776943, US 5286730, US 5387589, US 5496832, US 4829061, US 4963557, US 4648996, US 4351841, US 4284786, US 4965276, US 5268382, US 5459163, US 5504084, US 5494911, US 5532259, US 5700823, US 5700822, US 5610173, US 4968702, US 5639455, US 5466697, US 5366986, US 5173499, US 5391730, US 4968702, US 4910211, US 4968702, US 4603137, US 4347315, US 5051441, US 4894374, US 5470831, US 6034096, US 5356897, US 5478827, US 5624931, US 5219864, US 4894374, US 5530101, US 4753935, US 4786637, US 4894366, US 4929611, US 4956352, US 5110811, US 5254562, US 5266692, US 4916138, US 4940797, US 5087703, US 5011943, US 5194378, US 5196437, WO 8707276, WO 8805784, WO 8809183, WO 8908658, WO 9110650, WO 9102736, WO 9141982, WO 9200314, WO 9014359, WO 9200313, WO 9113899, WO 9113889, WO 9211275, WO 9205189, WO 9205179, WO 9118901, WO 9203441, WO 9200980, WO 9200314, WO 9316083, WO 9311130, WO 9312125, WO 9310122, WO 9310796, WO 9305059, WO 9305058, WO 9318050, WO 9318049, WO 9318048, WO 9318042, WO 9501355, WO 9429295, WO 9322286, WO 9425468, WO 9424095, WO 9420488, WO 9418208, WO 9418206, WO 9409010, WO 9522537, WO 9522536, WO 9408943, WO 9516691, WO 9515328, WO 9517381, WO 9514023, WO 9408943, WO 9506045, WO 9509857, WO 9504060, WO 9504738, WO 0058318, WO 0053190, WO 0058314, WO 0039129, WO 0034228, WO 0034248, WO 0032604, WO 9840380, WO 0024744, WO 0012514, WO 0009510, WO 0002879, WO 9955689, WO 9965909, WO 9965908, WO 9965450, WO 9947707, WO 9938829, WO 9940069, WO 9941239, WO 9509857, WO 9305058, WO 9931087, WO 9931060, WO 9929695, WO 9931066, WO 9931063, WO 9931064, WO 9925703, WO 9912890, WO 9920267, WO 9920274, WO 9915530, WO 9305058, WO 9305059, WO 9708182, WO 9828301, WO 9818780, WO 9816532, WO 9816531, WO 9816518, WO 9806719, WO 9809972, WO 9739999, WO 9748696, WO 9748695, WO 9744351, WO 9744052, WO 9317699, WO 9740028, WO 9606068, WO 9720814, WO 9716438, WO 9716437, WO 9716182, WO 9716068, WO 9714410, WO 9712888, WO 9712887, WO 9711080, WO 9711092, WO 9709298, WO 9640688, WO 9641807, WO 9631529, WO 9304679, WO 9630381, WO 9625936, WO 9624579, WO 9617845, WO 9615131, WO 9611200, WO 9613510, WO 9535299, WO 9535120, WO 9534568, WO 9534565, WO 9522538, WO 9515947, WO 9522534, WO 9522537, WO 9220688, WO 9402485, WO 9402137, WO 9402136, WO 9404540, WO 0270472, WO 0253543, WO 0257216, WO 0257237, WO 0251397, WO 0125238, WO 0119829, WO 0230938, WO 0228867, WO 0228866, WO 0218395, WO 0206268, WO 0025780, WO 0200661, WO 0190110, WO 0034296, WO 0033887, WO 0059880, WO 0039081, WO 0107401, WO 0105768, WO 0105750, WO 0076953, WO 9426265, WO 9305058, WO 9222294, WO 9307871, WO 9221313, WO 9305046, WO 9220688, WO 9218496, WO 9010631, WO 9007523, WO 9007520, WO 9002727, WO 9824762, WO 9809946, WO 9743434, WO 9809970, WO 9804521, WO 9732847, WO 9741148, WO 9739018, WO 9723453, WO 9709325, WO 9702285, WO 9702820, WO 9709315, WO 9428910, WO 9422863, WO 9412500, WO 0062778, WO 0056331, WO 9831227, WO 0040562, WO 0025786, WO 9615105, WO 9617874, WO 9616967, WO 9611199, WO 9611198, WO 9603419, WO 9531463, WO 9531463, WO 9528394, WO 9515309, WO 9422872, WO 0285928, WO 0272562 WO 0248122, WO 0250071, WO 0269904, WO 0228862, WO 0047595, WO 0125238, WO 0102359, WO 0253150, WO 0062778, WO 9202229, WO 9303036, WO 9219610, WO 9219609, WO 8903385, WO 9205180, WO 0015645, WO 0002839, WO 9942105, WO 9915501, WO 9856785, WO 9852933, WO 9852951, WO 9845249, WO 9832750, WO 9735575, WO 9409010, WO 9424304, WO 9507468, WO 9804279, WO 8908113, WO 9405685, WO 9102000, WO 9214476, WO 9214477, WO 9319763, WO 9747317, WO 9818468, WO 8907613, WO 8902889, WO 8805783, WO 9117748, WO 9519169, WO 0063210, WO 0048989, WO 9804521, WO 9746522, WO 9741148, WO 9739018, WO 9720811, WO 9705140, WO 9606855, WO 9602541, WO 9210295, WO 9509153, WO 0255541, WO 0202539, WO 0063210, WO 9509153, WO 9513277 WO 9508535, WO 9501175, WO 9424133, WO 9408589, WO 9415959, WO 8906968, WO 0015604, WO 9932138, WO 9900143, WO 9852606, WO 9117748, WO 9116339, WO 9520589, WO 9211021, WO 9213874, WO 9500543, WO 9531206, WO 9520589, WO 9715561, WO 9743251, WO 9741104, WO 9743251, WO 9723453, WO 9715561, WO 9705141, WO 9640143, WO 9749399, WO 9412184, WO 9426266, WO 9507902, WO 9509626, WO 9014826, WO 9117754, WO 9119495, WO 9308802 and WO 9314771. Additionally SMIS analogs include small molecules selected from the group consisting of cortisol, cyclophosphamide, 6-mercaptopurine, methotrexate and its polyglutamate derivatives, azathioprine metabolites, mizoribine, cyclosporin A, FK-506 (tacrolimus), rapamycin, leflunomide and its metabolites, brequinar, tacrolimus, mycophenolate mofetil, and mycophenolic acid. Further, preferred "SMIS" compound for which analogs with immunopotentiation capabilities exist include the Examples listed in Table 4.

Analogs of SMIS compounds are restricted to encompass substituent alterations and additions to the core scaffold. Core scaffolds of SMIS can be identified functionally by one skilled in the art as the minimal unit responsible for activity. Structurally, the core scaffold of a SMIS can be identified as the conserved region amongst several similar compounds (from the same source), typically listed adjacently. For example, the core scaffold for Examples 86-91 (Source: Aventis) is apparent by one skilled in the art as a substituted benzamide. Similarly, the core scaffold of Examples 111-15 is identified by the macrocyclic cyclosporine-like structural motif. Where a single species exists without multiple compounds to guide elucidation, e.g. Example 167, the core scaffold is confined to the central region consisting of a continuous aromatic or non-aromatic heterocyclic, polycyclic, or heteroalkyl group of at least 5 atoms (e.g. isobenzofuran-1 (3H)-one for Example 167) or an ion complex, such as ruthenium. Preferred scaffolds include steroids, terpenes, macrocycles, ruthenium complexes, cannabinoids, aminoazavinyl compounds, benzazole compounds, acylpiperazine compounds, indoledione compounds, tetrahydroisoquinoline (THIQ) compounds, anthraquinone compounds, indanedione compounds, pthalimide compounds, benzocyclodione compounds, aminobenzimidazole quinolinone (ABIQ) compounds, hydraphthalimide compounds, pyrazolopyrimidine compounds, quinazilinone compounds, quinoxaline compounds, triazine compounds, tetrahydropyrrolidinoquinoxaline compounds, pyrrole compounds, benzophenone compounds, sterol compound, and isoxazole compounds. Substituents suitable for alteration and/or addition to the core scaffold include, for example, substituted or unsubstituted alkyl, substituted or unsubstituted alkoxy, substituted or unsubstituted alkenyl, substituted or unsubstituted alkynyl, substituted or unsubstituted amino, substituted or unsubstituted alkylamino, substituted or unsubstituted dialkylamino, substituted or unsubstituted aryl, substituted or unsubstituted aralkyl, substituted or unsubstituted heteroaryl, substituted or unsubstituted heteroaralkyl, substituted or unsubstituted heterocyclyl, substituted or unsubstituted heterocyclylalkyl, substituted or unsubstituted carbonyl, substituted or unsubstituted carbonyloxy, substituted or unsubstituted sulfonyl, substituted or unsubstituted carbonylamino, substituted or unsubstituted aminocarbonyl, substituted or unsubstituted guanidinyl, or substituted or unsubstituted alkoxycarbonyl groups.

The term "steroid SMIP" refers to a synthetic fat-soluble organic SMIP having as a basis 17 carbon atoms arranged in four rings and including the sterols and bile acids, adrenal and sex hormones, certain natural drugs such as digitalis compounds, and the precursors of certain vitamins and the derivatives thereof. Preferred steroid scaffolds for derivitization include, among others, pregnane, estrane, cholestane, gonane, and androstane.

The term "macrocycle SMIP" refers to any non-aromatic or only partially aromatic SMIP having at least 12 contiguous atoms selected from, carbon, N, O, or S bound together to in a cyclic moiety. Preferred macrocycles include cyclosporine, sirolimus, tacrolimus and their derivatives.

The term "terpene SMIP" refers to a SMIP containing at least one terpene moiety.

The term "ruthenium complex SMIP" refers to a SMIP with multiple organic compounds ionically complexed around a central ruthenium atom. Preferred organic compounds to be complexed to the ruthenium atom are nitrogen containing heteroaryl (N_{Haryl}). Preferred N_{Haryl} compounds include, for example, acridine, carbazole, β-carboline, cinnoline, imidazole, indazole, indole, indolizine, isoindole, isoquinoline, isothiazole, oxazole, isoxazole, naphthyridine, perimidine, phenanthridine, phenanthroline, phenazine, phthalazine, pteridine, purine, pyrazine, pyrazole, pyridazine, pyridine, pyrimidine, pyrrole, pyrrolizine, quinazoline, quinoline, quinolizine, quinoxaline, and thiazole.

Reference to an "expoxide" is meant to indicate a ring-shaped organic compound consisting of an oxygen atom bonded to two other atoms, preferably of carbon, that are bonded to each other.

Usage of a 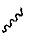 symbol that crosses a covalent bond is meant to indicate the point of attachment of a substituent, as in 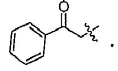 Similarly, usage of an asterisk also indicates the point of attachment of a substituent, as in, *-alkenyl-COOH.

Where multiple groups are combined to form a substituent, for example dialkylaminocarbonyl, the point of attachment is the last group captioned in the phrase, such as carbonyl in the aforementioned example. Although not all substituents with multiple combinations of groups described in the embodiments are explicitly defined below, for example arylsulfonylalkyloxycarbonylamino, their individual components are defined and their scope will be apparent to one skilled in the art as a summation of their individual components, such as *-NH-C(O)-O-alkyl-SO₂-aryl, in the aforementioned example.

The phrase "alkyl" refers to alkyl groups that do not contain heteroatoms. Thus the phrase includes straight chain alkyl groups such as methyl, ethyl, propyl, butyl, pentyl, hexyl, heptyl, octyl, nonyl, decyl, undecyl, dodecyl and the like. The phrase also includes branched chain isomers of straight chain alkyl groups, including but not limited to, the following which are provided by way of example: -CH(CH₃)₂, - CH(CH₃)(CH₂CH₃), -CH(CH₂CH₃)₂, -C(CH₃)₃, -C(CH₂CH₃)₃, -CH₂CH(CH₃)₂, - CH₂CH(CH₃)(CH₂CH₃), -CH₂CH(CH₂CH₃)₂₋, -CH₂C(CH₃)₃, -CH₂C(CH₂CH₃)₃, - CH(CH₃)CH(CH₃)(CH₂CH₃), -CH₂CH₂CH(CH₃)₂, -CH₂CH₂CH(CH₃)(CH₂CH₃), - CH₂CH₂CH(CH₂CH₃)₂, -CH₂CH₂C(CH₃)₃, -CH₂CH₂C(CH₂CH₃)₃, - CH(CH₃)CH₂CH(CH₃)₂, -CH(CH₃)CH(CH₃)CH(CH₃)₂, - CH(CH₂CH₃)CH(CH₃)CH(CH₃)(CH₂CH₃), and others. Thus, the phrase unsubstituted alkyl groups includes primary alkyl groups, secondary alkyl groups, and tertiary alkyl groups. Unsubstituted alkyl groups may be bonded to one or more carbon atom(s), oxygen atom(s), nitrogen atom(s), and/or sulfur atom(s) in the parent compound. Preferred unsubstituted alkyl groups include straight and branched chain alkyl groups and cyclic alkyl groups having 1 to 20 carbon atoms. More preferred such unsubstituted alkyl groups have from 1 to 10 carbon atoms while even more preferred such groups have from 1 to 5 carbon atoms. Most preferred unsubstituted alkyl groups include straight and branched chain alkyl groups having from 1 to 3 carbon atoms and include methyl, ethyl, propyl, and -CH(CH₃)₂.

The phrase "substituted alkyl" refers to an unsubstituted alkyl group as defined above in which one or more bonds to a carbon(s) or hydrogen(s) are replaced by a bond to non-hydrogen and non-carbon atoms such as, but not limited to, a halogen atom in halides such as F, Cl, Br, and I; a phosphorus atom in groups such as phosphate and dialkyl alkylphosphonate; oxygen atom in groups such as hydroxyl groups, alkoxy groups, aryloxy groups, and ester groups; a sulfur atom in groups such as thiol groups, alkyl and aryl sulfide groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as in trialkylsilyl groups, dialkylarylsilyl groups, alkyldiarylsilyl groups, and triarylsilyl groups; and other heteroatoms in various other groups. Substituted alkyl groups also include groups in which one or more bonds to a carbon(s) or hydrogen(s) atom is replaced by a bond to a heteroatom such as oxygen in carbonyl, carboxyl, and ester groups; nitrogen in groups such as imines, oximes, hydrazones, and nitriles. Preferred substituted alkyl groups include, among others, alkyl groups in which one or more bonds to a carbon or hydrogen atom is/are replaced by one or more bonds to fluorine atoms. One example of a substituted alkyl group is the trifluoromethyl group and other alkyl groups that contain the trifluoromethyl group. Another example is a carbonylalkyl or -alkyl-COOH group. Other alkyl groups include those in which one or more bonds to a carbon or hydrogen atom is replaced by a bond to an oxygen atom such that the substituted alkyl group contains a hydroxyl, alkoxy, aryloxy group, or heterocyclyloxy group. Still other alkyl groups include alkyl groups that have an amine, alkylamine, dialkylamine, arylamine, (alkyl)(aryl)amine,diarylamine, heterocyclylamine, (alkyl)(heterocyclyl)amine, (aryl)(heterocyclyl)amine, or diheterocyclylamine group.

The term "halogen" refers to iodine, bromine, chlorine or fluorine; "halo" as used herein refers to iodo, bromo, chloro or fluoro.

The term "haloalkyl" as used herein refers to a alkyl radical, as defined above, bearing at least one halogen substituent, for example, chloromethyl, fluoroethyl or trifluoromethyl and the like.

The term "amino" refers to -NH₂, when at the terminal position, or -NH-when conjoining 2 groups.

The term "nitro" refers to an -NO₂ substituent.

The term "cyano" refers to -CN.

The term "carboxylic acid" refers to -COOH.

The term "alkoxy" as used herein refers to RO- wherein R, for example, is alkyl such as defined above. Representative examples of alkoxy groups include methoxy, ethoxy, t-butoxy and the like.

The term "substituted alkoxy" as used herein refers to RO-, where R is, for example, an alkyl substituted, for example, with a halogen. RO is for example OCF₃, or further substituted with an aryl such as (2-chlorophenyl)methoxy.

The term "alkoxyalkyl" as used herein refers to an (alkyl)-O-(alkyl), wherein each alkyl group may be further substituted as defined below for optional substitution groups.

Reference to "malonate" is meant to indicate substituted alkoxy as shown:

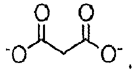

The term "alkenyl" as used herein refers to a branched or straight chain groups comprising two to twenty carbon atoms which also comprises one or more carbon-carbon double bonds. Representative alkenyl groups include prenyl, 2-propenyl (i.e., allyl), 3-methyl-2-butenyl, 3,7-dimethyl-2,6-octadienyl, 4,8-dimethyl-3,7-nonadienyl, 3,7,11-trimethyl-2,6,10-dodecatrienyl and the like.

The term "substituted alkenyl" as used herein refers to alkenyl groups that are substituted, for example, *-alkenyl-carbonyl-oxy- (*-alkenyl-C(O)-O-), diethyl hex-5-enylphosponate, and others with an alkyl or substituted alklyl group such as dialkyl phosphate or an ester such as an acetate ester.

The term "alkynyl" as used herein refers to a branched or straight chain groups comprising two to twenty carbon atoms which also comprises one or more carbon-carbon triple bonds. Representative alkynyl groups include propargyl and butynyl.

The term "substituted alkynyl" as used herein refers to alkynyl groups that are substituted, for example, propargylamine, and others with an alkyl or substituted alkyl group such as dialkyl phosphate or an ester such as an acetate ester

The term "alkylamino" as used herein refers to an amino group substituted with one alkyl groups such as C1-20 alkyl groups.

The term "substituted alkylamino" as used herein refers to an alkylamino substituted, for example, with a carboxylic acid, ester, hydroxy or alkoxy.

The term "dialkylamino" as used herein refers to an amino group substituted with two alkyl groups such as C1-20 alkyl groups.

The term "substituted dialkylamino" as used herein refers to a dialkylamino substituted, for example, with a carboxylic acid, ester, hydroxy or alkoxy.

The term "aminoalkyl" refers to an alkyl group substituted with an amino group.

The term "alkylaminoalkyl" refers to an alkyl group substituted with an aminoalkyl group, such as *-CH₂NHCH(CH₃)₂

The term "hydroxyalkylthio" as used herein refers to a thio radical to which is appended a hydroxyalkyl group, where the alkyl is for example ethyl. An example is hydroxyethylthio, -SCH₂CH₂OH.

The term "N-alkylsulfonamide" as used herein refers to the group - SO₂NHalkyl, where alkyl is, for example, octyl.

"Aminosulfonyl," refers herein to the group -S(O)₂-NH₂. "Substituted aminosulfonyl" refers herein to the group -S(O)₂-NRR' where R is alkyl and R' is hydrogen or an alkyl. The term "alkylaminosulfonyl" refers herein to the group -S(O)₂-NH-alkyl The term "aralkylaminosulfonlyaryl" refers herein to the group - aryl-S(O)₂-NH-aralkyl.

"Carbonyl" refers to the divalent group -C(O)-.

"Carbonyloxy" refers generally to the group -C(O)-O-, Such groups include esters, -C(O)-O-R, where R is alkyl, cycloalkyl, aryl, or aralkyl. The term "carbonyloxycycloalkyl" refers generally herein to both an "carbonyloxycarbocycloalkyl" and an "carbonyloxyheterocycloalkyl", i.e., where R is a carbocycloalkyl or heterocycloalkyl, respectively. The term "arylcarbonyloxy" refers herein to the group -C(O)-O-aryl, where aryl is a mono- or polycyclic, carbocycloaryl or heterocycloaryl. The term "aralkylcarbonyloxy" refers herein to the group -C(O)-O-aralkyl, where the aralkyl is aralkyl.

The term "sulfonyl" refers herein to the group -SO₂-, wherein an unsubstituted sulfonyl group is -SO₂H. "Alkylsulfonyl" refers to a substituted sulfonyl of the structure *-SO₂R - in which R is alkyl. Alkylsulfonyl groups employed in compounds of the present invention are typically alkylsulfonyl groups having from 1 to 6 carbon atoms in its backbone structure. Thus, typical alkylsulfonyl groups employed in compounds of the present invention include, for example, methylsulfonyl (i.e., where R is methyl), ethylsulfonyl (i.e., where R is ethyl), propylsulfonyl (i.e., where R is propyl), and the like. The term "arylsulfonyl" refers herein to the group -SO₂-aryl, or "heteroarylsulfonyl" refers herein to the group -SO₂-heteroaryl. The term "aralkylsulfonyl" refers herein to the group -SO₂-aralkyl or layed out, *-SO₂-alkyl-aryl-. "Heteroaralkylsulfonyl" refers to the same as aralkylsulfonyl, except with a heteroaryl substituent in place of an aryl.

As used herein, the term "carbonylamino" refers to the divalent group -NH-C(O)- in which the hydrogen atom of the amide nitrogen of the carbonylamino group can be replaced an alkyl, aryl, or aralkyl group. Such groups include moieties such as carbamate esters (*-NH-C(O)-O-R) and amides *-NH-C(O)-O-R, where R is a straight or branched chain alkyl, cycloalkyl, or aryl or aralkyl. The term "alkylcarbonylamino" refers to alkylcarbonylamino where R is an alkyl having from 1 to about 6 carbon atoms in its backbone structure. The term "arylcarbonylamino" refers to group -NH-C(O)-R where R is an aryl. Similarly, the term "aralkylcarbonylamino" refers to carbonylamino where R is a aralkyl.

As used herein, the term "guanidino" or "guanidyl" refers to moieties derived from guanidine, H₂N-C(=NH)-NH₂. Such moieties include those bonded at the nitrogen atom carrying the formal double bond (the "2"-position of the guanidine, e.g., diaminomethyleneamino, (H₂N)₂C=NH-*) and those bonded at either of the nitrogen atoms carrying a formal single bond (the "1-" and/or "3"-positions of the guandine, *e.g*., H₂N-C(=NH)-NH-). The hydrogen atoms at any of the nitrogens can be replaced with a suitable substituent, such as alkyl, aryl, or aralkyl.

The term "arylthiourea" refers to aryl-NC(S)N-* groups, wherein the nitrogen is the point of attachment. A preferred example of a substituted aryl-NC(S)N-alkylamino-* group is:

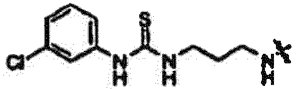

Representative substituted alkylcarbonylamino, alkyloxycarbanylamino, aminoalkyloxycarbonylamino, and arylcarbonylamino groups include, for example, those shown below. These groups can be further substituted as will be apparent to those having skill in the organic and medicinal chemistry arts in conjunction with the disclosure herein.

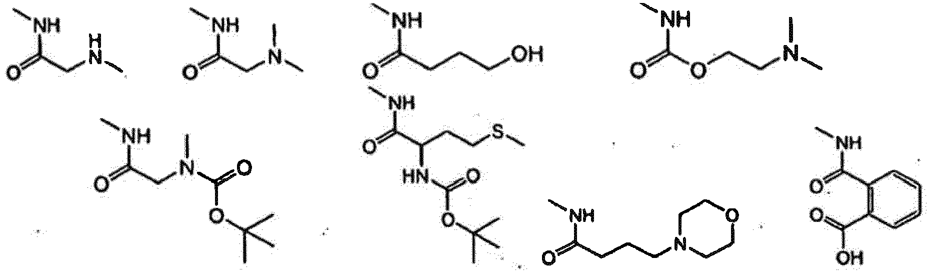

Representative substituted aminocarbonyl groups include, for example, those shown below. These can heterocyclo groups be further substituted as will be apparent to those having skill in the organic and medicinal chemistry arts in conjunction with the disclosure herein.

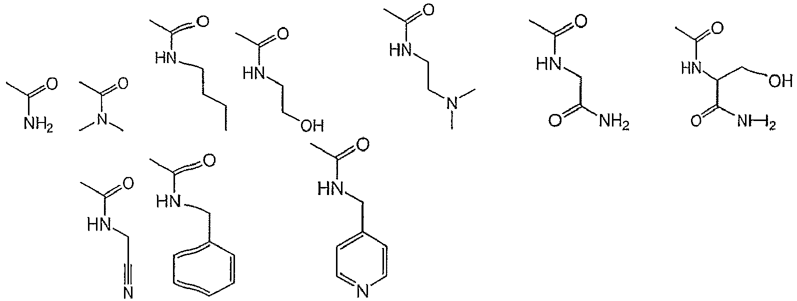

A preferred substituted aminocarbonylamino group (or urea) includes: 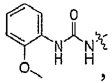 which is substituted with an anisole group.

Representative substituted alkoxycarbonyl groups include, for example, those shown below. These alkoxycarbonyl groups can be further substituted as will be apparent to those having skill in the organic and medicinal chemistry arts in conjunction with the disclosure herein.

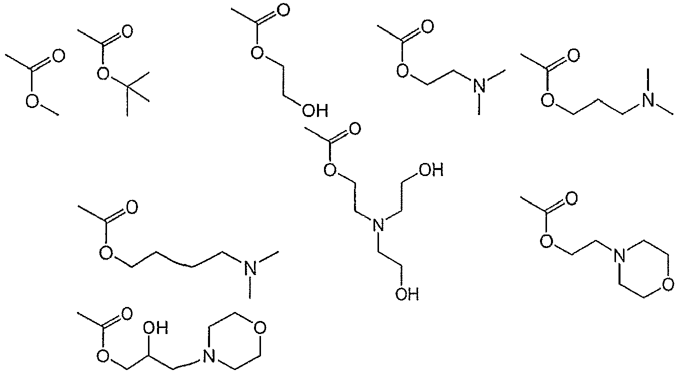

The phrase "carbocyclyl" includes cyclic alkyl groups such as cyclopropyl, cyclobutyl, cyclopentyl, cyclohexyl, cycloheptyl, and cyclooctyl and such rings substituted with straight and branched chain alkyl groups as defined above. The phrase also includes polycyclic alkyl groups such as, but not limited to, adarnantyl, norbornyl, and bicyclo[2.2.2]octyl and such rings optionally substituted as defined herein.

The phrase "aryl" refers to aryl groups that do not contain heteroatoms. Thus the phrase includes, but is not limited to, groups such as phenyl, biphenyl, anthracenyl, naphthenyl by way of example. Although the phrase "unsubstituted aryl" includes groups containing condensed rings such as naphthalene, it does not include aryl groups that have other groups such as alkyl or halo groups bonded to one of the ring members, as axyl groups such as tolyl are considered herein to be substituted aryl groups as described below. A preferred unsubstituted aryl group is phenyl. Unsubstituted aryl groups may be bonded to one or more carbon atom(s), oxygen atom(s), nitrogen atom(s), and/or sulfur atom(s) in the parent compound, however.

The phrase "substituted aryl group" has the same meaning with re spect to aryl groups that substituted alkyl groups had with respect to alkyl groups. However, a substituted aryl group also includes aryl groups in which one of the aromatic carbons is bonded to one of the non-carbon or non-hydrogen atoms described above and also includes aryl groups in which one or more aromatic carbons of the aryl group is bonded to a substituted and/or unsubstituted alkyl, alkenyl, or alkynyl group as defined herein. This includes bonding arrangements in which two carbon atoms of an aryl group are bonded to two atoms of an alkyl, alkenyl, or alkynyl group to define a fused ring system (e.g. dihydronaphthyl or tetrahydronaphthyl). Thus, the phrase "substituted aryl" includes, but is not limited to tolyl, and hydroxyphenyl among others.

Included within the definition of aryl groups are fused and unfused arylaryl groups. The term "unfused arylaryl" refers to a group or substituent to which two aryl groups, which are not condensed to each other, are bound. Exemplary unfused arylaryl compounds include, for example, phenylbenzene, diphenyldiazene, 4-methylthio-1-phenylbenzene, phenoxybenzene, (2-phenylethynyl)benzene, diphenyl ketone, (4-phenylbuta-1,3-diynyl)benzene, phenylbenzylamine, (phenylmethoxy)benzene and the like. Preferred substituted unfused arylaryl groups include: 2-(phenylamino)-N-[4-(2-phenylethynyl)phenyl]acetamide, 1,4-diphenylbenzene, N-[4-(2-phenylethynyl)phenyl]-2-[benzylamino]acetamide- 2-amino-N-[4-(2-phenylethynyl)phenyl]propanamide, 2-amino-N-[4-(2-phenylethynyl)phenyl]acetamide, 2-(cyclopropylamino)-N-[4-(2-phenylethynyl)phenyl]acetamide, 2-(ethylamino)-N-[4-(2-phenylethynyl)phenyl]acetamide, 2-[(2-methylpropyl)amino]-N-[4-(2-phenylethynyl)phenyl]acetamide, 5-phenyl-2H-benzo[d] 1,3-dioxolene, 2-chloro-1-methoxy-4-phenylbenzene, 2-[(imidazolylmethyl)amino]-N-[4-(2-phenylethynyl)phenyl]acetamide, 4-phenyl-1-phenoxybenzene, N-(2-aminoethyl)[4-(2-phenylethynyl)phenyl]carboxamide, 2-{[(4-fluorophenyl)methyl]amino}-N-[4-(2-phenylethynyl)phenyl]acetamide, 2-{[(4-methylphenyl)methyl]amino}-N-[4-(2-phenylethynyl)phenyl]acetamide, 4-phenyl-1-(trifluoromethyl)benzene, 1-butyl-4-phenylbenzene, 2-(cyclohexylamino)-N-[4-(2-phenylethynyl)phenyl]acetamide, 2-(ethylmethylamino)-N-[4-(2-phenylethynyl)phenyl]acetamide, 2-(butylamino)-N-[4-(2-phenylethynyl)phenyl]acetamide, N-[4-(2-phenylethynyl)phenyl]-2-(4-pyridylamino)acetamide, N-[4-(2-phenylethynyl)phenyl]-2-(quinuclidin-3-ylamino)acetamide, N-[4-(2-phenylethynyl)phenyl]pyrrolidin-2-ylcarboxamide, 2-amino-3-methyl-N-[4-(2-phenylethynyl)phenyl]butanamide, 4-(4-phenylbuta-1,3-diynyl)phenylamine, 2-(dimethylamino)-N-[4-(4-phenylbuta-1,3-diynyl)phenyl]acetamide, 2-(ethylamino)-N-[4-(4-phenylbuta-1,3-diynyl)phenyl]acetamide, 4-ethyl-1-phenylbenzene, 1-[4-(2-phenylethynyl)phenyl]ethan-1-one, N-(1-carbamoyl-2-hydroxypropyl)[4-(4-phenylbuta-1,3-diynyl)phenyl]carboxamide, N-[4-(2-phenylethynyl)phenyl]propanamide, 4-methoxyphenyl phenyl ketone, phenyl-N-benzamide, (tert-butoxy)-N-[(4-phenylphenyl)methyl]carboxamide, 2-(3-phenylphenoxy)ethanehydroxamic acid, 3-phenylphenyl propanoate, 1-(4-ethoxyphenyl)-4-methoxybenzene, and [4-(2-phenylethynyl)phenyl]pyrrole. The term "fused arylaryl" as used herein refers to an aryl group as previously defined which is condensed, and fully conjugated to an aryl group. Representative fused arylaryl groups include biphenyl, 4-(1-naphthyl)phenyl, 4-(2-naphthyl)phenyl and the like.

The term "aralkyl" as used herein refers to an alkyl radical to which is appended an aryl group. Representative aralkyl groups include benzyl, phenylethyl, hydroxybenzyl, fluorobenzyl, fluorophenylethyl and the like.

The term "heteroaralkyl" as used herein refers to an alkyl radical to which is appended a heteroaryl group. Representative heteroaralkyl groups include methylpyridine, 4-*sec*-butylpyrimidine and the like.

The term "heterocyclylalkyl" as used herein refers to an alkyl radical to which is appended a heterocyclyl group. Representative heterocyclylalkyl groups include methylpiperizine, epichlorohydrin, *sec*-butylpiperidine and the like.

The term "carbocyclylalkyl" as used herein refers to an alkyl radical to which is appended a carbocyclyl group. Representative carbocyclylalkyl groups include isopropylcyclohexane, 1 -ethyl-2-fluorocyclopentane and the like.

The term "arylalkenyl" as used herein refers to an alkenyl radical to which is appended an aryl group. Representative arylalkenyl groups include styrene, 1-((Z)-prop-1-enyl)benzene, and the like.

The term "heteroarylalkenyl" as used herein refers to a alkenyl radical to which is appended a heteroaryl group. Representative heteroarylalkenyl groups include 4-vinylpyrimidine, 4-((Z)-prop-1-enyl)pyridine and the like.

The term "heterocyclylalkenyl" as used herein refers to a alkenyl radical to which is appended a heterocyclyl group. Representative heterocyclylalkenyl groups include vinylpiperizine, 4-((E)-prop-1-enyl)piperidine and the like.

The term "carbocyclylalkenyl" as used herein refers to a alkenyl radical to which is appended a carbocyclyl group. Representative carbocyclylalkenyl groups include vinylcyclohexane, (prop-1-en-2-yl)cyclohexane and the like.

The term "aryloxy" as used herein refers to RO- wherein R is an aryl group. Representative aryloxy groups include benzyloxy, biphenyloxy and the like.

The term "heteroaryloxy" as used herein refers to RO- wherein R is a heteroaryl group.

The term "arylalkoxy" as used herein refers to an alkoxy radical to which is appended an aryl group. Representative arylalkoxy group include benzylmethoxy, phenylethoxy and the like.

As used herein, representative heterocyclyl or heterocyclo groups include, for example, those listed and shown below (where the point of attachment of the substituent group, and the other substituent groups shown below, is through the upper left-hand bond). These heterocyclyl groups can be further substituted and may be attached at various positions as will be apparent to those having skill in the organic and medicinal chemistry arts in conjunction with the disclosure herein. Those heterocyclyl groups include, for example, pyrrolidine, piperidine, methylpyrrolidine, pyrrolidine-3-ylamine, dimethylpyrrolidin-3-ylamine, 2-aminoquinuclidine, pyrrolidin-2-one, tetrahydrofuranyl, pyrrolidin-3-ol, 4-piperidylpiperidine, 1-benzyl-4-piperidylamine, homopiperidine, homopiperizine, homomorpholine, methylpyrrolidine,

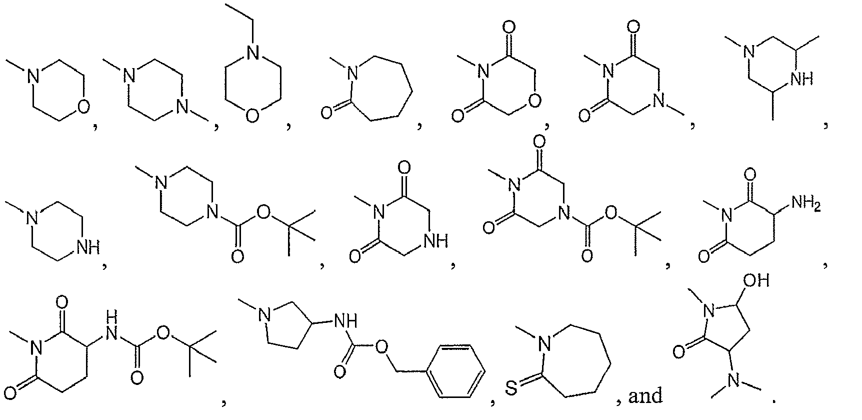

Representative heteroaryl groups include, for example, acridine, carbazole, β-carboline, cinnoline, furan, imidazole, indazole, indole, indolizine, isoindole, isoquinoline, isothiazole, oxazole, isoxazole, naphthyridine, perimidine, phenanthridine, phenanthroline, phenazine, phthalazine, pteridine, purine, pyrazine, pyrazole, pyridazine, pyridine, pyrimidine, pyrrole, pyrrolizine, quinazoline, quinoline, quinolizine, quinoxaline, thiazole, 4H-pyran-4-one as well as those shown below. These heteroaryl groups can be further substituted and may be attached at various positions as will be apparent to those having skill in the organic and medicinal chemistry arts in conjunction with the disclosure herein.

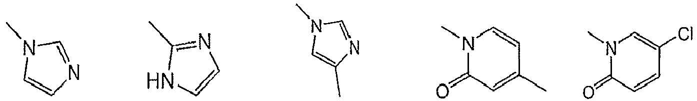

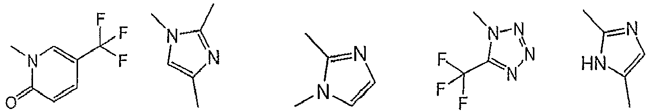

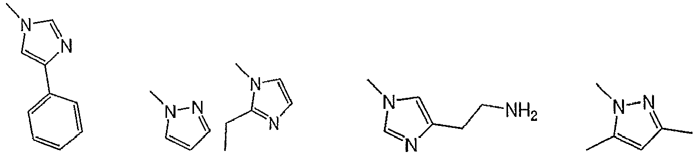

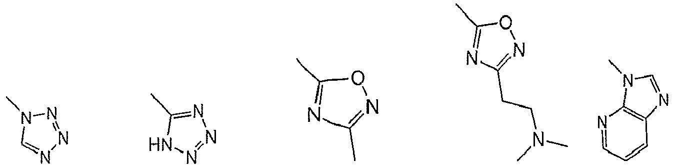

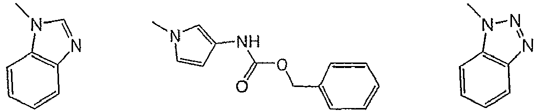

Included within the definition of heteroaryl groups are fused and unfused arylheteroaryl, heteroarylaryl, and heteroarylheteroaryl groups. The term "unfused heteroarylaryl" refers to a unfused arylaryl group where one of the aryl groups is a heteroaryl group. Exemplary heteroarylaryl groups include, for example, 2-phenylpyridine, phenylpyrrole, 3-(2-phenylethynyl)pyridine, phenylpyrazole, 5-(2-phenylethynyl)-1,3-dihydropyrimidine-2,4-dione, 4-phenyl-1,2,3-thiadiazole, 2-(2-phenylethynyl)pyrazine, 2-phenylthiophene, phenylimidazole, 3-(2-piperazinylphenyl)furan, 3-(2,4-dichlorophenyl)-4-methylpyrrole, and the like. Preferred substituted unfused heteroarylaryl groups include: 5-(2-phenylethynyl)pyrimidine-2-ylamine, 1-methoxy-4-(2-thienyl)benzene, 1-methoxy-3-(2-thienyl)benzene, 5-methyl-2-phenylpyridine, 5-methyl-3-phenylisoxazole, 2-[3-(trifluoromethyl)phenyl]furan, 3-fluoro-5-(2-furyl)-2-methoxy-1-prop-2-enylbenzene, (hydroxyimino)(5-phenyl(2-thienyl))methane, 5-[(4-methylpiperazinyl)methyl]-2-phenylthiophene, 2-(4-ethylphenyl)thiophene, 4-methylthio-l-(2-thienyl)benzene, 2-(3-nitrophenyl)thiophene, (tert-butoxy)-N-[(5-phenyl(3-pyridyl))methyl]carboxamide, hydroxy-N-[(5-phenyl(3-pyridyl))methyl] amide, 2-(phenylmethylthio)pyridine, and benzylimidazole.

The term "unfused heteroarylheteroaryl" refers to an unfused arylaryl group where both of the aryl groups is a heteroaryl group. Exemplary heteroarylheteroaryl groups include, for example, 3-pyridylimidazole, 2-imidazolylpyrazine, and the like. Preferred substituted unfused heteroarylheteroaryl groups include: 2-(4-piperazinyl-3-pyridyl)furan, diethyl(3-pyrazin-2-yl(4-pyridyl))amine, and dimethyl{2-[2-(5-methylpyrazin-2-yl)ethynyl](4-pyridyl)}amine.

The term "fused heteroarylaryl" as used herein refers to an aryl group as previously defined which is condensed, and fully conjugated with a heteroaryl group. Representative fused heteroarylaryl groups include quinoline, quinazoline and the like.

The term "fused heteroarylheteroaryl" as used herein refers to a heteroaryl group as previously defined which is condensed, and fully conjugated with another heteroaryl group. Representative fused heteroarylheteroaryl groups include pyrazalopyrimidine, imidazoquinoline and the like.

The term "imineheterocyclyl" refers to moieties having an imine substituent bound distally (w/ respect to the point attachment) to a heterocyclyl group. An example of an imineheterocyclyl is N-(piperazin-1-yl)ethanimine.

The term "fused multicycle" refers to a polycyclic group having at least three conjoined rings. Representative fused multicycle groups include anthrazine, phenanthroline, perimidine, trytanthrin and the like.

"Substituted" refers to the definite replacement of hydrogen with one or more monovalent or divalent radicals. Suitable substitution groups include, those described herein for particular groups, as well as hydroxyl, nitro, amino, imino, cyano, halo, thio, thioamido, amidino, imidino, oxo, oxamidino, methoxamidino, imidino, guanidino, sulfonamido, carboxyl, formyl, alkyl, substituted alkyl, haloalkyl, alkoxy, haloalkoxy, alkoxyalkyl, alkylcarbonyl, arylcarbonyl, aralkylcarbonyl, heteroarylcarbonyl, heteroaralkylcarbonyl, alkylthio, aminoalkyl, cyanoalkyl, benzyl, pyridyl, pyrazolyl, pyrrole, thiophene, imidazolyl, and the like. "Unsubstituted" refers to the group as defined with no further substitutions except for the appropriate number H substituents, as will be apparent to one skilled in the art. For example sulfonyl refers to -SO₂- and unsubstituted sulfonyl refers to -SO₂H.

The numbering of carbon atoms on the cyclical structures and superscripts on R-groups is to prevent degeneracy in R-group labeling. It is not meant to signify anything except a particular position on a group. For instance, 'R_{b} at position 2 is ethyl' would imply:

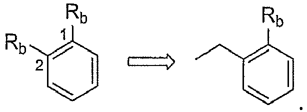

The invention also includes isotopically-labeled SMIP compounds, that are structurally identical to those disclosed above, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chlorine, such as ²H, ³H, ¹³C, ¹⁴C, ¹⁵N, ¹⁸O, ¹⁷O, ³¹P, ³²P, ³⁵S, ¹⁸F and ³⁶Cl, respectively. Compounds of the present invention, prodrugs thereof, and pharmaceutically acceptable salts of said compounds and of said prodrugs that contain the aforementioned isotopes and/or other isotopes of other atoms are within the scope of this invention. Certain isotopically labeled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased in vivo half-life or reduced dosage requirements and, hence, may be preferred in some circumstances. Isotopically labeled compounds of this invention and prodrugs thereof can generally be prepared by carrying out known or referenced procedures and by substituting a readily available isotopically labeled reagent for a non-isotopically labeled reagent.

Various compounds and methods of their synthesis are disclosed in the following US patents, US patent application publications, and PCT publications: US 4810692, US 4617315, US 4988680, US 5087619, US 5001124, US 4987139, US 5023264, US 5023263, US 4975372, US 4981792, US 5064835, US 5120842, US 5120727, US 5120725, US 5118678, US 5118677, US 5130307, US 5100883, US 5102876, US 5093338, US 5143918, US 5091389, US 5120726, US 5233036, US 5227467, US 5221740, US 5247076, US 5208241, US 5262423, US 5260300, US 5258389, US 5247119, US 5376663, US 5373014, US 5371225, US 5362735, US 5360794, US 5352783, US 5310903, US 5302584, US 5444072, US 5387680, US 5389639, US 6121257, US 6071947, US 6150373, US 6051590, US 6015809, US 5252732, US 5874411, US 5877184, US 5252732, US 5247076, US 5344925, US 5777105, US 5739169, US 5696156, US 5679705, US 5631282, US 5561228, US 5563145, US 5525610, US 5523408, US 5512687, US 5463048, US 5451604, US 5461054, US 5302584, US 5162334, US 5284877, US 5284840, US 5256790, US 5252579, US 5262533, US 2002016460, US 2002061905, US 6440991, US 6399626, US 6399773, US 6331547, US 6110922, US 5759550, US 5192773, US 5194447, US 5189042, US 5162333, US 5162334, US 5149701, US 5147877, US 4885276, US 4314061, US 4895872, US 4847299, US 4525299, US 5679640, US 5385910, US 5385908, US 5385909, US 5378836, US 5362718, US 5470878, US 5346912, US 5359073, US 6100259, US 5550214, US 5455230, US 6136817, US 5385910, US 5730979, US 58173 11, US 5189039, US 5171864, US 5169963, US 5183906, US 5006520, US 50913 81, US 5905090, US 5665772, US 5260323, US 5091381, US 4675382, US 5321009, US 5776943, US 5286730, US 5387589, US 5496832, US 4829061, US 49635 57, US 4648996, US 4351841, US 4284786, US 4965276, US 5268382, US 54591 63, US 5504084, US 5494911, US 5532259, US 5700823, US 5700822, US 56101 73, US 4968702, US 5639455, US 5466697, US 5366986, US 5173499, US 5391730, US 4968702, US 4910211, US 4968702, US 4603137, US 4347315, US 5051441, US 4894374, US 5470831, US 6034096, US 5356897, US 5478827, US 5624931, US 5219864, US 4894374, US 5530101, US 4753935, US 4786637, US 48943 66, US 4929611, US 4956352, US 5110811, US 5254562, US 5266692, US 49161 38, US 4940797, US 5087703, US 5011943, US 5194378, US 5196437, WO 8707276, WO 8805784, WO 8809183, WO 8908658, WO 9110650, WO 9102736, WO 9101982, WO 9200314, WO 9014359, WO 9200313, WO 9113899, WO 9113 889, WO 9211275, WO 9205189, WO 9205179, WO 9118901, WO 9203441, WO 9200980, WO 9200314, WO 9316083, WO 9311130, WO 9312125, WO 9310 122, WO 9310796, WO 9305059, WO 9305058, WO 9318050, WO 9318049, WO 9318048, WO 9318042, WO 9501355, WO 9429295, WO 9322286, WO 9425468, WO 9424095, WO 9420488, WO 9418208, WO 9418206, WO 9409010, WO 9522537, WO 9522536, WO 9408943, WO 951669 , WO 9515328, WO 9517381, WO 9514023, WO 9408943, WO 9506045, WO 9509857, WO 9504060, WO 9504738, WO 0058318, WO 0053190, WO 0058314, WO 0039129, WO 0034228, WO 0034248, WO 0032604, WO 9840380, WO 0024744, WO 0012514, WO 0009510, WO 0002879, WO 9955689, WO 9965909, WO 9965908, WO 9965450, WO 9947707, WO 9938829, WO 9940069, WO 9941239, WO 9509857, WO 9305058, WO 9931087, WO 9931060, WO 9929695, WO 9931066, WO 9931063, WO 9931064, WO 9925703, WO 9912890, WO 9920267, WO 9920274, WO 9915530, WO 9305058, WO 9305059, WO 9708182, WO 9828301, WO 9818780, WO 9816532, WO 9816531, WO 9816518, WO 9806719, WO 9809972, WO 9739999, WO 9748696, WO 9748695, WO 9744351 , WO 9744052, WO 9317699, WO 9740028, WO 9606068, WO 9720814, WO 9716438, WO 9716437, WO 9716182, WO 9716068, WO 9714410, WO 9712888, WO 9712887, WO 9711080, WO 9711092, WO 9709298, WO 9640688, WO 9641807, WO 9631529, WO 9304679, WO 9630381, WO 9625936, WO 9624579, WO 9617845, WO 9615131, WO 9611200, WO 9613510, WO 9535299, WO 95 35120, WO 9534568, WO 9534565, WO 9522538, WO 9515947, WO 9522534, WO 9522537, WO 9220688, WO 9402485, WO 9402137, WO 9402136, WO 94 04540, WO 0270472, WO 0253543, WO 0257216, WO 0257237, WO 0251397, WO 0125238, WO 0119829, WO 0230938, WO 0228867, WO 0228866, WO 02 18395, WO 0206268, WO 0025780, WO 0200661, WO 0190110, WO 0034296, WO 0033887, WO 0059880, WO 0039081, WO 0107401, WO 0105768, WO 01 05750, WO 0076953, WO 9426265, WO 9305058, WO 9222294, WO 9307871, WO 9221313, WO 9305046, WO 9220688, WO 9218496, WO 9010631, WO 90 07523, WO 9007520, WO 9002727, WO 9824762, WO 9809946, WO 9743434, WO 9809970, WO 9804521, WO 9732847, WO 9741148, WO 9739018, WO 97 23453, WO 9709325, WO 9702285, WO 9702820, WO 9709315, WO 9428910, WO 9422863, WO 9412500, WO 0062778, WO 0056331, WO 9831227, WO 0040562, WO 0025786, WO 9615105, WO 9617874, WO 9616967, WO 9611199, WO 9611198, WO 9603419, WO 9531463, WO 9531463, WO 9528394, WO 95 15309, WO 9422872, WO 0285928, WO 0272562 WO 0248122, WO 0250071, WO 0269904, WO 0228862, WO 0047595, WO 0125238, WO 0102359, WO 02 53150, WO 0062778, WO 9202229, WO 9303036, WO 9219610, WO 9219609, WO 8903385, WO 9205180, WO 0015645, WO 0002839, WO 9942105, WO 99 15501, WO 9856785, WO 9852933, WO 9852951, WO 9845249, WO 9832750, WO 9735575, WO 9409010, WO 9424304, WO 9507468, WO 9804279, WO 89 08113, WO 9405685, WO 9102000, WO 9214476, WO 9214477, WO 9319763, WO 9747317, WO 9818468, WO 8907613, WO 8902889, WO 8805783, WO 91 17748, WO 9519169, WO 0063210, WO 0048989, WO 9804521, WO 9746522, WO 9741148, WO 9739018, WO 9720811, WO 9705140, WO 9606855, WO 96 02541, WO 9210295, WO 9509153, WO 0255541, WO 0202539, WO 0063210, WO 9509153, WO 9513277 WO 9508535, WO 9501175, WO 9424133, WO 9408589, WO 9415959, WO 8906968, WO 0015604, WO 9932138, WO 9900143, WO 9852606, WO 9117748, WO 9116339, WO 9520589, WO 9211021, WO 92 13874, WO 9500543, WO 9531206, WO 9520589, WO 9715561, WO 9743251, WO 9741104, WO 9743251, WO 9723453, WO 9715561, WO 9705141, WO 9640143, WO 9749399, WO 9412184, WO 9426266, WO 9507902, WO 9509626, WO 9014826, WO 9117754, WO 9119495, WO 9308802 and WO 9314771, which have been found within context of this invention to be useful for immune potentiation. The entire disclosure of these U.S. and international publications is incorporated herein by this reference. Other compounds or intermediates of interest in the present invention were purchased from commercially available sources using the following method= the chemical structure of interest was drawn into the ACD-SC database (from MDL Information Systems). A search of the following companies/institutions, among others, retrieved the identified compound's supplier and purchasing information: ASDI, ASINEX, BIONET, CHEMBRIDGE, CHEMDIV, CHEMEX, CHEMSTAR, COMGENEX, CSC, INTERBIOSCREEN, LABOTEST, MAYBRIDGE, MICROSOURCE/GENESIS, OLIVIA, ORION, PEAKDALE, RYAN SCIENTIFIC, SPECS, TIMTEC, U OF FLORIDA, and ZELINSKY.
Compositions of the invention may be administered in conjunction with one or more antigens for use in therapeutic, prophylactic, or diagnostic methods of the present invention. Preferred antigens include those listed below. Additionally, the compositions of the present invention may be used to treat or prevent infections caused by any of the below-listed microbes.
In addition to combination with the antigens described below, the compositions of the invention may also be combined with an adjuvant as described herein. Antigens for use with the invention include, but are not limited to, one or more of the following antigens set forth below, or antigens derived from one or more or the pathogens set forth below:

### A. BACTERIAL ANTIGENS

Bacterial antigens suitable for use in the invention include proteins, polysaccharides, lipopolysaccharides, and outer membrane vesicles which may be isolated, purified or derived from a bacteria. In addition, bacterial antigens may include bacterial lysates and inactivated bacteria formulations. Bacteria antigens may be produced by recombinant expression. Bacterial antigens preferably include epitopes which are exposed on the surface of the bacteria during at least one stage of its life cycle. Bacterial antigens are preferably conserved across multiple serotypes. Bacterial antigens include antigens derived from one or more of the bacteria set forth below as well as the specific antigens examples identified below.

*Neisseria meningitides: Meningitides* antigens may include proteins (such as those identified in References 1 - 7), saccharides (including a polysaccharide, oligosaccharide or lipopolysaccharide), or outer-membrane vesicles (References 8, 9, 10, 11) purified or derived from *N*. *meningitides* serogroup A, C, W135, Y, and/or B. Meningitides protein antigens may be selected from adhesions, autotransporters, toxins, Fe acquisition proteins, and membrane associated proteins (preferably integral outer membrane protein).

*Streptococcus pneumoniae: Streptococcus pneumoniae* antigens may include a saccharide (including a polysaccharide or an oligosaccharide) or protein from *Streptococcus pneumoniae.* Saccharide antigens may be selected from serotypes 1, 2, 3, 4, 5, 6B, 7F, 8, 9N, 9V, 10A, 11A, 12F, 14, 15B, 17F, 18C, 19A, 19F, 20, 22F, 23F, and 33F. Protein antigens may be selected from a protein identified in WO 98/18931, WO 98/18930, US Patent No. 6,699,703, US Patent No. 6,800,744, WO 97/43303, and WO 97/37026. Streptococcus pneumoniae proteins may be selected from the Poly Histidine Triad family (PhtX), the Choline Binding Protein family (CbpX), CbpX truncates, LytX family, LytX truncates, CbpX truncate-LytX truncate chimeric proteins, pneumolysin (Ply), PspA, PsaA, Sp128, Sp101, Sp130, Sp125 or Sp133.

*Streptococcus pyogenes (Group A Streptococcus):* Group A Streptococcus antigens may include a protein identified in WO 02/34771 or WO 2005/032582 (including GAS 40), fusions of fragments of GAS M proteins (including those described in WO 02/094851, and Dale, Vaccine (1999) 17:193-200, and Dale, Vaccine 14(10): 944-948), fibronectin binding protein (Sfb1), Streptococcal heme-associated protein (Shp), and Streptolysin S (SagA).

*Moraxella catarrhalis:* Moraxella antigens include antigens identified in WO 02/18595 and WO 99/58562, outer membrane protein antigens (HMW-OMP), C-antigen, and/or LPS.

*Bordetella pertussis:* Pertussis antigens include petussis holotoxin (PT) and filamentous haemagglutinin (FHA) from *B. pertussis,* optionally also combination with pertactin and/or agglutinogens 2 and 3 antigen.

*Staphylococcus aureus:* Staph aureus antigens include S. *aureus* type 5 and 8 capsular polysaccharides optionally conjugated to nontoxic recombinant *Pseudomonas aeruginosa* exotoxin A, such as StaphVAX™, or antigens derived from surface proteins, invasins (leukocidin, kinases, hyaluronidase), surface factors that inhibit phagocytic engulfment (capsule, Protein A), carotenoids, catalase production, Protein A, coagulase, clotting factor, and/or membrane-damaging toxins (optionally detoxified) that lyse eukaryotic cell membranes (hemolysins, leukotoxin, leukocidin).

*Staphylococcus epidermis: S. epidermidis* antigens include slime-associated antigen (SAA).

*Tetanus:* Tetanus antigens include tetanus toxoid (TT), preferably used as a carrier protein in conjunction/conjugated with the compositions of the present invention.

*Diphtheria:* Diphtheria antigens include diphtheria toxin, preferably detoxified, such as CRM₁₉₇, additionally antigens capable of modulating, inhibiting or associated with ADP ribosylation are contemplated for combination/co-administration/conjugation with the compositions of the present invention, the diphtheria toxoids are preferably used as carrier proteins.

*Haemophilus influenzae B (Hib):* Hib antigens include a Hib saccharide antigen.

*Pseudomonas aeruginosa:* Pseudomonas antigens include endotoxin A, Wzz protein, *P. aeruginosa* LPS, more particularly LPS isolated from PAO 1 (05 serotype), and/or Outer Membrane Proteins, including Outer Membrane Proteins F (OprF) *(*Infect Immun. 2001 May; 69(5): 3510-3515).

*Legionella pneumophila* (Legionnairs' Disease): L. pneumophila antigens may optionally derived from cell lines with disrupted *asd* genes *(*Infect Immun. 1998 May; 66(5): 1898).

*Streptococcus agalactiae (Group B Streptococcus):* Group B Streptococcus antigens include a protein or saccharide antigen identified in WO 02/34771, WO 03/093306, WO 041041157, or WO 2005/002619 (including proteins GBS 80, GBS 104, GBS 276 and GBS 322, and including saccharide antigens derived from serotypes Ia, Ib, Ia/c, II, III, IV, V, VI, VII and VIII).

*Neiserria gonorrhoeae:* Gonorrhoeae antigens include Por (or porin) protein, such as PorB *(see* Zhu et al., Vaccine (2004) 22:660 - 669), a transferring binding protein, such as TbpA and TbpB (See Price et al., Infection and Immunity (2004) 71(1):277 - 283), a opacity protein (such as Opa), a reduction-modifiable protein (Rmp), and outer membrane vesicle (OMV) preparations *(see* Plante et al., J Infectious Disease (2000) 182:848 - 855), also see e.g. WO99/24578, WO99/36544, WO99/57280, WO02/079243).

*Chlamydia trachomatis:* Chlamydia trachomatis antigens include antigens derived from serotypes A, B, Ba and C are (agents of trachoma, a cause of blindness), serotypes L₁, L₂ & L₃ (associated with Lymphogranuloma venereum), and serotypes, D-K. Chlamydia trachomas antigens may also include an antigen identified in WO 00/37494, WO 03/049762, WO 03/068811, or WO 05/002619.

*Treponema pallidum* (Syphilis): Syphilis antigens include TmpA antigen.

*Haemophilus ducreyi* (causing chancroid): Ducreyi antigens include outer membrane protein (DsrA).

*Enterococcus faecalis or Enterococcus faecium:* Antigens include a trisaccharide repeat or other *Enterococcus* derived antigens provided in US Patent No. 6,756,361.

*Helieobacter pylori:* H pylori antigens include Cag, Vac, Nap, HopX, HopY and/or urease antigen.

*Staphylococcus saprophyticus:* Antigens include the 160 kDa hemagglutinin of S. *saprophyticus* antigen.

*Yersinia enterocolitica* Antigens include LPS (Infect Immun. 2002 August; 70(8): 4414).

*E. coli:* E. coli antigens may be derived from enterotoxigenic *E. coli* (ETEC), enteroaggregative *E. coli* (EAggEC), diffusely adhering *E*. *coli* (DAEC), enteropathogenic *E. coli* (EPEC), and/or enterohemorrhagic *E. coli* (EHEC).

*Bacillus anthracis* (anthrax): *B. anthracis* antigens are optionally detoxified and may be selected from A-components (lethal factor (LF) and edema factor (EF)), both of which can share a common B-component known as protective antigen (PA).

*Yersinia pestis* (plague): Plague antigens include F1 capsular antigen (Infect Immun. 2003 Jan; 71(1)): 374-383, LPS *(*Infect Immun. 1999 Oct; 67(10): 5395), Yersinia pestis V antigen *(*Infect Immun. 1997 Nov; 65(11): 4476-4482).

*Mycobacterium tuberculosis:* Tuberculosis antigens include lipoproteins, LPS, BCG antigens, a fusion protein of antigen 85B (Ag85B) and/or ESAT-6 optionally formulated in cationic lipid vesicles (Infect Immun. 2004 October; 72(10): 6148), Mycobacterium tuberculosis (Mtb) isocitrate dehydrogenase associated antigens (Prove Natl Acad Sci USA. 2004 Aug 24; 101(34): 12652), and/or MPT51 antigens (Infect Immun. 2004 July; 72(7): 3829).

*Rickettsia:* Antigens include outer membrane proteins, including the outer membrane protein A and/or B (OmpB) (Biochim Biophys Acta. 2004 Nov 1;1702(2):145), LPS, and surface protein antigen (SPA) *(*J Autoimmun. 1989 Jun;2 Suppl:81).

*Listeria monocytogenes* : Antigens derived from *L. monocytogenes* are preferably used as carriers/vectors for intracytoplasmic delivery of conjugates/associated compositions of the present invention.

*Chlamydia pneumoniae:* Antigens include those identified in WO 02/02606.

*Vibrio cholerae:* Antigens include proteinase antigens, LPS, particularly lipopolysaccharides of Vibrio cholerae II, O1 Inaba O-specific polysaccharides, V. cholera 0139, antigens of IEM108 vaccine (Infect Immun. 2003 Oct;71(10):5498-504), and/or Zonula occludens toxin (Zot).

*Salmonella typhi* (typhoid fever): Antigens include capsular polysaccharides preferably conjugates (Vi, i.e. vax-TyVi).

*Borrelia burgdorferi* (Lyme disease): Antigens include lipoproteins (such as OspA, OspB, Osp C and Osp D), other surface proteins such as OspE-related proteins (Erps), decorin-binding proteins (such as DbpA), and antigenically variable VI proteins. , such as antigens associated with P39 and P13 (an integral membrane protein, Infect Immun. 2001 May; 69(5): 3323-3334), VIsE Antigenic Variation Protein *(*J Glin Microbiol. 1999 Dec; 37(12): 3997).

*Porphyromonas gingivalis:* Antigens include *P. gingivalis* outer membrane protein (OMP).

*Klebsiella:* Antigens include an OMP, including OMP A, or a polysaccharide optionally conjugated to tetanus toxoid.

Where not specifically referenced, further bacterial antigens of the invention may be capsular antigens, polysaccharide antigens or protein antigens of any of the above. Further bacterial antigens may also include an outer membrane vesicle (OMV) preparation. Additionally, antigens include live, attenuated, split, and/or purified versions of any of the aforementioned bacteria. The bacterial or microbial derived antigens of the present invention may be gram-negative or gram-positive and aerobic or anaerobic.

Additionally, any of the above bacterial-derived saccharides (polysaccharides, LPS, LOS or oligosaccharides) can be conjugated to another agent or antigen, such as a carrier protein (for example CRM₁₉₇). Such conjugation may be direct conjugation effected by reductive amination of carbonyl moieties on the saccharide to amino groups on the protein, as provided in US Patent No. 5,360,897 and Can J Biochem Cell Biol, 1984 May;62(5):270-5. Alternatively, the saccharides can be conjugated through a linker, such as, with succinamide or other linkages provided in Bioconjugate Techniques, 1996 and CRC, Chemistry of Protein Conjugation and Cross-Linking, 1993.

### B. VIRAL ANTIGENS

Viral antigens suitable for use in the invention include inactivated (or killed) virus, attenuated virus, split virus formulations, purified subunit formulations, viral proteins which may be isolated, purified or derived from a virus, and Virus Like Particles (VLPs). Viral antigens may be derived from viruses propagated on cell culture or expressed recombinantly. Viral antigens preferably include epitopes which are exposed on the surface of the virus during at least one stage of its life cycle. Viral antigens are preferably conserved across multiple serotypes. Viral antigens include antigens derived from one or more of the viruses set forth below as well as the specific antigens examples identified below.

*Orthomyxovirus:* Viral antigens may be derived from an Orthomyxovirus, such as Influenza A, B and C. Orthomyxovirus antigens may be selected from one or more of the viral proteins, including hemagglutinim (HA), neuraminidase (NA), nucleoprotein (NP), matrix protein (M1), membrane protein (M2), one or more of the transcriptase components (PB1, PB2 and PA). Preferred antigens include HA and NA.

Influenza antigens may be derived from interpandemic (annual) flu strains. Alternatively influenza antigens may be derived from strains with the potential to cause pandemic a pandemic outbreak (i.e., influenza strains with new haemagglutinin compared to the haemagglutinin in currently circulating strains, or influenza strains which are pathogenic in avian subjects and have the potential to be transmitted horizontally in the human population, or influenza strains which are pathogenic to humans).

*Paramyxoviridae* viruses: Viral antigens may be derived from Paramyxoviridae viruses, such as Pneumoviruses (RSV), Paramyxoviruses (PIV) and Morbilliviruses (Measles).

*Pneumovirus:* Viral antigens may be derived from a Pneumovirus, such as Respiratory syncytial virus (RSV), Bovine respirartory syncytial virus, Pneumonia virus of mice, and Turkey rhinotracheitis virus. Preferably, the Pneumovirus is RSV. Pneumovirus antigens may be selected from one or more of the following proteins, including surface proteins Fusion (F), Glycoprotein (G) and Small Hydrophobic protein (SH), matrix proteins M and M2, nucleocapsid proteins N, P and L and nonstructural proteins NS1 and NS2. Preferred Pneumovirus antigens include F, G and M. See e.g., J Gen Virol. 2004 Nov; 85(Pt 11):3229). Pneumovirus antigens may also be formulated in or derived from chimeric viruses. For example, chimeric RSV/PIV viruses may comprise components of both RSV and PIV.

*Paramyxovirus:* Viral antigens may be derived from a Paramyxovirus, such as Parainfluenza virus types 1 - 4 (PIV), Mumps, Sendai viruses, Simian virus 5, Bovine parainfluenza virus and Newcastle disease virus. Preferably, the Paramyxovirus is PIV or Mumps. Paramyxovirus antigens may be selected from one or more of the following proteins: Hemagglutinin -Neuraminidase (HN), Fusion proteins F1 and F2, Nucleoprotein (NP), Phosphoprotein (P), Large protein (L), and Matrix protein (M). Preferred Paramyxovirus proteins include HN, F1 and F2. Paramyxovirus antigens may also be formulated in or derived from chimeric viruses. F or example, chimeric RSV/PIV viruses may comprise components of both RSV and PIV. Commercially available mumps vaccines include live attenuated mumps virus, in either a monovalent form or in combination with measles and rubella vaccines (MMR).

*Morbillivirus:* Viral antigens may be derived from a Morbillivirus, such as Measles. Morbillivirus antigens may be selected from one or more of the following proteins: hemagglutinin (H), Glycoprotein (G), Fusion factor (F), Large protein (L), Nucleoprotein (NP), Polymerase phosphoprotein (P), and Matrix (M). Commercially available measles vaccines include live attenuated measles virus, typically in combination with mumps and rubella (MMR).

*Picornavirus:* Viral antigens may be derived from Picornaviruses, such as Enteroviruses, Rhinoviruses, Heparnavirus, Cardioviruses and Aphthoviruses. Antigens derived from Enteroviruses, such as Poliovims are preferred.

*Enterovirus:* Viral antigens may be derived from an Enterovirus, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71. Preferably, the Enterovirus is poliovirus- Enterovirus antigens are preferably selected from one or more of the following Capsid proteins VP1, VP2, VP3 and VP4. Commercially available polio vaccines include Inactivated Polio Vaccine (IPV) and Oral poliovirus vaccine (OPV).

*Heparnavirus:* Viral antigens may be derived from an Heparnavirus, such as Hepatitis A virus (HAV). Commercially available HAV vaccines include inactivated HAV vaccine.

*Togavirus:* Viral antigens may be derived from a Togavirus, such as a Rubivirus, an Alphavirus, or an Arterivirus. Antigens derived from Rubivirus, such as Rubella virus, are preferred. Togavirus antigens may be selected from EL , E2, E3, C, NSP-1, NSPO-2, NSP-3 or NSP-4. Togavirus antigens are preferably selected from E1, E2 or E3. Commercially available Rubella vaccines include a live cold-adapted virus, typically in combination with mumps and measles vaccines (MMR).

*Flavivirus:* Viral antigens may be derived from a Flavivirus, such as Tick-borne encephalitis (TBE), Dengue (types 1, 2, 3 for 4), Yellow Fever, Japanese encephalitis, West Nile encephalitis, St. Louis encephalitis, Russian spring-summer encephalitis, Powassan encephalitis. Flavivirus antigens may be selected from PrM, M, C, E, NS-1, NS-2a, NS2b, NS3, NS4a, NS4b, and NS5. Flavivirus antigens are preferably selected from PrM, M and E. Commercially available TBE vaccine include inactivated virus vaccines.

*Pestivirus:* Viral antigens may be derived from a Pestivirus, such as Bovine viral diarrhea (BVDV), Classical swine fever (CSFV) or Border disease (BDV).

*Hepadnavirus:* Viral antigens may be derived from a Hepadnavirus, such as Hepatitis B virus. Hepadnavirus antigens may be selected from surface antigens (L, M and S), core antigens (HBc, HBe). Commercially available HBV vaccines include subunit vaccines comprising the surface antigen S protein.

*Hepatitis C virus:* Viral antigens may be derived from a Hepatitis C virus (HCV). HCV antigens may be selected from one or more of E1, E2, E1/E2, NS345 polyprotein, NS 345-core polyprotein, core, and/or peptides from the nonstructural regions (Houghton et al., Hepatology (1991) 14:381).

*Rhabdovirus:* Viral antigens may be derived from a Rhabdovirus, such as a Lyssavirus (Rabies virus) and Vesiculovirus (VSV). Rhabdovirus antigens may be selected from glycoprotein (G), nucleoprotein (N), large protein (L), nonstructural proteins (NS). Commercially available Rabies virus vaccine comprise killed virus grown on human diploid cells or fetal rhesus lung cells.

*Caliciviridae;* Viral antigens may be derived from Calciviridae, such as Norwalk virus.

*Coronavirus:* Viral antigens may be derived from a Coronavirus, SARS, Human respiratory coronavirus, Avian infectious bronchitis (IBV), Mouse hepatitis virus (MHV), and Porcine transmissible gastroenteritis virus (TGEV). Corona virus antigens may be selected from spike (S), envelope (E), matrix (M), nucLeocapsid (N), and Hemagglutinin-esterase glycoprotein (HE). Preferably, the Coronavirus antigen is derived from a SARS virus. SARS viral antigens are described in WO 04/92360;

Retrovirus: Viral antigens may be derived from a Retrovims, such as an Oncovirus, a Lentivirus or a Spumavirus. Oncovirus antigens may be derived from HTLV-1, HTLV-2 or HTLV-5. Lentivirus antigens may be derived from HIV-1 or HIV-2. Retrovirus antigens may be selected from gag, pol, env, tax, tat, rex, rev, nef, vif, vpu, and vpr. HIV antigens may be selected from gag (p24gag and p55gag), env (gp160 and gp41), pol, tat, nef, rev vpu, miniproteins, (preferably p55 gag and gp140v delete). HIV antigens may be derived from one or more of the following strains: HIV_{IIIb}, HIV_{SF2}, HIV_{LAV}, HIV_{LAI}, HIV_{MN}, HIV-1_{CM235}, HIV-1_{US4}.

*Reovirus:* Viral antigens may be derived from a Reovirus, such as an Orthoreovirus, a Rotavirus, an Orbivirus, or a Coltivirus. Reovirus antigens may be selected from structural proteins λ1, λ2, λ3, *µ*1, *µ*2, σ1, σ2, or σ3, or nonstructural proteins σNS, *µ*NS, or σ1s. Preferred Reovirus antigens may be derived from a Rotavirus. Rotavirus antigens may be selected from VP1, VP2, VP3, VP4 (or the cleaved product VP5 and VP8), NSP 1, VP6, NSP3, NSP2, VP7, NSP4, or NSPS. Preferred Rotavirus antigens include VP4 (or the cleaved product VP5 and VP8), and VP7.

*Parvovirus:* Viral antigens may be derived from a Parvovirus, such as Parvovirus B 19. Parvovirus antigens may be selected from VP-1, VP-2, VP-3, NS-1 and NS-2. Preferably, the Parvovirus antigen is capsid protein VP-2.

*Delta hepatitis virus (HDV):* Viral antigens may be derived HDV, particularly δ-antigen from HDV (see, e.g., U.S. Patent No. 5,378,814).

*Hepatitis E virus (HEV):* Viral antigens may be derived from HEV.

*Hepatitis G virus (HGV):* Viral antigens may be derived from HGV.

*Human Herpesvirus:* Viral antigens may be derived from a Human Herpesvirus, such as Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8). Human Herpesvirus antigens may be selected from immediate early proteins (α), early proteins (*β*), and late proteins (γ). HSV antigens may be derived from HSV-1 or HSV-2 strains. HSV antigens may be selected from glycoproteins gB, gC, gD and gH, fusion protein (gB), or immune escape proteins (gC, gE, or gI). VZV antigen may be selected from core, nucleocapsid, tegument, or envelope proteins. A live attenuated VZV vaccine is commercially available. EBV antigens may be selected from early antigen (EA) proteins, viral capsid antigen (VCA), and glycoproteins of the membrane antigen (MA). CMV antigens may be selected from capsid proteins, envelope glycoproteins (such as gB and gH), and tegument proteins

*Papovaviruses:* Antigens may be derived from Papovaviruses, such as Papillomaviruses and Polyomaviruses. Papillomaviruses include HPV serotypes 1, 2, 4, 5, 6, 8, 11, 13, 16, 18, 31, 33, 35, 39, 41, 42, 47, 51, 57, 58, 63 and 65. Preferably, HPV antigens are derived from serotypes 6, 11, 16 or 18. HPV antigens may be selected from capsid proteins (L1) and (L2), or E1 - E7, or fusions thereof. HPV antigens are preferably formulated into virus-like particles (VLPs). Polyomyavirus viruses include BK virus and JK virus. Polyomavirus antigens may be selected from VP1, VP2 or VP3.

Further provided are antigens, compositions, methods, and microbes included in Vaccine, 4th Edition (Plotkin and Orenstein ed. 2004); Medical Microbiology 4th Edition (Murray et al. ed. 2002); Virology, 3rd Edition (W.K. Joklik ed. 1988); Fundamental Virology, 2nd Edition (B.N. Fields and D.M. Knipe, eds. 1991), which are contemplated in conjunction with the compositions of the present invention.

### FUNGAL ANTIGENS

In some embodiments compositions of the present invention are combined with fungal antigens for use in methods of the present invention, including treatment or prevention of mycoses. Fungal antigens for use herein, associated with vaccines include those described in: U.S. Pat. Nos. 4,229,434 and 4,368,191 for prophylaxis and treatment of trichopytosis caused by Trichophyton mentagrophytes; U. S. Pat. Nos. 5,277,904 and 5,284,652 for a broad spectrum dermatophyte vaccine for the prophylaxis of dermatophyte infection in animals, such as guinea pigs, cats, rabbits, horses and lambs, these antigens comprises a suspension of killed *T. equinum, T.* mentagrophytes (var. granulare), *M. canis* and/or *M gypseum* in an effective amount optionally combined with an adjuvant; U.S. Pat. Nos. 5,453,273 and 6, 132, 733 for a ringworm vaccine comprising an effective amount of a homogenized, formaldehyde-killed fungi, i.e., *Microsporum canis* culture in a carrier; U.S. Pat. No. 5,948,413 involving extracellular and intracellular proteins for pythiosis. Additional antigens identified within antifungal vaccines include Ringvac bovis LTF-130 and Bioveta.

Further, fungal antigens for use herein may be derived from Dennatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme.*

Fungal pathogens for use as antigens or in derivation of antigens in conjunction with the compositions of the present invention comprise *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliennondi, Cladosporiuin carrionii, Coccidioides immitis, Blastomyces dermatidis, Cryptococcus neoformans, Geotrichum clavatum, Histoplasma capsulatum, Klebsiella pneumoniae, Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei,* Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, and Saksenaea spp.

Other fungi from which antigens are derived include Alternaria spp, Curvularia spp, Helminthosporium spp, Fusarium spp, Aspergillus spp, Penicillium spp, Monolinia spp, Rhizoctonia spp, Paecilomyces spp, Pithomyces spp, and Cladosporium spp.

Processes for producing a fungal antigens are well known in the art (see US Patent No. 6,333,164). In a preferred method a solubilized fraction extracted and separated from an insoluble fraction obtainable from fungal cells of which cell wall has been substantially removed or at least partially removed, characterized in that the process comprises the steps of: obtaining living fungal cells; obtaining fungal cells of which cell wall has been substantially removed or at least partially removed; bursting the fungal cells of which cell wall has been substantially removed or at least partially removed; obtaining an insoluble fraction; and extracting and separating a solubilized fraction from the insoluble fraction.

### STD ANTIGENS

Embodiments of the invention include compositions and methods related to a prophylactic and therapeutic treatments for microbes that can be neutralized prior to infection of a cell. In particular embodiments, microbes (bacteria, viruses and/or fungi) against which the present compositions and methods can be implement include those that cause sexually transmitted diseases (STDs) and/or those that display on their surface an antigen that can be the target or antigen composition of the invention. In a preferred embodiment of the invention, compositions are combined with antigems derived from a viral or bacterial STD. Antigens derived from bacteria or viruses can be administered in conjunction with the compositions of the present invention to provide protection against at least one of the following STDs, among others: chlamydia, genital herpes, hepatitis (particularly HCV), genital warts, gonorrhoea, syphilis and/or chancroid (See, WO00/15255).

In another embodiment the compositions of the present invention are co-administered with an antigen for the prevention or treatment of an STD.

Antigens derived from the following viruses associated with STDs, which are described in greater detail ab ove, are preferred for co-administration with the compositions of the present invention: hepatitis (particularly HCV), HPV, HIV, or HSV.

Additionally, antigens derived from the following bacteria associated with STDs, which are described in greater detail above, are preferred for co-administration with the compositions of the present invention: *Neiserria gonorrhoeae, Chlamydia pneumoniae, Chlamydia trachomatis, Treponema pallidum,* or *Haemophilus ducreyl_*

### RESPIRATORY ANTIGENS

The invention provides methods of preventing and/or treating infection by a respiratory pathogen, including a virus, bacteria, or fungi such as respiratory syncytial virus (RSV), PIV, SARS virus, influenza, *Bacillus anthracis,* particularly by reducing or preventing infection and/or one or more symptoms of respiratory virus infection. A composition comprising an antigen described herein, such as one derived from a respiratory virus, bacteria or fungus is administered in conjunction with the compositions of the present invention to an individual which is at risk of being exposed to that particular respiratory microbe, has been exposed to a respiratory microbe or is infected with a respiratory virus, bacteria or fungus. The composition(s) of the present invention is/are preferably co-administered at the same time or in the same formulation with an antigen of the respiratory pathogen. Administration of the composition results in reduced incidence and/or severity of one or more symptoms of respiratory infection.

### TUMOR ANTIGENS

One embodiment of the present involves a tumor antigen or cancer antigen in conjunction with the compositions of the present invention. Tumor antigens can be, for example, peptide-containing tumor antigens, such as a polypeptide tumor antigen or glycoprotein tumor antigens. A tumor antigen can also be, for example, a saccharide-containing tumor antigen, soch as a glycolipid tumor antigen or a ganglioside tumor antigen. The tumor antigen can further be, for example, a polynucleotide-containing tumor antigen that expresses a polypeptide-containing tumor antigen, for instance, an RNA vector construct or a DNA vector construct, such as plasmid DNA.

Tumor antigens appropriate for the practice of the present invention encompass a wide variety of molecules, such as (a) polypeptide-containing tumor antigens, including polypeptides (which can range, for example, from 8-20 amino acids in length, although lengths outside this range are also common), lipopolypeptides and glycoproteins, (b) saccharide-containing tumor antigens, including poly-saccharides, mucins, garigliosides, glycolipids and glycoproteins, and (c) polynucleotides that express antigenic polypeptides.

The tumor antigens can be, for example, (a) full length molecules associated with cancer cells, (b) homologs and modified forms of the same, including molecules with deleted, added and/or substituted portions, and (c) fragments of the same. Tumor antigens can be provided in recombinant form. Tumor antigens include, for example, class I-restricted antigens recognized by CD8+ lymphocytes or class II-restricted antigens recognized by CD4+ lymphocytes.

Numerous tumor antigens are known in the art, including: (a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors), (b) mutated antigens, for example, p53 (associated with various solid tumors, e.g., colorectal, lung, head and neck cancer), p21/Ras (associated with, e.g., melanoma, pancreatic cancer and colorectal cancer), CDK4 (associated with, e.g., melanoma), MUM1 (associated with, e.g., melanoma), caspase-8 (associated with, e.g., head and neck cancer), CIA 0205 (associated with, e.g., bladder cancer), HLA-A2-R1701, beta catenin (associated with, e.g., melanoma), TCR (associated with, e.g., T-cell non-Hodgkins lymphoma), BCR-abl (associated with, e.g., chronic myelogenous leukemia), triosephosphate isomerase, KIA 0205, CDC-27, and LDLR-FUT, (c) over-expressed antigens, for example, Galectin 4 (associated with, e.g., colorectal cancer), Galectin 9 (associated with, e.g., Hodgkin's disease), proteinase 3 (associated with, e.g., chronic myelogenous leukemia), WT 1 (associated with, e.g., various leukemias), carbonic anhydrase (associated with, e.g., renal cancer), aldolase A (associated with, e.g., lung cancer), PRAME (associated with, e.g., melanoma), HER-2/neu (associated with, e.g., breast, colon, lung and ovarian cancer), alpha-fetoprotein (associated with, e.g., hepatoma), KSA (associated with, e.g., colorectal cancer), gastrin (associated with, e.g., pancreatic and gastric cancer), telomerase catalytic protein, MUC-1 (associated with, e.g., breast and ovarian cancer), G-250 (associated with, e.g., renal cell carcinoma), p53 (associated with, e.g., breast, colon cancer), and carcinoembryonic antigen (associated with, e.g., breast cancer, lung cancer, and cancers of the gastrointestinal tract such as colorectal cancer), (d) shared antigens, for example, melanoma-melanocyte differentiation antigens such as MART-1/Melan A, gp100, MC1R, melanocyte-stimulating hormone receptor, tyrosinase, tyrosinase related protein-I/TRP1 and tyrosinase related protein-2/TRP2 (associated with, e.g., melanoma), (e) prostate associated antigens such as PAP, PSA, PSMA, PSH-P1, PSM-P1, PSM-P2, associated with e.g., prostate cancer, (f) immunoglobulin idiotypes (associated with myeloma and B cell lymphomas, for example), and (g) other tumor antigens, such as polypeptide- and saccharide-containing antigens including (i) glycoproteins such as sialyl Tn and sialyl Le^{x} (associated with, e.g., breast and colorectal cancer) as well as various mucins; glycoproteins may be coupled to a carrier protein (e.g., MUC-1 may be coupled to KLH); (ii) lipopolypeptides (e.g., MUC-1 linked to a lipid moiety); (iii) polysaccharide s (e.g., Globo H synthetic hexasaccharide), which may be coupled to a carrier proteins (e.g., to KLH), (iv) gangliosides such as GM2, GM12, GD2, GD3 (associated with, e.g., brain, lung cancer, melanoma), which also may be coupled to carrier proteins (e.g., KLH). Additional tumor antigens which are known in the art include p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and the like. These as well as other cellular components are described for example in United States Patent Application 20020007173 and references cited therein.

Polynucleotide-containing antigens in accordance with the present invention typically comprise polynucleotides that encode polypeptide cancer antigens such as those listed above. Preferred polynucleotide-containting antigens include DNA or RNA vector constructs, such as plasmid vectors (e.g., pCMV), which are capable of expressing polypeptide cancer antigens *in vivo.*

Tumor antigens may be derived, for example, from mutated or altered cellular components. After alteration, the cellular components no longer perform their regulatory functions, and hence the cell may experience uncontrolled growth. Representative examples of altered cellular components include ras, p53, Rb, altered protein encoded by the Wilms' tumor gene, ubiquitin, mucin, protein encoded by the DCC, APC, and MCC genes, as well as receptors or receptor-like structures such as neu, thyroid hormone receptor, platelet derived growth factor (PDGF) receptor, insulin receptor, epidermal growth factor (EGF) receptor, and the colony stimulating factor (CSF) receptor. These as well as other cellular components are described for example in U.S. Patent No. 5,693,522 and references cited therein.

Additionally, bacterial and viral antigens, may be used in conjunction with the compositions of the present invention for the treatment of cancer. In particular, carrier proteins, such as CRM₁₉₇, tetanus toxoid, or *Salmonella typhimurium* antigen can be used in conjunction/conjugation with compounds of the present invention for treatment of cancer. The cancer antigen combination therapies will show increased efficacy and bioavailability as compared with existing therapies.

Additional information on cancer or tumor antigens can be found, for example, in Moingeon P, "Cancer vaccines," Vaccine, 2001, 19:1305-1326; Rosenberg SA, "Progress in human tumor immunology and immunotherapy," Nature, 2001, 411:380-384; Dermine, S. et al, "Cancer Vaccines and Immunotherapy," British Medical Bulletin, 2002, 62, 149-162; Espinoza-Delgado I., "Cancer Vaccines," The Oncologist, 2002, 7(suppl3):20-33; Davis, I.D. et al., "Rational approaches to human cancer immunotherapy," Journal of Leukocyte Biology, 2003, 23: 3-29; Van den Eynde B, et al., "New tumor antigens recognized by T cells," Curr. Opin. Immunol., 1995, 7:674-81; Rosenberg SA, "Cancer vaccines based on the identification of genes encoding cancer regression antigens, Imnunol. Today, 1997, 18:175-82; Offiinga R et al., "Design and evaluation of antigen-specific vaccination strategies against cancer," Current Opin. Immunol., 2000, 2:576-582; Rosenberg SA, "A new era for cancer immunotherapy based on the genes that encode cancer antigens," Immunity, 1999, 10:281-7; Sahin U et al., "Serological identification of human tumor antigens," Curr. Opin. Immunol., 1997, 9:709-16; Old LJ et al., "New paths in human cancer serology," J. Exp. Med., 1998, 187:1163-7; Chaux P, et al., "Identification of MAGE-3 epitopes presented by HLA-DR molecules to CD4(+) T lymphocytes," J. Exp. Med., 1999, 189:767-78; Gold P, et al., "Specific carcinoembryonic antigens of the human digestive system," J. Exp. Med., 1965, 122:467-8; Livingston PO, et al., Carbohydrate vaccines that induce antibodies against cancer: Rationale," Cancer Immunol. Immunother., 1997, 45:1-6; Livingston PO, et al., Carbohydrate vaccines that induce antibodies against cancer: Previous experience and future plans," Cancer Immunol. Immunother., 1997, 45:10-9; Taylor-Papadimitriou J, "Biology, biochemistry and immunology of carcinoma-associated mucins," Immunol. Today, 1997, 18:105-7; Zhao X-J et al., "GD2 oligosaccharide: target for cytotoxic T lymphocytes," J. Exp. Med., 1995, 182:67-74; Theobald M, et al., "Targeting p53 as a general tumor antigen," Proc. Natl. Acad. Sci. USA, 1995, 92:11993-7; Gaudernack G, "T cell responses against mutant ras: a basis for novel cancer vaccines," Immunotechnology, 1996,2:3-9; WO 91/02062; U.S. Patent No. 6,015,567; WO 01/08636; WO 96/30514; U.S. Patent No. 5,846,538; and U.S. Patent No. 5,869,445.

### PEDIATRIC/GERIATRIC ANTIGENS

In one embodiment the compositions of the present invention are used in conjunction with an antigen for treatment of a pediatric populati on, as in a pediatric antigen. In a more particular embodiment the pediatric population is less than about 3 years old, or less than about 2 years, or less than about 1 years old. In another embodiment the pediatric antigen (in conjunction with the composition of the present invention) is administered multiple times over at least 1, 2, or 3 years.

In another embodiment the compositions of the present invention are used in conjunction with an antigen for treatment of a geriatric population, as in a geriatric antigen.

### OTHER ANTIGENS

Other antigens for use in conjunction with the compositions of the present include hospital acquired (nosocomial) associated antigens.

In another embodiment, parasitic antigens are contemplated in conjunction with the compositions of the present invention. Examples of parasitic antigens include those derived from organisms causing malaria and/or Lyme disease.

In another embodiment, the antigens in conjunction with the compositions of the present invention are associated with or effective against a mosquito born illness. In another embodiment, the antigens in conjunction with the compositions of the present invention are associated with or effective against encephalitis. In another embodiment the antigens in conjunction with the compositions of the present invention are associated with or effective against an infection of the nervous system.

In another embodiment, the antigens in conjunction with the compositions of the present invention are antigens transmissible through blood or body fluids.

### ANTIGEN FORMULATIONS

In other aspects of the invention, methods of producing microparticles having adsorbed antigens are provided. The methods comprise: (a) providing an emulsion by dispersing a mixture comprising (i) water, (ii) a detergent, (iii) an organic solvent, and (iv) a biodegradable polymer selected from the group consisting of a poly(α-hydroxy acid), a polyhydroxy butyric acid, a polycaprolactone, a polyorthoester, a polyanhydride, and a polycyanoacrylate. The polymer is typically present in the mixture at a concentration of about 1% to about 30% relative to the organic solvent, while the detergent is typically present in the mixture at a weigh-to-weight detergent-to-polymer ratio of from about 0.00001:1 to about 0.1:1 (more typically about 0.0001:1 to about 0.1:1, about 0.001:1 to about 0.1:1, or about 0.005:1 to about 0.1:1); (b) removing the organic solvent from the emulsion; and (c) adsorbing an antigen on the surface of the microparticles. In certain embodiments, the biodegradable polymer is present at a concentration of about 3% to about 10% relative to the organic solvent.

Microparticles for use herein will be formed from materials that are sterilizable, non-toxic and biodegradable. Such materials include, without limitation, poly(α-hydroxy acid), polyhydroxybutyric acid, polycaprolactone, polyorthoester, polyanhydride, PACA, and polycyanoacrylate. Preferably, microparticles for use with the present invention are derived from a poly(α-hydroxy acid), in particular from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials which have a variety of molecular weights and, in the case of the copolymers such as PLG, a variety of lactide:glycolide ratios, the selection of which will be largely a matter of choice, depending in part on the coadministered macromolecule. These parameters are discussed more fully below.

Further antigens may also include an outer membrane vesicle (OMV) preparation.

Additional formulation methods and antigens (especially tumor antigens) are provided in U.S. Patent Serial No. 09/581,772.

### ANTIGEN REFERENCES

The following references include antigens useful in conjunction with the compositions and methods of the present invention:
1 International patent application WO99/24578
2 International patent application WO99/36544.
3 International patent application WO99/57280.
4 International patent application WO00/22430.
5 Tettelin et al. (2000) Science 287:1809-1815.
6 International patent application WO96/29412.
7 Pizza et al. (2000) Science 287:1816-1820.
8 PCT WO 01/52885.
9 Bjune et al. (1991) Lancet 338(8775).
10 Fuskasawa et al. (1999) Vaccine 17:2951-2958.
11 Rosenqist et al. (1998) Dev. Biol. Strand 92:323-333.
12 Constantino et al. (1992) Vaccine 10:691-698.
13 Constantino et al. (1999) Vaccine 17:1251-1263.
14 Watson (2000) Pediatr Infect Dis J 19:331-332.
15 Rubin (20000) Pediatr Clin North Am 47:269-285,v.
16 Jedrzejas (2001) Microbiol Mol Biol Rev 65:187-207.
17 International patent application filed on 3^{rd} July 2001 claiming priority from GB-0016363.4;WO 02/02606; PCT IB/01/00166.
18 Kalman et al. (1999) Nature Genetics 21:385-389.
19 Read et al. (2000) Nucleic Acids Res 28:1397-406.
20 Shirai et al. (2000) J. Infect. Dis 181(Suppl 3):S524-S527.
21 International patent application WO99/27105.
22 International patent application WO00/27994.
23 International patent application WO00/37494.
24 International patent application WO99/28475.
25 Bell (2000) Pediatr Infect Dis J 19:1187-1188.
26 Iwarson (1995) APMIS 103:321-326.
27 Gerlich et al. (1990) Vaccine 8 Suppl:S63-68 & 79-80.
28 Hsu et al. (1999) Clin Liver Dis 3:901-915.
29 Gastofsson et al. (1996) N. Engl. J. Med. 334-:349-355.
30 Rappuoli et al. (1991) TIBTECH 9:232-238.
31 Vaccines (1988) eds. Plotkin & Mortimer. ISBN 0-7216-1946-0.
32 Del Guidice et al. (1998) Molecular Aspects of Medicine 19:1-70.
33 International patent application WO93/018150.
34 International patent application WO99/53310.
35 International patent application WO98/04702.
36 Ross et al. (2001) Vaccine 1 9:135-142.
37 Sutter et al. (2000) Pediatr Clin North Am 47:287-308.
38 Zimmerman & Spann (1999) Am Fan Physician 59:113-118, 125-126.
39 Dreensen (1997) Vaccine 15 Suppl"S2-6.
40 MMWR Morb Mortal Wkly rep 1998 Jan 16:47(1):12, 9.
41 McMichael (2000) Vaccine 19 Suppl 1: S101-107.
42 Schuchat (1999) Lancer 353 (9146):51-6.
43 GB patent applications 0026333.5, 0028727.6 & 0105640.7.
44 Dale (1999) Infect Disclin North Am 13:227-43, viii.
45 Ferretti et al. (2001) PNAS USA 98: 4658-4663.
46 Kuroda et al. (2001) Lancet 357(9264):1225-1240; see also pages 1218-1219.
47 Ramsay et al. (2001) Lancet 357(9251):195-196.
48 Lindberg (1999) Vaccine 17 Suppl 2:S28-36.
49 Buttery & Moxon (2000) J R Coil Physicians Long 34:163-168.
50 Ahmad & Chapnick (1999) Infect Dis Clin North Am 13:113-133, vii.
51 Goldblatt (1998) J. Med. Microbiol. 47:663-567.
52 European patent 0 477 508.
53 U.S. Patent No. 5,306,492.
54 International patent application WO98/42721.
55 Conjugate Vaccines (eds. Cruse et al.) ISBN 3805549326, particularly vol. 10:48-114.
56 Hermanson (1996) Bioconjugate Techniques ISBN: 012323368 & 012342335X.
57 European patent application 0372501.
58 European patent application 0378881.
59 European patent application 0427347.
60 International patent application WO93/17712.
61 International patent application WO98/58668
62 European patent application 0471177.
63 International patent application WO00/56360.
64 International patent application WO00/67161.

The contents of all of the above cited patents, patent applications and journal articles are incorporated by reference as if set forth fully herein.

Nomenclature for the Example compounds was provided using ACD Name version 5.07 software (November 14, 2001) available from Advanced Chemistry Development, Inc. Some of the compounds and starting materials were named using standard IUPAC nomenclature.

The foregoing may be better understood by reference to the following Examples, Methods, and Schemes that are presented for illustration and not to limit the scope of the inventive concepts. Those groups of compounds that do not have experimental procedures relating to their synthesis are either commercially available, described by procedures incorporated herein by reference, or are easily synthesized by one skilled in the art from easily recognizable, commercially available starting materials.

All patents, patent applications and publications referred to herein are incorporated by reference in their entirety.

### EXAMPLES

### I. Synthetic

Unless otherwise specified, the numbering of the R groups in the Examples section is not meant to correspond to the numbering used throughout the rest of the specification. Reference to a particular reaction by name will be apparent to one skilled in the art and/or described in: Name Reactions and Reagents in Organic Synthesis, Mundy et al. 1988, which is incorporated by reference. Additionally, particular reagents may be added, or reactions modified as will be apparent to one skilled in the art and/or as described in: March's Advanced Organic Chemistry: Reactions, Mechanisms, and Structure, 5th Edition.

### Synthesis of Example 83:

Synthesis of analogs of 5'-modified derivatives of bredinin, the 5'-phosphate 2, the 5'-deoxy derivative **3,** and the 5'-*O*-(3-aminopropyl)carbamate 4 (Fig. 1) were attempted.

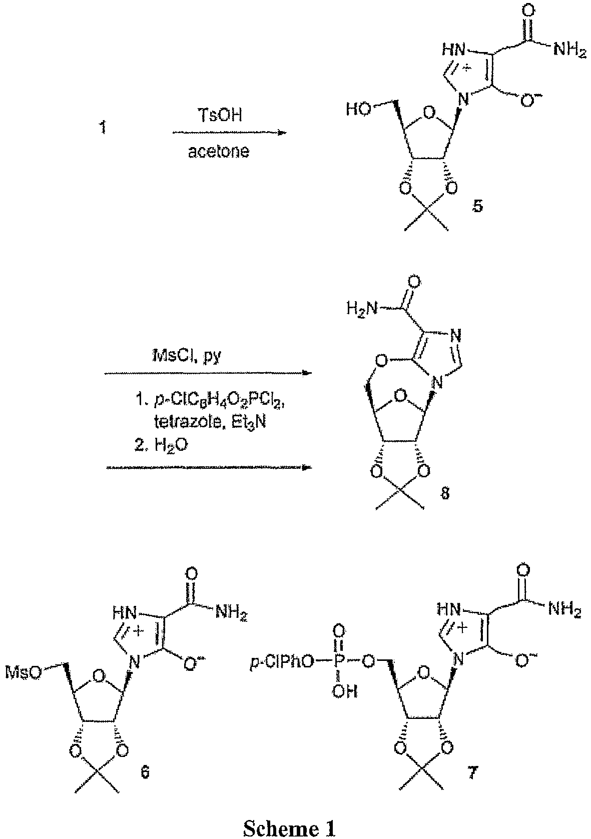

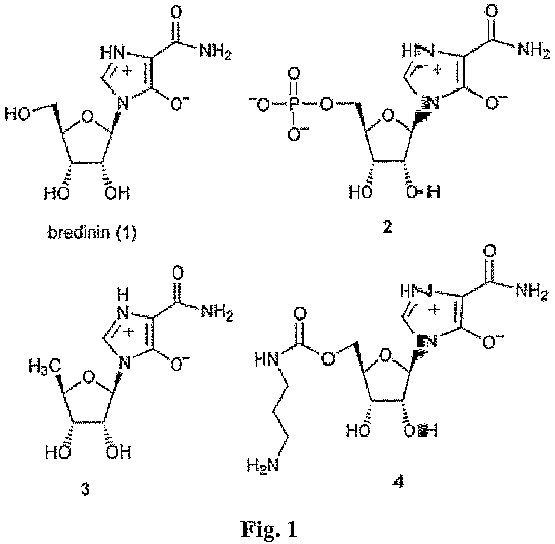

A phosphoramidite method with o-xylylene *N,N-*diethylphosphoramidite (XEPA) ¹³ is effective in this system (Scheme 2). Treatment of 17 with XEPA and tetrazole in CH₂Cl₂, followed by oxidation with aq. I₂, gives the corresponding 5'-phosphotriester 19 in 70% yield. The isopropylidene and Boc groups of 19 are removed simultaneously with 90% aq. TFA, and the resulting product, without purification, is heated with (EtO)₃CH in DMF at 90 °C to give the bredinin 5'-phosphate derivative 20 in 47% yield from 19. Hydrogenation of 20 with Pd-carbon in MeOH furnished bredinin 5'-phosphate 2, which is isolated as a disodium salt in 89% yield, after successive treatment with Dowex 50 (H⁺) and Diaion WK-20 (Na⁺) resins.

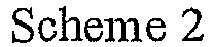

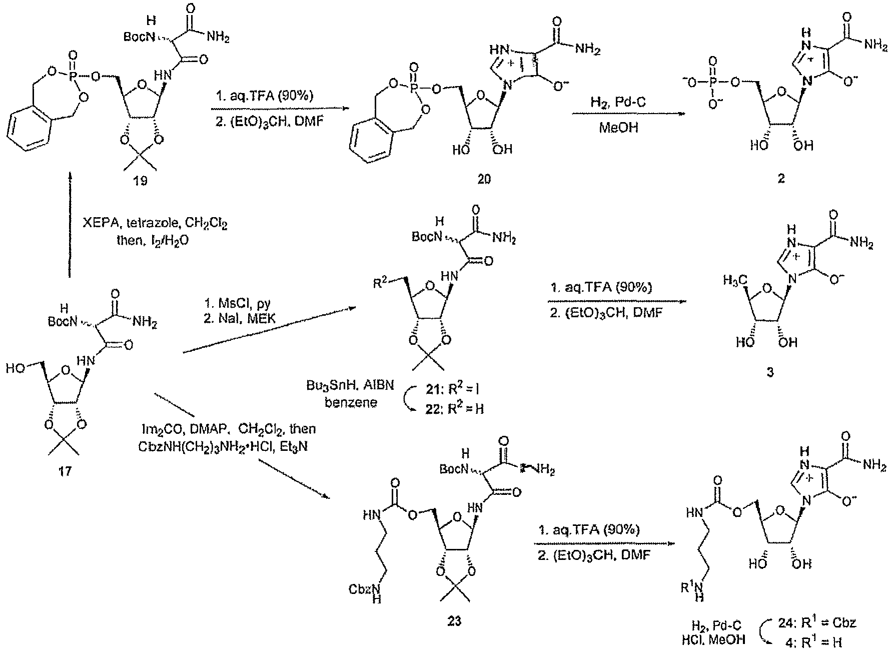

### Synthesis of Example 102:

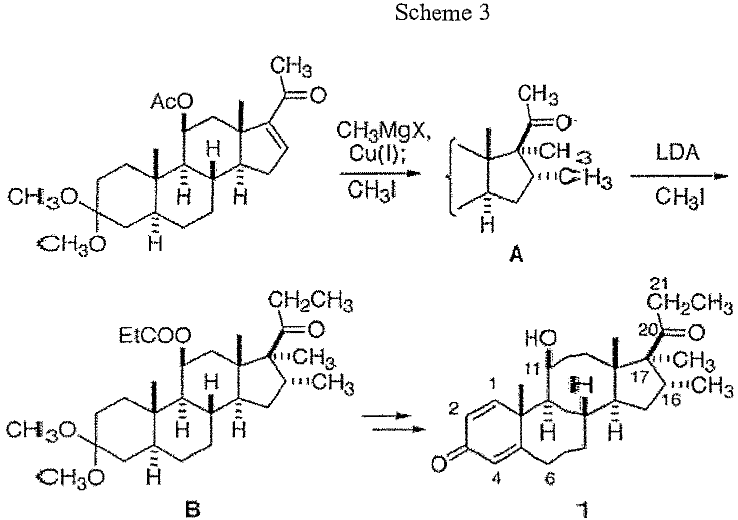

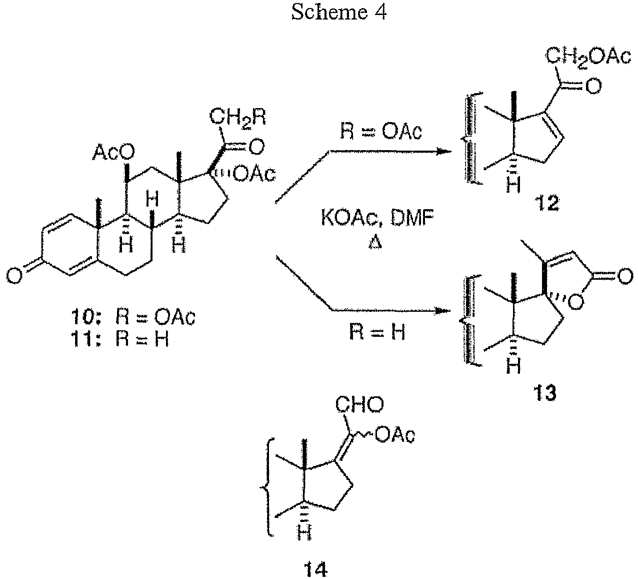

### Synthesis of Cyclosporin Examples (111-115):

Most naturally occurring cyclosporins studied, include most of *N*-desMe analogues, and can be produced by the common strain of *Tolypocladium infantum* Gams (NRRL 8044), but several other fungi could provide either limited panels of cyclosporin analogues, such as *Cylindrotrichum oligospermum* (SDZ 214-103 and all of its [Thr², D-Hiv⁸, Leu¹⁰]-CsA congeners), or unique cyclosporins such as *Acremonium luzulae* (Fuckel) W. Gams ([Thr², Leu⁵, Ala¹⁰]-CsA), *Tolypocladium terricola* ([Leu⁴]-CsA), a mutant strain of *Tolypocladium inflatum* NRRL 8044 ([Thr², Leu¹⁰]-C sA), and a *Leptostroma* anamorph of *Hypoderma eucalyptii* Cooke and Harkn ([-Thr²-, Ile⁵]-CsA). Most other analogues are obtained by precursor-directed biosynthesis, and other analogues can be obtained by chemical modification. For the synthesis of cyclosporin, the peptide bond between the L-alanine in position 7 and the D-alanine in position 8 is chosen for the cyclization step for two main reasons. The intrachain H-bonds between amide groups of the linear peptide may operate so as to stabilize the open chain in folded conformations approximating the cyclic structure of cyclosporin and thus assist cyclization. The strategy is al. so specifically chosen to preclude an N-methylamino acid at the N- and C-teminus of the undecapeptide, since bond formation between N-methylated amino acid residues presents more difficulties than for non-N-methylated derivatives. Therefore, bond formation between the only consecutive pair of non-Nmethylated amino acids in cyclosporin is the logical choice for the cyclization step.

Using the synthetic approach potentially any amino acid of the peptide chain of cyclosporin (fig. la) can be modified and specific aspects of the structure-activity relationships examined one after the other.

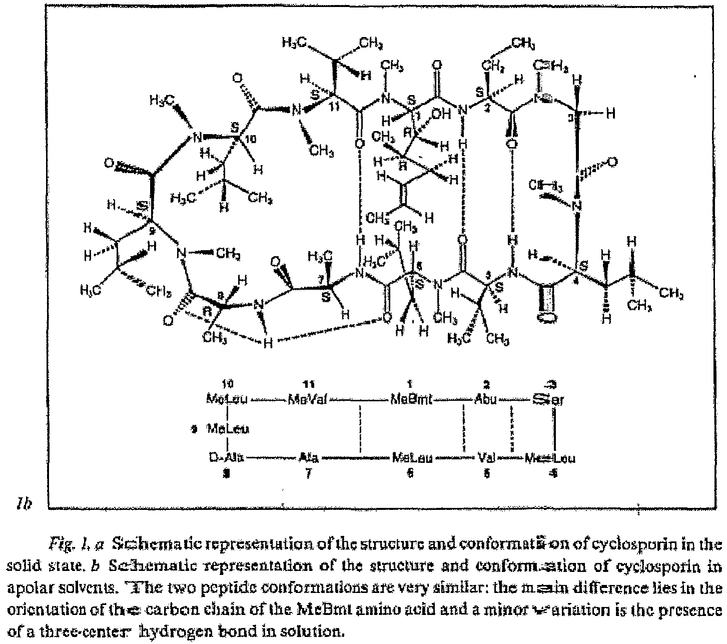

For the synthesis of the undecapeptide, a fragment-condensation technique introducing the amino acid MeBmt at the end of the synthesis is used. In this way, the number of steps after the introduction of this aminoacid is minimized. The peptide fragments are built up in the direction shown by the arrows in figure 3 using the step sequence which is indicated numerically. The amino groups of the amino acids and peptides being reacted are protected with a tert-butoxycarbonyl group (Boc) and the carboxy groups with a benzyloxy group (benzylester; OBzl). The carboxy groups are generally activated using a variation of the mixed pivalic anhydride method reported by Zaoral [14] and adapted by us for N -methyl-amino-acid derivatives [15] allowing slow anhydride formation in CHCl₃ at -20 C with piva loylchloride (= trimethylacetyl chloride) in presence of 2 equivalents of a tertiary base such as N-methylmorpholine before adding the amino acid or peptide esters to be coupled as free bases. The Boc - protecting groups of the peptide intermediates are removed with CF₃COOH at -20 C, the acids neutralized with NaHCO3 and the peptide bases isolated. The benzyloxy protecting groups of the peptide intermediates are removed with H₂ and Pd on C in ethanol. The tetrapeptide Boc-D-Ala-MeLeu-MeLeu-MeVal-OBzl was made from the left to the right by forming bonds 1, 2 and 3 (fig. 3). Bond 4 is made and the dipeptide Boc-Abu-Sar-OBzl synthesized. The tetrapeptide Boc-MeLeu-Val-MeLeu-Ala-OBzl is synthesized from the left to the right by forming bonds 5, 6 and 7 in that order. Then, by forming the bond 8, the hexapeptide Boc-Abu-Sar-MeLeu-Val-MeLeu-Ala-OBzl is prepared. To prepare the protected heptapeptide from the protected MeBmt amino acid and the hexapeptide, the dicyclohexylcarbodiimide (DCCI) coupling method is used in presence of N-hydroxybenztriazol (BtOH). The final amide linka ge 10 is made to produce the undecapeptide Boc-D-Ala-MeLeu- MeLeu- MeVal-MeBmt- Abu- Sar- MeLeu- Val- MeLeu- Ala- Obzl by coupling Boc-D-Ala-MeLeu-MeLeu-MeVal-OH with the heptapeptide by using the reagent (1H-benzo[d][1, 2, 3]triazol-1-yloxy)-tris-(dimethylamino) phosphonium hexafluorophosphate, developed by Castro et al., in presence of N-methylmorpholine in CH₂Cl₂ at room temperature. The ester group of the undecapeptide is removed by hydrolysis with NaOH at 0 C and the Boc group with CF₃COOH at -20 C, then, the unprotected H-D-Ala-MeLeu-MeLeu-MeVal-MeBmt-Abu-Sar-MeLeu-Val-MeLeu-Ala-OH is cyclized in CH₂Cl₂ (0.0002 M) using 4 equivalents of propane phosphonic acid anhydride (CH₃CH₂CH₂POVn in presence of 5 equivalents of 4-dimethylaminopyridine (1 day at room temperature) to give crystalline cyclosporin, isolated in 65% yield. Using this fragment-condensation technique, it is now possible to synthesize very effciently cyclosporin in 30% yield with re spect to the amino acid MeBmt. Thus, due to the molecular weight increase during this synthesis, 1.7 g of cyclosporin can be produced starting from 1 g of the amino acid MeBmt.

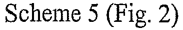

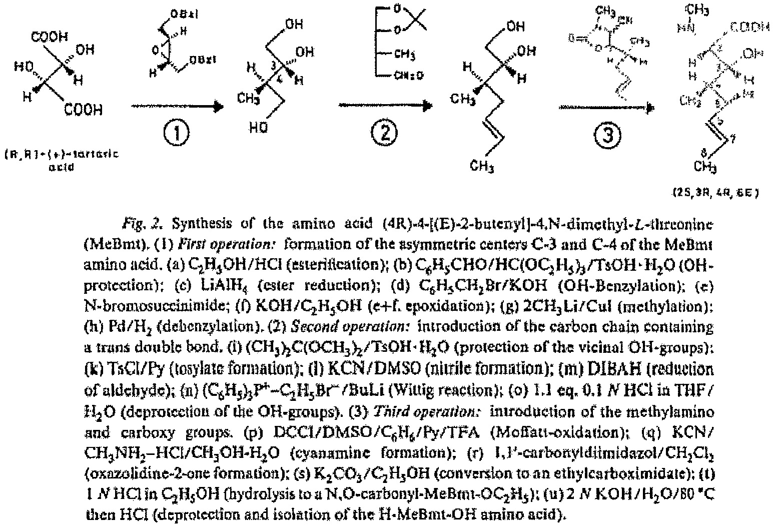

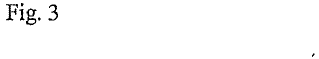

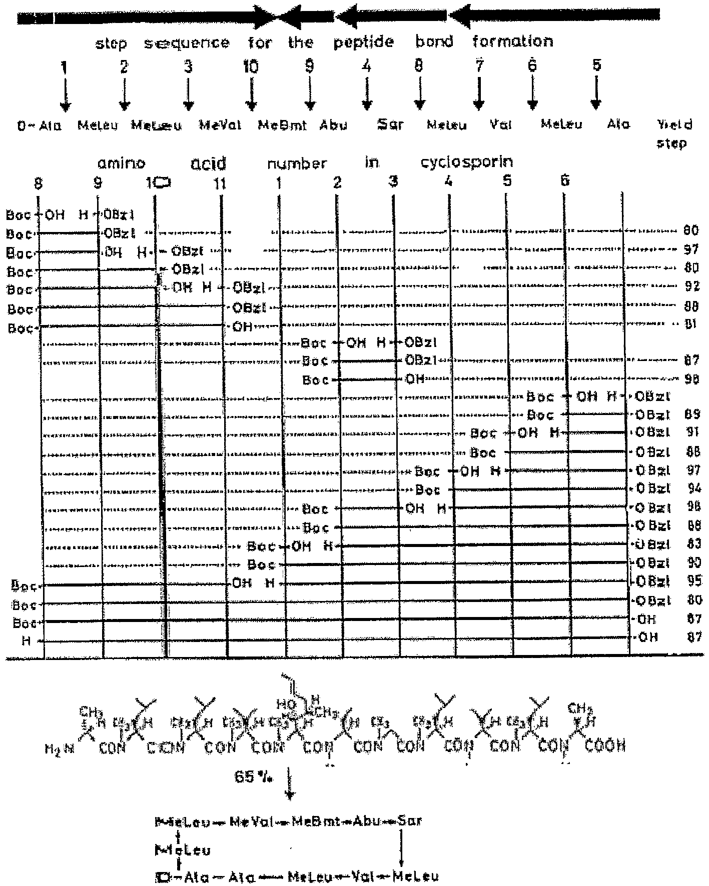

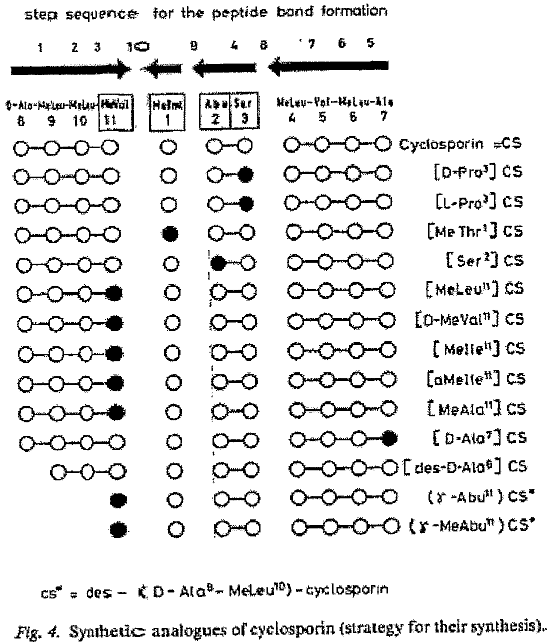

### Synthesis of Example 129:

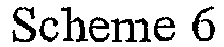

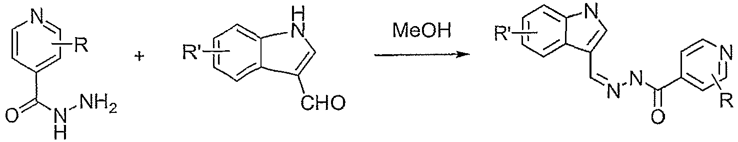

Reagents are added together in MeOH (or CH₂Cl₂ or THF) and stirred for at least 0.5 h at RT. To facilitate the reaction, the solution may be heated. Progress is monitored by TLC and HPLC. Work-up involves quenching with aqueous solution and extracting product in CH₂Cl₂.

### Synthesis of Example 130

The syntheses of the compounds (4a, 5a-d) are outlined in Scheme 7. 5-Substituted 2-mercapto-1H-benzimidazoles (2a, b) are synthesized from corresponding acetanilide derivatives (1a, b). [10] 4-Substituted 2-(chloromethyl)pyridines (3a-c) are purchased or derived from corresponding picoline-1-oxides via Pummerer-like reamangement and chlorination with thionyl chloride in chloroform. Coupling reactions of 3a-c with 2a or 2b are performed in aqueous basic conditions to obtain the sulfenyl derivatives (4a-d), and continuous selective oxidation of the benzimidazolyl sulfur atom using m-chloroperbenzoic acid are subjected to obtain sulfoxides 5a-d.

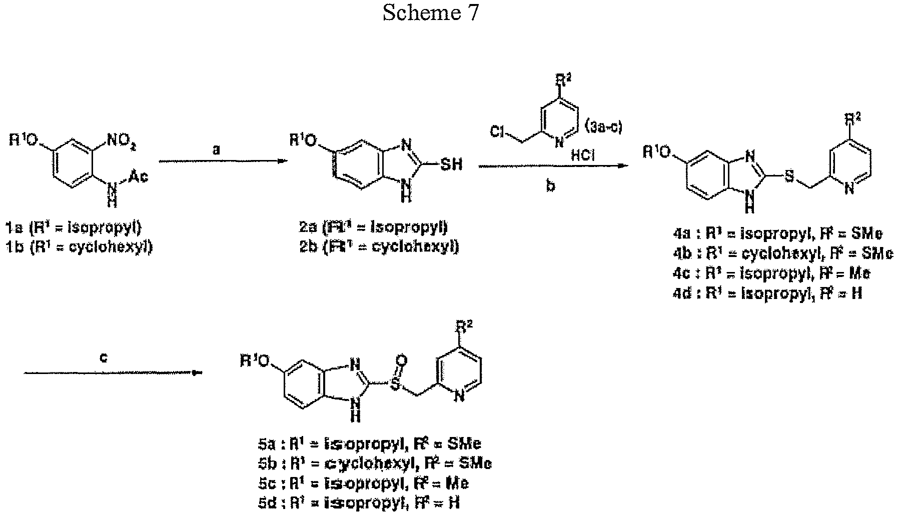

### Synthesis of Example 133

The synthesis of (alkylimino)-1,4-dihydroquinolines is illustrated in Scheme 8. The key synthetic precursors, 4-chloroquinolines 12, are prepared from commercially available anilines according to the well-established route of Price and Roberts. Conversion into the hexyl ether analog 13 is accomplished by treatment of 4-chloroquinoline with hexanol in the presence of base and phase transfer catalyst tris(2-(2-methoxyethoxy)-ethyllamine, TDA-1. The alkylamine group is installed using a modified procedure of the Surrey method, whereby the 4-chloroquinoline derivative 12 is heated with an excess of alkylamine to furnish 4-(alkylamino)quinoline 14. Alkylation on the quinoline nitrogen was performed by treatment of the 4-substituted quinolines 13 and 14 with the appropriate alkyl halide or benzylic halide to afford target molecules.

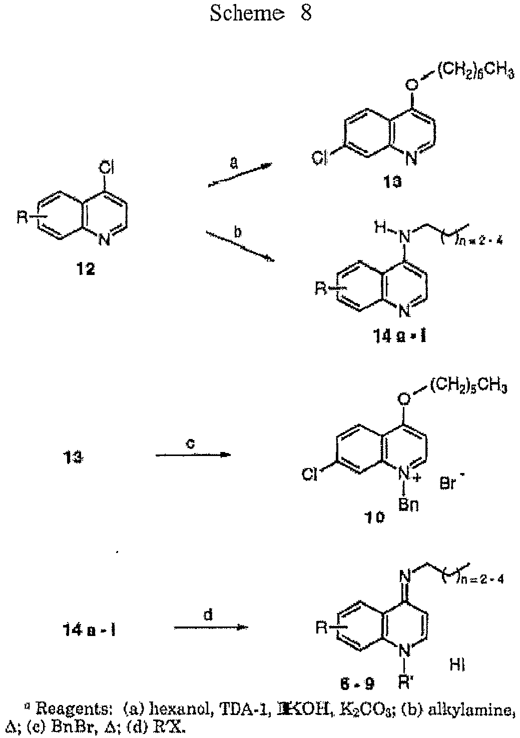

### Synthesis of Example 145:

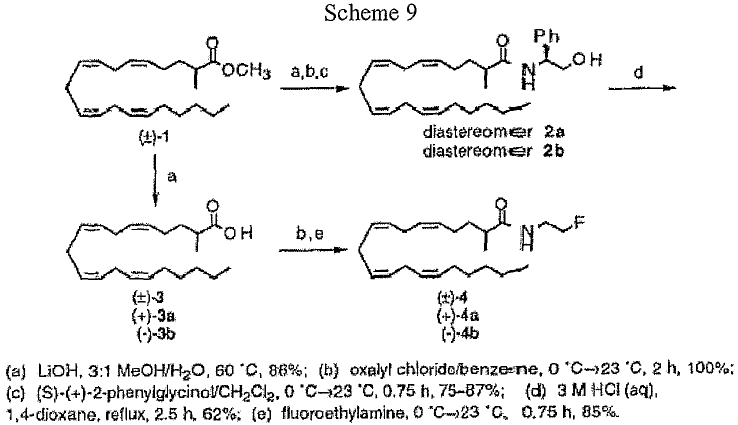

Example 145 (+)-2-methylarachidonyl-2'-fluoroethylamide (4) (Scheme 9), is synthesized from methyl arachidonate by o-alkylation with methyl iodide to give ester (+)-1, which is hydrolyzed to afford acid (+)-3. It is converted to its acid chloride and treated with excess fluoroethylamine to give the final product.

The current objective is accomplished by performing a resolution of the acid precursor and then converting it to the enantiomeric products. To resolve acid (+)-3, it is converted to its acid chloride and then treated with (S)-(+)-2-phenylglycino120 to give amide 2 as a diastereomeric mixture which is easily separated by flash chromatography to give diastereomers 2a (87%; Rf 0.37 (50% ethyl acetate/hexanes); [o]21D +54° (c 0.14, MeOH)) and 2b (75%; *RfO.* 19 (50% ethyl acetate/hexanes); led 21[> +28° (c 0.15, MeOH)). 2 1 Each amide is hydrolyzed to give the corresponding enantiomeric acids 3a (62%) and 3b (77%). To check the enantiomeric purity of acids 3a and 3b (i.e., to verify that no racemization occurred during the amide hydrolysis), they are again converted to their respective diastereomeric amides 2 and analyzed by HPLC eluting with 55% ethyl acetate/hexanes. Regenerated diastereomer 2a shows a single peak eluting at Rt 7.69 min, which indicated >98°k de. The optical purity of 3b i s determined similarly-HPLC analysis of regenerated 2b (Rt 15.82 min) indicating 98% ee (i.e., 1% of diastereomer 2a is detected).

The enantiomeric acids 3a and 3b are then converted to the target fluoroethylamides (+)-4a and (-)-4b as described above for the racemic compound. Enantiomer (+)-4a is obtained in 82% yield; [o]21D +19.9 ° (c 0.99, MeOH); MS rrdz 364 (M+I). Similarly enantiomer (-)-4b is obtained in 89% yield; [O] 21D-17.1° (c 0.98, MeOH); MS *m*/*z* 364 (M+I). Finally, assuming there is no further racemization during the conversion of acids 3a/3b to fluoroethylamides 4a/4b, (a reasonable assumption considering that the same reaction procedures are used when regenerating amides 2a/2b), assuming that amide 4a is >98% ee and that amide 4b was 98% ee, corresponding to the ee's of the regenerated amides 2a/2b, which is consistent with the optical rotations of 4a and 4b within experimental error.

### Synthesis of Examples 134-136:

The approach described in Scheme 10 is chosen primarily because it permits the use of either (-) or (+) α-pinene as the starting point. The optically active pinenes are then be converted into the verbenols **8a** and **8b** which leads to the optically active 9-ketocannabinoids **(6a** and **6b).** Likewise, the choice of the (f)-a-pinene as the starting material gives a desired racemate. Fixing the 6a,10a ring juncture trans early in the synthesis avoids the separation of isomers that can be a drawb ack. The appropriate verbenol **8a** or **8b** is converted into the AB isomers **9a** or 9b by reaction with the resorcinol (1) in a manner similar to that reported for the synthesis of A8-THC. Acetylation to **10a** or **10b** followed by photolysis gives the isomers **11a** or **11b.** During the photolysis, the 1-acetate group is removed so that it is necessary to reacetylate the isomers to give **12a** or **12b** which are subsequently ozonized to yield the keto acetates **13a** or **13b.** Hydrolysis of the 1-acetate affords the desired optically active ketones **6a** or **6b.** From (-)-a-pinene is obtained the isomer **6a;** from (+)-a-pinene, the isomer **6b.** The assigmment of absolute stereochemistry rests upon the fact that (-) -verbenol has been converted into (-)-A9-THC whose absolute stereochemistry has been shown to be 6aR,lOaR.

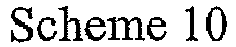

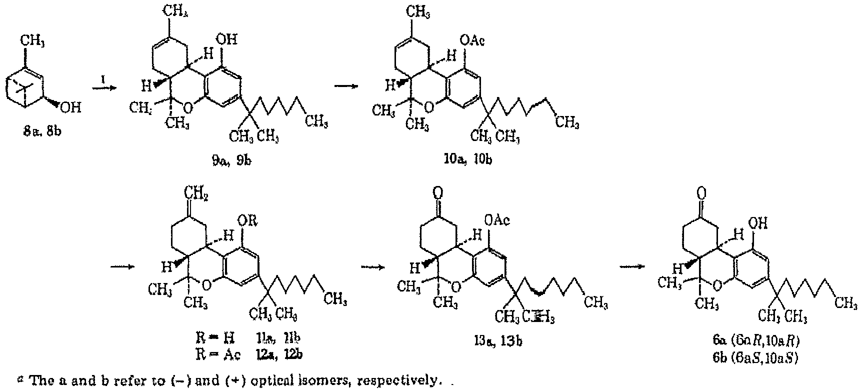

### Synthesis of Example 143:

A microorganism (e.g. IT-121 strain), belonging to the genus Myrothecium and having the ability to produce TAN-1140 is cultured in a potato/glucose agar culture medium at 20-30 deg.C and pH4-10 for 2 weeks to provide the compound of Example 143, which in turn may be further derivatized.

### Synthesis of Example 152:

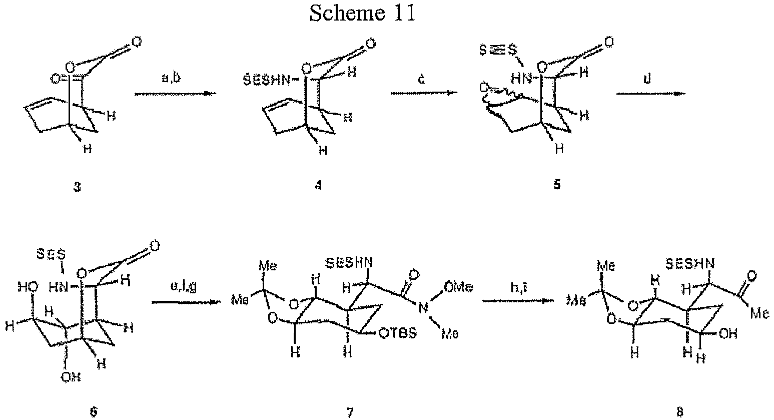

(a) Me₃SiCH₂CH₂SO₂NSO (SESNSO)/BF,Et₂O, ClCH₂CH₂Cl, 42 °C; (b) NaCNBH₃, tert-amyl alcohol; (c) mCPBA, CH₂Cl₂; (d) HCOOH; MeOH/Et₃N; (e) Me₂AlNMe(OMe), THF; (f) Me₂C(OMe)₂/p-TsOH, DMF; (9) TBSOTf/2,6-lutidine, DMF, -15 °C; (h) MeMgBr, THF; (i) n-Bu₄NF, THF, room temperature.

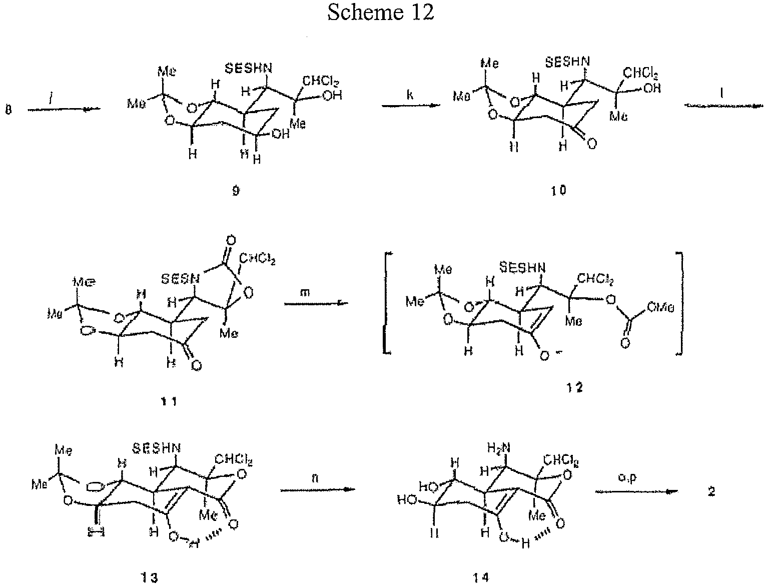

(j) Cl₂CHLi/CeCl3, Et₂O, -100 O °C; (**k**) CrO₃/pyr, CH₂Cl₂; (1) MeOCOCl, Et₃N, 4-pyrrolidinopyridine; (m) NaOMe, MeOH; (**n**) n-Bu₄NF, THF, 52 °C; MeOH/HCl; (**0**) Cbz-_{L}-alanine/DCC/Et₃N, DMF; (**p**) H₂/Pd-C, MeOH/HOAc, 0.5 N HCl.

### Analog Synthesis of Example 161:

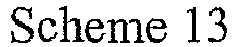

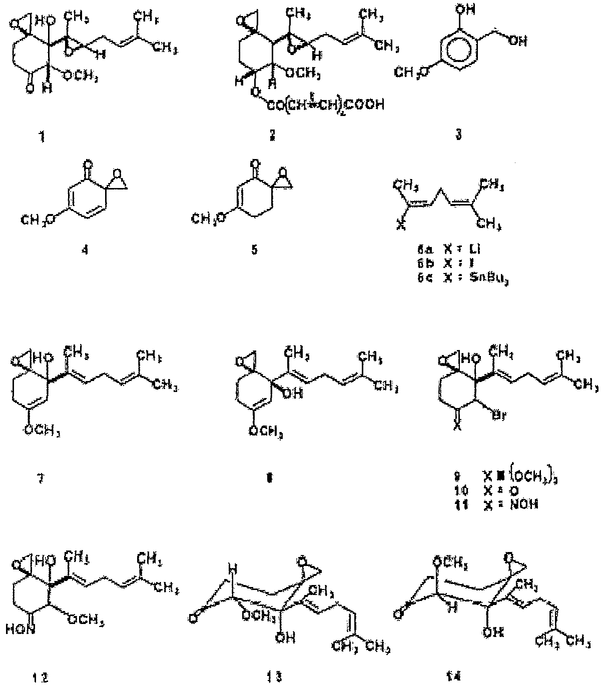

Structurally, ovalicin (1) is very similar to the antibiotic fumagillin (2), which has been synthesized and well described. We report herein a short, stereocontrolled synthesis of ovalicin. Phenol 3 is prepared from 2,4-dihydroxybenzoic acid by (1) reaction with 2.5 equiv of methyl iodide and 2 equiv of potassium carbonate in acetone at reflux for 16 h to give after extractive isolation and chromatography on silica gel (sg) methyl 2-hydroxy-4-methoxybenzoate (83%) and (2) subsequent reduction with 1.8 equiv of sodium bis(2-methoxyethoxy)aluminum di-hydride in ether at reflux for 30 min (97% yield). Reaction of 3 with aqueous sodium periodate solution4 (0.3 M) in tetra-hydrofuran (THF) at 23 "C for 1.5 h affords after extractive isolation and filtration through neutral alumina (1 : 1 hexane : ethyl acetate) dienone epoxide 4 in 61% yield. Reduction of the double bond of 4 by catalytic hydrogenation under a wide variety of conditions results in formation of the phenol 3. However, reduction using diimides generates, by addition of acetic acid solution (1 M in dimethoxyethane (DME) 12 equiv) over a 24 h period to a stirred mixture of 4 and potassium azodicarboxylate (18 equiv) at 45 °C in DME given after filtration and chromatography on neutral alumina (1 : 1 hexane-ethyl acetate), the desired epoxy enone 5 (77%).

Vinyllithium reagent 6a is prepared stereospecifically from vinyl iodide 6b which is synthesized by a method in 90% overall yield from acetone (2,4,6-triisopropylbenzyl)sulfonyl-hydrazone as follows. To a solution of this hydrazone in DME at -78 °C was added n-butyllithium (2.15 equiv). The mixture is warmed to -66 °C over 20 min and recooled to -78 °C after which 1-bromo-3-methyl-2-butene (1.2 equiv) is added. The solution is warmed to -66 °C over 20 min, stirred for 1 h at -66 °C and recooled to -78 °C. Following addition of *N,N-*N',N'-tetramethylethylenediamine (3.3 equiv) and n-butyllithium (1.1 equiv), the reaction mixture is warmed to -3 °C over 2h, recooled to -78 OC and treated with tri-n-butyltin chloride (1.25 equiv) at -78 °C for 35 min, at -35 °C for 1 h, and finally at room temperature for 45 min. Extractive isolation and chromatography on triethylamine deactivated sg (hexane eluent) gives *6c* as a single isomer with a small amount of tetra-n-butyltin side product (>90% purity by ¹H NMR analysis). Vinyltin 6c is treated with iodine (1.2 equiv) at room temperature in methylene chloride for 1 h to afford, after extractive workup and chromatography on sg (pentane eluent), 6b.

A solution of 6b in ether at -78 °C is stirred with tert-butyllithium (1.6 equiv) for 3.25 h and then epoxy enone 5 (0.8 equiv) in toluene is added to the resulting vinyllithium reagent 6a. After stirring at -78 °C for 1.25 h and extractive workup a 17:1 mixture (by ¹H NMR analysis) of diastereoisomers 7 and 8 (sg TLC Rfvalues 0.58 and 0.72, respectively, with 1: 1 hexane-ethyl acetate) is isolated. Separation of the mixture is achieved on triethylamine deactivated sg (4: 1 hexane-ethyl acetate containing 1% triethylamine cosolvent) to give pure 7 in 83% yield. Reaction of 7 with N-bromosuccinimide (1.12 equiv) in methanol at 0 "C cleanly provides a 3:1 mixture (by ¹H NMR analysis) of bromo ketal *9* and the corresponding a-bromo a'-enol ether (sg TLC Rfvalues 0.33 and 0.44, respectively, with 4:1 hexane-ethyl acetate) in quantitative yield. The mixtures is stirred at room temperature in acetone-water (1.5:1) with a catalytic amount of p-toluenesulfonic acid for 24 h to afford, after extractive workup, bromo ketone 10 (94%) which is of good purity by ¹H NMR analysis. Chromatography on sg at 4 °C (4:1 hexane-ethyl acetate) gives pure 10 (55% overall from 7). Reaction of bromo ketone 10 with hydroxylamine hydrochloride (3.56 equiv) in acetic acid buffered to pH 6 with potassium acetate (1:1 equiv) at room temperature for 1 h gives, after extractive workup, bromo oxime 11 (quantitative). Treatment of 11 in methanol with triethylamine (5 equiv) for 2 h at room temperature and 24 h at 48 °C yielded, after extractive isolation, the a-methoxy oxime 12 (quantitative recovery, >90% purity by ¹H NMR analysis). Hydrolysis of the oxime is carried out with aqueous titanium trichloride solution (4 equiv) in methanol buffered with aqueous ammonium acetate solution (pH 6; 18 equiv) for 2.5 h at room temperature to provide, after extractive isolation, a 1:1 mixture of diastereoisomers 13 and 14. Treatment of the mixture with potassium carbonate (2 equiv) in methanol at 0 °C for 2 h results in complete isomerization of 14 to 13, which is isolated in pure condition (63% from 12) by chromatography on triethylamine-deactivated sg (3:1 hexane-ethyl acetate). The conversion of 13 to ovalicin (1) is carried out as follows. A solution of 13 in benzene is treated with vanadyl acetylacetonate (0.14 equiv) followed by a solution of dry tert-butyl hydroperoxide in toluene (2 equiv) for 2 h at room temperature to give stereospecifically (by TLC and ¹H NMR analyses) (*)-ovalkin (1), which is isolated by chromatography on triethylamine-deactivated sg (3:1 hexane-ethyl acetate) in 89% yield. Synthetic (*)-1 is indistinguishable from an authentic sample by ¹H NMR, IR, mass spectra, 13C NMR analyses as well as chromatographic mobility on sg in several solvent systems. Further derivatization may occur by reaction and opening of the epoxide.

### Synthesis of Example 162:

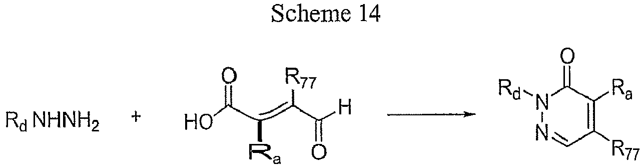

wherein R₇₇ is a group as defined in the detailed description. 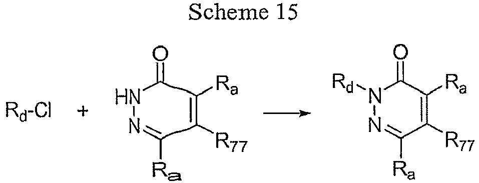 wherein R₇₇ is a group as defined in the detailed description.

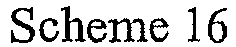

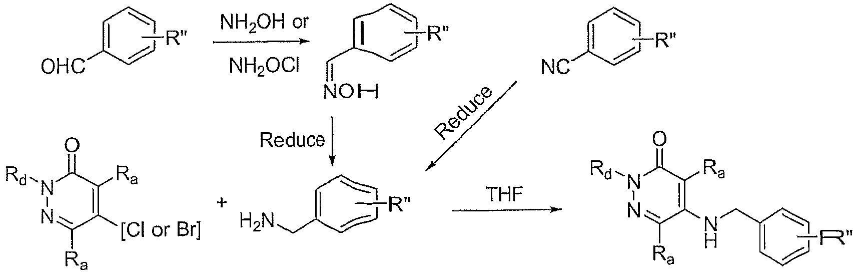

### II. Mechanistic

Method 1: Derivatization of a SMIS imidazoquinoline to a create SMIP imidazoquinoline.

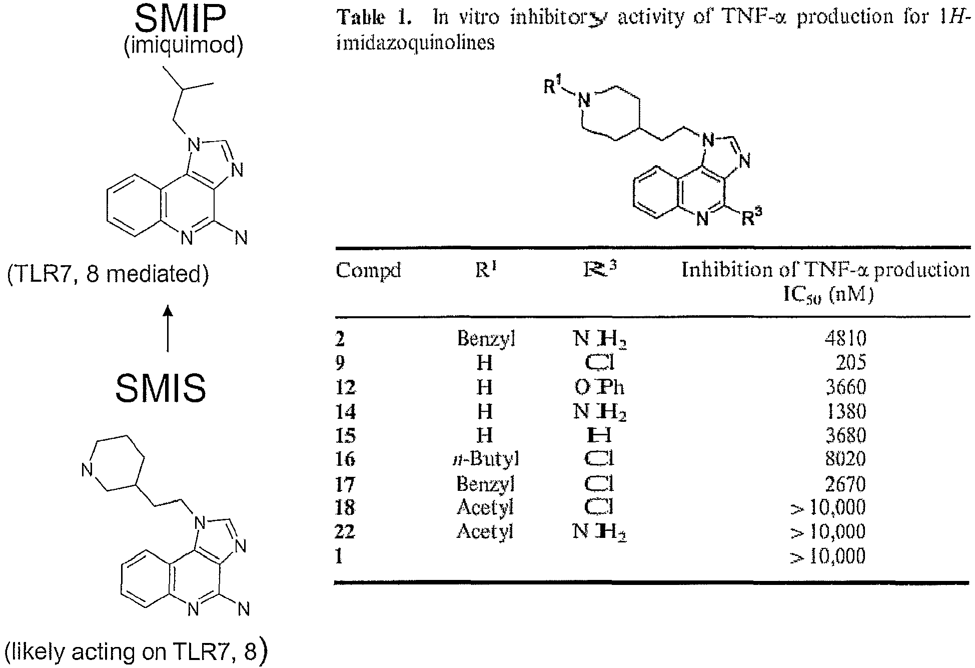

Method 2: Derivatization of a SMIS fused tetracyclopyrmidinone to a create SMIP fused tetracyclopyrmidinone.

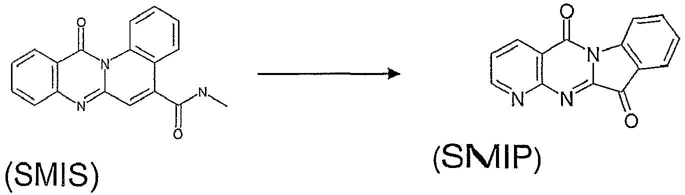

**TABLE 2: NOVEL SMIP COMPOUNDS AND THEIR SMIS ANALOGS**

| SMIP Example | SMIP Hit | SMIS Source | Immunosupressor | Mechanism |
|---|---|---|---|---|
| 16 | 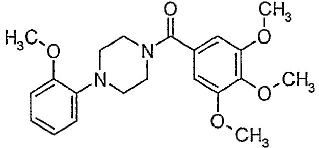 | Astrazeneca | 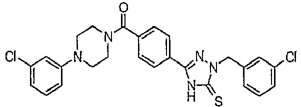 | chemokine receptor (WO 0177087) |
| 51 | 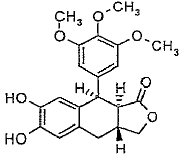 | Universidaa de Salamanca | 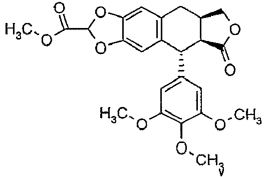 | Cell proliferation (EP711765); J. Med. Chem, 39, 2865 |
| 21 | 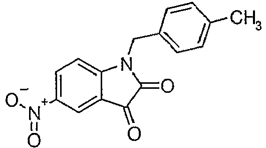 | SmithKline Beecham | 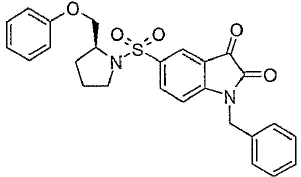 | Caspases 3 & 7 (WO 0122966) |
| 26 | 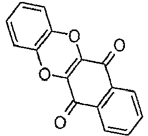 | Sterling Winthrop Pharmaceuticals Research Division | 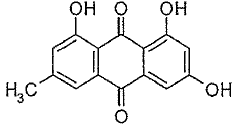 | tyrosine kinase, BBRC 287:829; Biochem 34:12404 |
| 2 | 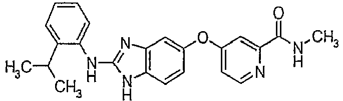 | Applied Research Systems ARS Holding N.V.; ADIR et cie | 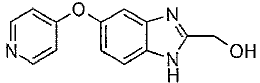 | JNK (WO 01/47920), IKK (WO2001000610, 2001030774) |
| 47 | 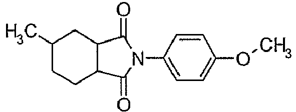 | Celegene | 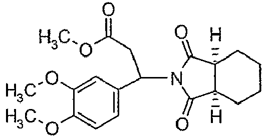 | phosphodiesterase III, IV (WO 9712859) |
| 32 | 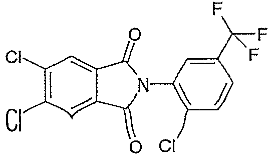 | Shire, Mary; Celegene | 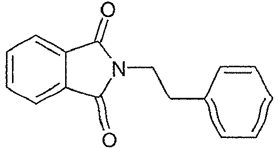 | inhibitor for TNFα production (WO 9712859,9620926) |
| 39 | 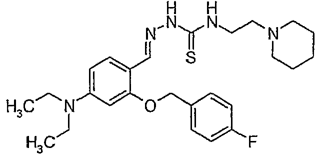 | Cheil Jedang ; Tularic | 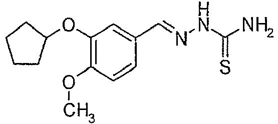 | phosphodiesterase IV(WO 0073280); IKK (WO 02/41843) |

Nomenclature for the Example compounds was provided using ACD Name version 5.07 software (November 14, 2001) available from Advanced Chemistry Development, Inc. Some of the compounds and starting materials were named using standard IUPAC nomenclature.

Examples 2-67 of Table 3 were synthesized following the synthetic methodology described above in the Examples and Schemes, and screened following the methods that directly follow the table. The precursors are readily recognizable by one skilled in the art and are commercially available from Aldrich (Milwaukee, WI) or Acros Organics (Pittsburgh, PA), among others.

**TABLE 3**

| **Example** | **Structure** | **Name** | **MH+** |
|---|---|---|---|
| 2 | 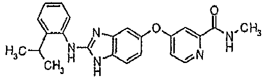 | N-methyl-4-[(2-{[2-(1-methylethyl)phenyl]amino}-1H-benzimidazol-5-yl)oxy]pyridine-2-carboxamide | 402.5 |
| 3 | 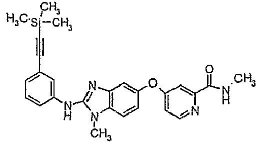 | N-methyl-4-{[1-methyl-2-({3-[(trimethylsilyl)ethynyl]phenyl}am ino)-1H-benzimidazol-5-yl]oxy}pyridine-2-carboxamide | 470.6 |
| 4 | 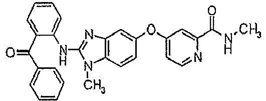 | N-methyl-4-[(1-methyl-2-{[2-(phenylcarbonyl)phenyl]amino}-1H-benzimidazol-5-yl)oxy]pyridine-2-carboxamide | 478.5 |
| 5 | 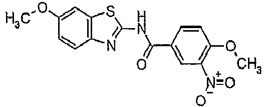 | 4-(methyloxy)-N-[6-(methyloxy)-1,3-benzothiazol-2-yl]-3-nitrobenzamide | 360.4 |
| 6 | 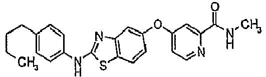 | 4-({2-[(4-butylphenyl)amino]-1,3-benzothiazol-5-yl}oxy)-N-methylpyridine-2-carboxamide | 433.5 |
| 7 | 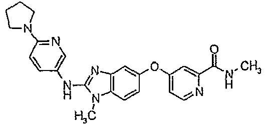 | N-methyl-4-({1-methyl-2-[(6-pyrrolidin-1-ylpyridin-3-yl)amino]-1H-benzimidazol-5-yl}oxy)pyridine-2-carboxamide | 444.5 |
| 8 | 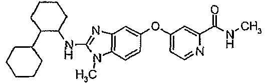 | 4-({2-[1,1'-bi(cyclohexyl)-2-ylamino]-l1-methyl-1H-benzimidazol-5-yl}oxy)-N-methylpyridine-2-carboxamide | 462.6 |
| 9 | 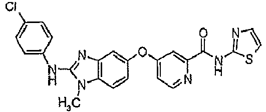 | 4-({2-[(4-chlorophenyl)amino]-1-methyl-1H-benzimidazol-5-yl}oxy)-N-1,3-thiazol-2-ylpyridine-2-carboxamide | 477.9 |
| 10 | 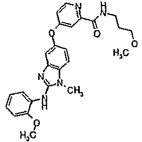 | 4-[(1-methyl-2-{[2-(methyfoxy)phenyl]amino}-1H-benzimidazol-5-yl)oxy]-N-[3-(methyloxy)propyl]pyridine-2-carboxamide | 462.5 |
| 11 | 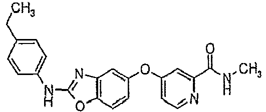 | 4-({2-[(4-ethylphenyl)amino]-1,3-benzoxazol-5-yl}oxy)-N-methylpyridine-2-carboxamide | 389.4 |
| 12 | 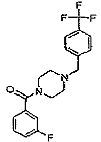 | 1-[(3-fluorophenyl)carbonyl]-4-{[4-(trifluoromethyl)phenyl]methyl}pi perazine | 367.4 |
| 13 | 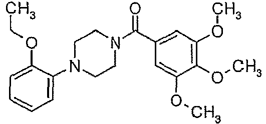 | 1-[2-(ethyloxy)phenyl]-4-{[3,4,5-tris(methyloxy)phenyl]carbonyl}p iperazine | 401.5 |
| 14 | 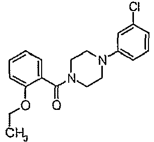 | 1-(3-chlorophenyl)-4-{[2-(ethyloxy)phenyl]carbonyl}pipera zine | 345.8 |
| 15 | 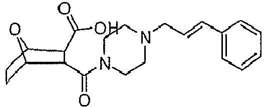 | 3-({4-[(2E)-3-phenylprop-2-enyl]piperazin-1-yl}carbonyl)-7-oxabicyclo[2.2.1]heptane-2-carboxylic acid | 371.4 |
| 16 | 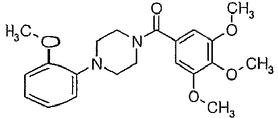 | 1-[2-(methyloxy)phenyl]-4-{[3,4,5-tris(methyloxy)phenyl]carbonyl}p iperazine | 387.4 |
| 17 | 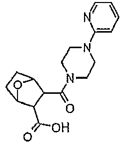 | 3-[(4-pyridin-2-ylpiperazin-1-yl)carbonyl]-7-oxabicyclo[2.2.1]heptane-2-carboxylic acid | 332.4 |
| 18 | 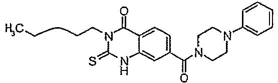 | 3-pentyl-7-[(4-phenylpiperaxin-1-yl)carbonyl]-2-thioxo-2,3-dihydroquinazolin-4(1H)-one | 437.6 |
| 19 | 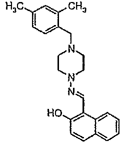 | 1-[(E)-({4-[(2,4-dimethylphenyl)methyl]piperazin -1-yl}imino)methyl]naphthalen-2-ol | 374.5 |
| 20 | 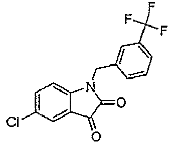 | 5-chloro-1-{[3-(trifluoromethyl)phenyl]methyl}-1H-indole-2,3-dione | 340.7 |
| 21 | 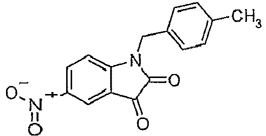 | 1-[(4-methylphenyl)methyl]-5-nitro-1H-indole-2,3-dione | 297.3 |
| 22 | 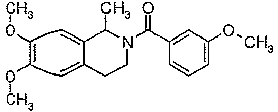 | 1-methyl-6,7-bis(methyloxy)-2-{[3-(methyloxy)phenyl]carbonyl}-1,2,3,4-tetrahydroisoquinoline | 342.4 |
| 23 | 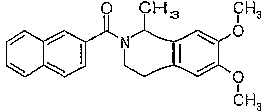 | 1-methyl-6,7-bis(methyloxy)-2-(naphthalen-2-ylcarbonyl)-1,2,3,4-tetrahydroisoquinoline | 362.4 |
| 24 | 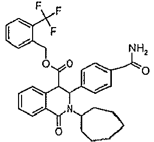 | [2-(trifluoromefhyl)phenyl]methyl 3-[4-(aminocarbonyl}phenyl]-2-cycloheptyl-1-oxo-1,2,3,4-tetrahydroisoquinoline-4-carboxylate | 565.6 |
| 25 | 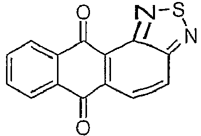 | anthra[1,2-c][1,2,5]thiadiazole-6,11-dione | 267.3 |
| 26 | 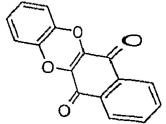 | benzo[b]oxanthrene-6,11-dione | 265.2 |
| 27 | 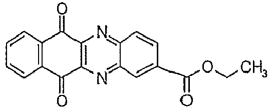 | ethyl 6,11-dioxo-6,11-dihydrobenzo[b]phenazine-2-carboxylate | 333.3 |
| 28 | 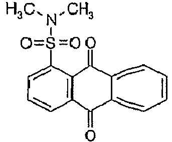 | N,N-dimethyl-9,10-dioxo-9,10-dihydroanthracene-1-sulfonamide | 316.3 |
| 29 | 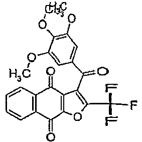 | 2-(trifluoromethyl)-3-{[3,4,5-tris(methyloxy)phenyl]carbonyl}n aphtho[2,3-b]furan-4,9-dione | 461.4 |
| 30 | 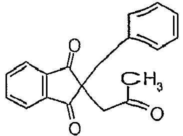 | 2-(2-oxopropyl)-2-phenyl-1H-indene-1,3(2H)-dione | 279.3 |
| 31 | 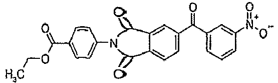 | ethyl 4-{5-[(3-nitrophenyl)carbonyl]-1,3-dioxo-1,3-dihydro-2H-isoindol-2-yl}benzoate | 445.4 |
| 32 | 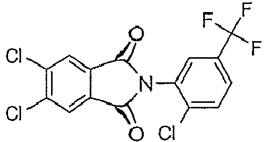 | 5,6-dichloro-2-[2-chloro-5-(trifluoromethyl)phenyl]-1H-isoindole-1,3(2H)-dione | 395.6 |
| 33 | 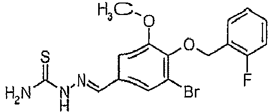 | 3-bromo-4-{[(2-fluorophenyl)methyl]oxy}-5-(methyloxy)benzaldehyde thiosemicarbazone | 413.3 |
| 34 | 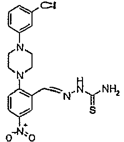 | 2-[4-(3-chlorophenyl)piperazin-1-yl]-5-nitrobenzaldehyde thiosemicarbazone | 419.9 |
| 35 | 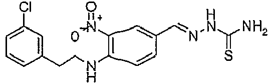 | 4-{[2-(3-chlorophenyl)ethyl]amino}-3-nitrobenzaldehyde thiosemicarbazone | 378.9 |
| 36 | 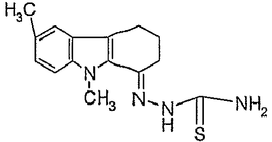 | (1 E)-6,9-dimethyl-2,3,4,9-tetrahydro-1H-carbazol-1-one thiosemicarbazone | 287.4 |
| 37 | 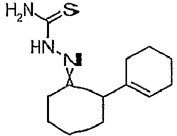 | (2E)-1,1'-bi(cyclohexan)-1-en-2-one thiosemicarbazone | 252.4 |
| 38 | 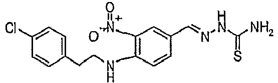 | 4-{[2-(4-chlorophenyl)ethyl]amino}-3-nitrobenzaldehyde thiosemicarbazone | 378.9 |
| 39 | 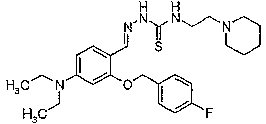 | 4-(diethylamino)-2-{[(4-fluorophenyl)methyl]oxy}benzald ehyde N-(2-piperidin-1-ylethyl)thiose micarbazone | 486.7 |
| 40 | 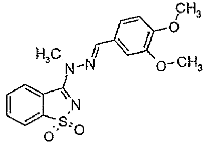 | 3,4-bis(methyloxy)benzaldehyde (1,1-dioxido-1,2-benzisothiazol-3-yl)(methyl)hydrazone | 360.4 |
| 41 | 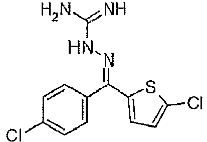 | (2E)-2-[(4-chlorophenyl)(5-chlorothien-2-yl)methylidene]hydrazinecarboxi midamide | 314.2 |
| 42 | 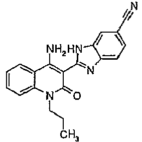 | 2-(4-amino-2-oxo-1-propyl-1,2-dihydroquinolin-3-yl)-1H-benzimidazole-6-carbonitrile | 344.4 |
| 43 | 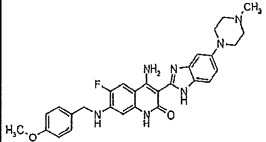 | 4-amino-6-fluoro-7-({[4-(methyloxy)phenyl]methyl}amino )-3-[5-(4-methylpiperazin-1-yl)-1H-benzimidazol-2-yl]quinolin-2(1H)-one | 528.6 |
| 44 | 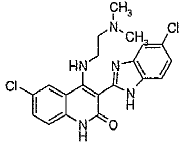 | 6-chloro-3-(5-chloro-1H-benzimidazol-2-yl)-4-{[2-(dimethylamino)ethyl]amino}quin olin-2(1H)-one | 417.3 |
| 45 | 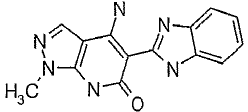 | 4-amino-5-(1H-benzimidazol-2-yl)-1-methyl-1,7-dihydro-6H-pyrazolo[3,4-b]pyridin-6-one | 281.3 |
| 46 | 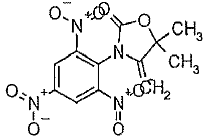 | 5,5-dimethyl-4-methylidene-3-(2,4,6-trinitrophenyl)-1,3-oxazolidin-2-one | 339.2 |
| 47 | 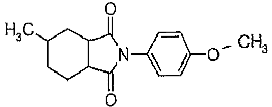 | 5-methyl-2-[4-(methyloxy)phenyl]hexahydro-1H-isoindole-1,3(2H)-dione | 274.3 |
| 48 | 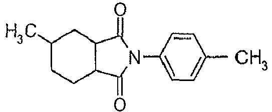 | 5-methyl-2-(4-methylphenyl)hexahydro-1H-isoindole-1,3(2H)-dione | 258.3 |
| 49 | 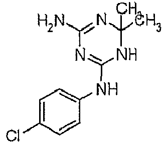 | N∼2∼-(4-chlorophenyl)-6,6-dimethyl-1,6-dihydro-1,3,5-triazine-2,4-diamine | 252.7 |
| 50 | 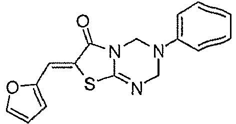 | (7Z)-7-(furan-2-ylmethylidene)-3-phenyl-3,4-dihydro-2H-[1,3]thiazolo[3,2-a][1,3,5]triazin-6(7H)-one | 312.4 |
| 51 | 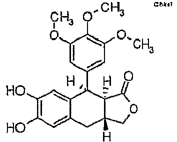 | (3aR,9R,9aR)-6,7-dihydroxy-9-[3,4,5-tris(methyloxy)phenyl]-3a,4,9,9a-tetrahydronaphtho[2,3-c]furan-1(3H)-one | 387.4 |
| 52 | 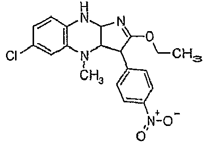 | 6-chloro-2-(ethyloxy)-4-methyl-3-(4-nitrophenyl)-3a,4,9,9a-tetrahydro-3H-pyrrolo[2,3-b]quinoxaline | 387.8 |
| 53 | 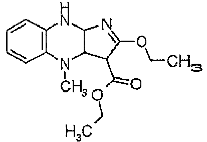 | ethyl 2-(ethyloxy)-4-methyl-3a,4,9,9a-tetrahydro-3H-pyrrolo[2,3-b]quinoxaline-3-carboxylate | 304.4 |
| 54 | 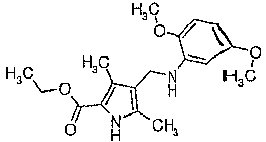 | ethyl 4-({[2,5-bis(methyloxy)phenyl]amino}met hyl)-3,5-dimethyl-1H-pyrrole-2-carboxylate | 333.4 |
| 55 | 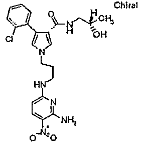 | 1-{3-[(6-amino-5-nitropyridin-2-yl)amino]propyl}-4-(2-chlorophenyl)-N-[(2S)-2-hydroxypropyl]-1H-pyrrole-3-carboxamide | 473.9 |
| 56 | 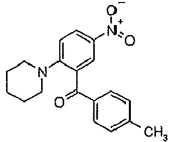 | (4-methylphenyl)(5-nitro-2-piperidin-1-ylphenyl)methanone | 325.4 |
| 57 | 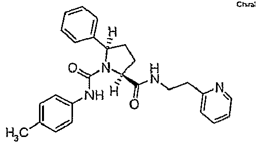 | (2S,5R)-N∼1∼-(4-methylphenyl)-5-phenyl-N∼2∼-(2-pyridin-2-ylethyl)pyrrolidine-1,2-dicarboxamide | 429.5 |
| 58 | 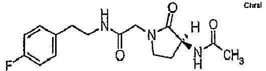 | 2-[(3S)-3-(acetylamino)-2-oxopyrrolidin-1-yl]-N-[2-(4-fluorophenyl)ethyl]acetamide | 322.4 |
| 59 | 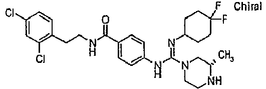 | N-[2-(2,4-dichlorophenyl)ethyl]-4-({(Z)-[(4,4-difluorocyclohexyl)imino][(3S)-3-methylpiperazin-1-yl]methyl}amino)benzamide | 553.5 |
| 60 | 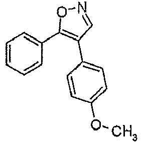 | 4-[4-(methyloxy)phenyl]-5-phenylisoxazole | 252.3 |
| 61 | 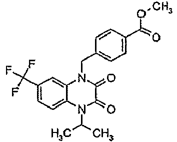 | methyl 4-{[4-(1-methylethyl)-2,3-dioxo-7-(trifluoromethyl)-3,4-dihydroquinoxalin-1 (2H)-yl]methyl}benzoate | 421.4 |
| 62 | 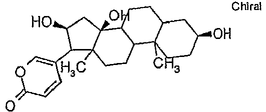 | (3beta,16beta)-3,14,16-trihydroxybufa-20,22-dienolide | 403.5 |
| 63 | 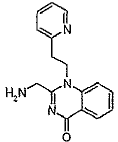 | 2-(aminomethyl)-1-(2-pyridin-2-ylethyl)quinazolin-4(1H)-one | 281.3 |
| 64 | 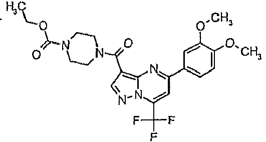 | ethyl 4-{[5-[3,4-bis(methyloxy)phenyl]-7-(trifluoromethyl)pyra zolo[1,5-a]pyrimidin-3-yl]carbonyl}piperazine-1-carboxylate | 508.5 |
| 65 | 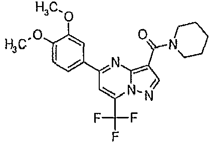 | 5-[3,4-bis(methyloxy)phenyl]-3-(piperidin-1-ylcarbonyl)-7-(trifluoromethyl)pyrazolo[1,5-a]pyrimidine | 435.4 |
| 66 | 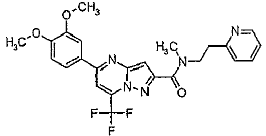 | 5-[3,4-bis(methyloxy)phenyl]-N-methyl-N-(2-pyridin-2-ylethyl)-7-(trifluoromethyl)pyrazolo[1,5-a]pyrimidine-2-carboxamide | 486.5 |
| 67 | 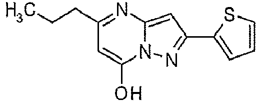 | 5-propyl-2-thien-2-ylpyrazolo[1,5-a]pyrimidin-7-ol | 260.3 |

**TABLE 4**

| Example | Structure | Drug | Company | Action | Indication | Reference |
|---|---|---|---|---|---|---|
| 68 | 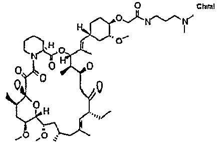 | A-85917 | Abbott Laboratories | Immunosuppressant | Immune disorder; Autoimmune disease | WO 9113889 |
| 69 | 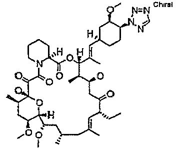 | ascrolimus | Abbott Laboratories | Immunosuppressant | Atopic dermatitis; Psoriasis; Dermatitis | WO 9113889 |
| 70 | 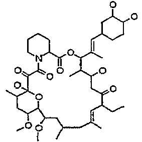 | L-683742 | Merck & Co Inc | Immunosuppressant | Transplant rejection | WO 9113889 |
| 71 | 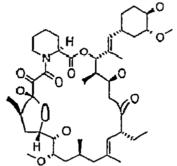 | L-685487 | Merck & Co Inc | Immunosuppressant | Immune disorder | US4975372 |
| 72 | 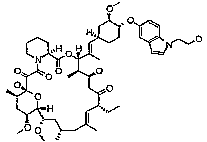 | L-732531 | Merck & Co Inc | Immunosuppressant | Autoimmune disease; Transplant rejection | US-05310903; EP532088-19930317;JP94116274-19940426;US5252732-19931012; WO9305058-19930318 |
| 73 | 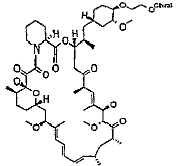 | everolimus | Novartis AG | Anticancer; IL-2 synthesis inhibitor; Immunosuppressant | Inflammatory bowel disease; Rheumatoid arthritis; Transplant rejection; Solid tumor | WO-09409010-19940428; WO9735575-19971002; EP663916-19950726; JP96502266-19960312; US5665772-19970909; WO9424304-19941027 WO9507468-19950316; WO9804279-19980205 |
| 74 | 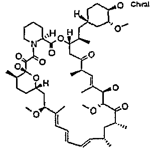 | sirolimus | Wyeth Pharmaceuticals | Protein synthesis inhibitor; Immunosuppressant | Transplant rejection | WO9214476-19920903; WO9214477-1920903; US5321009-19940614; US5776943-19980707; US5286730-19940215; WO9319763-19931014; US5387589-19950207; US5496832-19960305; WO9747317-19971218; WO9818468-19980507 |
| 75 | 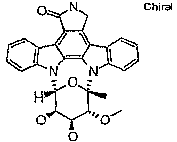 | MLR-52 | Abbott Laboratories | Anti-inflammatory; Protein kinase C inhibitor; Immunosuppressant | Inflammation | WO 0230941; WO 9707081 |
| 76 | 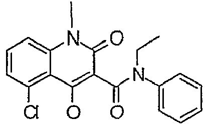 | laquinimod | Active Biotech AB | Immunomodulator; Immunosuppressant | Multiple sclerosis | EP 1073639-20010207; JP 2002513006-20020508; US 6077851-20000620; WO 9955678-19991104; EP 1224172-20020724; JP 2003512454-20030402; US 6395750-20020528; WO 0130758-20010503 |
| 77 | 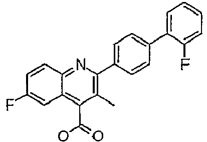 | brequinar | Bristol-Myers Squibb Pharma Co | Anticancer; Immunosuppressant; Dehydrogenase inhibitor | Immune disorder; Psoriasis; Transplant rejection; Carcinoma: Cancer | US5393891: US5707844 |
| 78 | 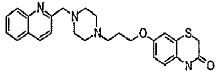 | VUF-K-8788 | Gifu Pharmaceutical University | Histamine antagonist; Immunosuppressant | Asthma | EP957100-19991117; WO9902520-199901 |
| 79 | 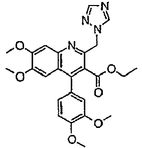 | TAK-603 | Takeda Chemical Industries Ltd | Immunosuppressant; Bone resorption inhibitor; Cytokine synthesis inhibitor | Inflammation; Arthritis; Psoriasis; Rheumatoid arthritis; Bone disease; Graft versus host disease; Crohns disease | EP608870-940803; JP95118266-950509; US5436247-950725; EP634169-950118; JP95069890-950314 |
| 80 | 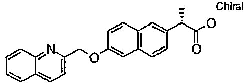 | WY-50295-tromethamine | Wyeth-Ayerst Pharmaceuticals. Inc | LT antagonist; 5-Lipoxygenase inhibitor; Anti-inflammatory; Immunosuppressant | Inflammation; Arthritis; Psoriasis; Asthma | US5173488; US6177477; AU8819209-19890202; EP301813-19890201; GB2207428-19890201 |
| 81 | 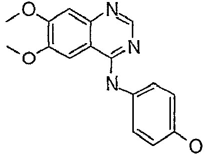 | apoptosis stimulators, Hughes Institute | Hughes Institute | Anticancer; Apoptosis stimulator; Jak3 tyrosine kinase inhibitor; Immunosuppressant | Allergy; Acute lymphoblastic leukemia; Leukemia; Motor neurone disease; Transplant rejection; Cancer; Insulin dependent diabetes | WO 2004013091; WO 2003101380; WO 2003065995 WO 2003065971 WO 2003149045; U.S. 5,480,883 |
| 82 | 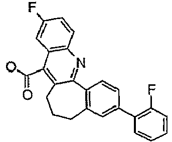 | KF-20444 | Kyowa Hakko Kogyo Co Ltd | Anticancer; Immunosuppressant; Dehydrogenase inhibitor | Rheumatoid arthritis; Autoimmune disease; Transplant rejection; Cancer | US5371225-19941206; WO9322286-19931111 |
| 83 | 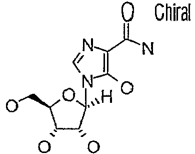 | mizoribine | Asahi Kasei Pharma Corp | Anticancer; Immunosuppressant; IMP dehydrogenase inhibitor | Renal disease; Viral infection; Transplant rejection; Cancer | See above |
| 84 | 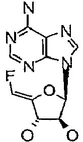 | MDL-28842 | Hoechst Marion Roussel Inc | Anticancer; Anti-inflammatory; Immunosuppressant; Adenosylhomocysteinase inhibitor | Inflammation; Cancer | EP304889-19890301; US4997925-19910305; AU9163842-19910418; EP422638-19910417; JP91133992-19910607; US4997924-19910305 |
| 85a | 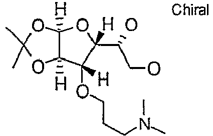 | amiprilose | Greenwich Pharmaceuticals Inc | Immunosuppressant | Rheumatoid arthritis | US 4,017,608 |
| 85b | 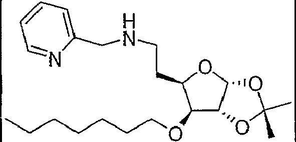 | GW-92527 | Boston Life Sciences | Immunosuppressant | Arthritis, rheumatoid | |
| 86 | 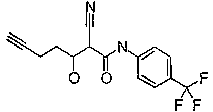 | FK-778 | Aventis Pharma AG | Anticancer, antimetabolite; Immunosuppressant; DNA modulator; Antiviral | Multiple sclerosis; Autoimmune disease; Transplant rejection; Dermatological disease | EP 551230-930714 US 5308865 |
| 87 | 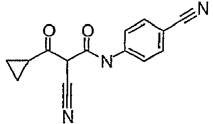 | HMR-1279 | Aventis Pharma AG | Immunosuppressant; DNA modulator | Multiple sclerosis; Autoimmune disease; Transplant rejection; Dermatological disease | EP 484223-920506; JP 92288048-921013 US 5240960 |
| 88 | 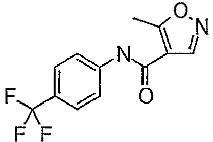 | leflunomide | Aventis Pharma AG | Anti-inflammatory; Immunosuppressant | Multiple sclerosis; Rheumatoid arthritis; Transplant rejection | US 4351841-19820928; US 4284786-19810818; US 4965276-19901023; US 5268382-19931207; US 5459163-19951017; US 5504084-19960402; US 5494911-19960227; US 5532259-19960702; WO 9117748-19911128; US 5700823-19971223; WO 9519169-19950720; US 5700822-19971223; US 5610173-19970311 |
| 89 | 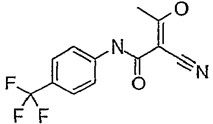 | teriflunomide | Aventis Pharma AG | Dihydroorotate dehydrogenase inhibitor; Protein tyrosine kinase inhibitor; Immunosuppressant | Multiple sclerosis | WO 9117748-911128 |
| 90 | 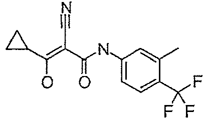 | HR-325 | Hoechst Marion Roussel Inc | Anticancer; Immunosuppressant | Rheumatoid arthritis; Cancer | EP484223-920506; JP92288048-921013 |
| 91 | 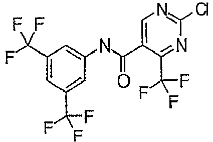 | NF-kB/AP-1 inhibitor, Tanabe | Signal Research Division | Nuclear factor kappa B inhibitor; Anti-inflammatory; Protein synthesis inhibitor; Immunosuppressant; Jun N terminal kinase-1a modulator | Inflammation; Osteoarthritis; Psoriasis; Rheumatoid arthritis; Transplant rejection | WO9709315-97031 |
| 92 | 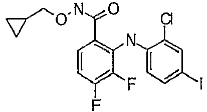 | MAP/ERK kinase (MEK)-1 inhibitors, Pfizer | Pfizer Inc | Anticancer; Immunosuppressant; MEK-1 protein kinase inhibitor | Non-small-cell lung cancer; Pancreas tumor; Breast tumor; Transplant rejection; Cancer; Colon tumor | WO 9901426,19990114, 9837881, 19980903; US 19970701,19970228 |
| 93 | 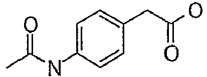 | actarit | Nippon Shinyaku Co Ltd | Immunosuppressant | Immune disorder; Rheumatoid arthritis | DE3317107-831124; US4720506-880119 |
| 94 | 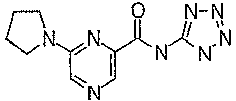 | HSR-6071 | Hokuriku Seiyaku KK | Immunosuppressant | Allergy; Asthma | EP227026-19870701; JP87153284-19870708; JP88112578-19880517; US4792547-19881220 |
| 95 | 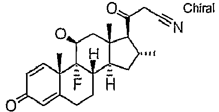 | RU-24858 , | Aventis Pharma AG | Steroidal anti-inflammatory; Glucocorticoid agonist; Receptor modulator; Immunosuppressant | Immune disorder; Inflammation; Asthma | WO 9739018-19971023 |
| 96 | 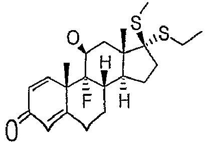 | tipredane | Bristol-Myers Squibb Co | LT antagonist; Anti-inflammatory; Immunosuppressant; Arachidonic acid antagonist | Allergy; Inflammation; Ocular disease; Asthma | US-04361559 |
| 97 | 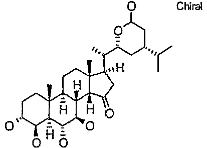 | IPL-576092 | Inflazyme Pharmaceuticals. Ltd | Anti-inflammatory; Immunosuppressant | Inflammation; Ocular disease; Asthma; Dermatitis | WO-09414451 |
| 98 | 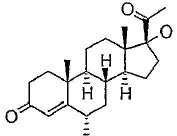 | CBP-1011 | InKine Pharmaceutical Co Inc | Anti-inflammatory; Corticosteroid agonist; Immunosuppressant | Thrombocytopenic purpura; Inflammatory bowel disease; Asthma; Systemic lupus erythematosus; Ulcerative colitis; Autoimmune disease; Crohns disease | US 3280155 WO 9832718 |
| 99 | 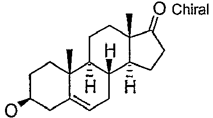 | prasterone, Genelabs/Watso n | Leland Stanford Junior University | Nootropic agent; Immunosuppressant; Androgen agonist; NO modulator | Systemic lupus erythematosus | WO-09408589-940428 |
| 100 | 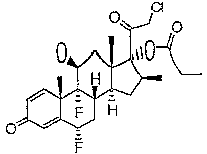 | halobetasol propionate | Novartis AG | Corticosteroid agonist; Immunosuppressant | Psoriasis | WO 2003047329 WO 9832718 |
| 101 | 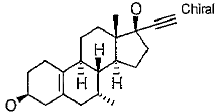 | Org-4094 | NV Organon | Immunosuppressant | Autoimmune disease | US 5,512,556 |
| 102 | 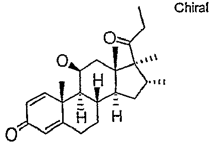 | rimexolone | NV Organon | Steroidal anti-inflammatory; PAF antagonist; Corticosteroid agonist; Immunosuppressant | Rheumatoid arthritis; Uveitis; Cataract; Ocular inflammation | See above for syntheses |
| 103 | 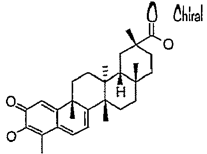 | celastrol (cancer), Schering-Plough | Schering-Plough Research Institute | Topoisomerase I inhibitor; Anticancer; Protein tyrosine kinase inhibitor; Immunosuppressant | Cancer | US5580562 |
| 104 | 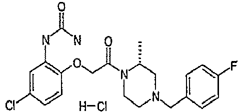 | BX-471 | Berlex Biosciences | Anti-inflammatory; CCR1 chemokine antagonist; Immunosuppressant; Imaging agent | Atopic dermatitis; Alzheimers disease; Multiple sclerosis; Psoriasis; Rheumatoid arthritis; Autoimmune disease; Transplant rejection | WO-09856771-19981217; US6207665-20010327 |
| 105 | 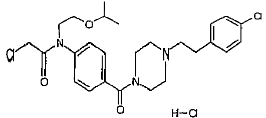 | CGP-47969A | Novartis AG | IL-1 release inhibitor; IL synthesis inhibitor; Anti-inflammatory; Immunosuppressant | Rheumatoid arthritis; Autoimmune disease | US5888969; US5888978; US6664256; US6680316; EP524146-930120; JP93202014-930810; US5286728-940215 |
| 106 | 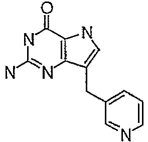 | peldesine | BioCryst Pharmaceuticals Inc | Anticancer; Purine nucleoside phosphorylase inhibitor; Immunosuppressant; Antiviral | HIV infection; Multiple sclerosis; Ocular disease; Psoriasis; Rheumatoid arthritis; Non-Hodgkin lymphoma; Transplant rejection; Dermatitis | US-04985433-910115; US5008270-910416; WO9106548-910516 |
| 107 | 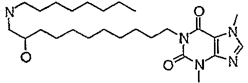 | CT-2576 | Cell Therapeutics Inc | Immunosuppressant | Acquired Immune Deficiency Syndrome; Transplant rejection; Cancer | US5859222; US6274363; US5470878-951128; WO9422863-941013 |
| 108 | 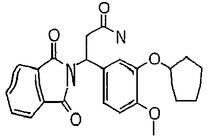 | CC-1088 | Celgene Corp | Cytokine release inhibitor; Anti-Inflammatory; PDE 4 inhibitor; TNF alpha synthesis inhibitor; Immunosuppressant | Myelodysplastic syndrome; Inflammation; Crohns disease | WO-09723457-970703 |
| 109 | 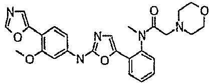 | IMPDH inhibitors (immunosuppres sants), BMS | Bristol-Myers Squibb Pharmaceutical Research Institute | Immunosuppressant; IMP dehydrogenase inhibitor | Arthritis | WO03099206A2; WO03099206A3; US20030232866A1; US20040077878A1; WO04032875A2; EP1126843-20010829; US6399773-20020604; WO0025780-2000051 |
| 110 | 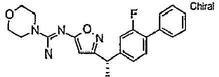 | SMP-114 | Sumitomo Pharmaceuticals, Co Ltd | Anti-inflammatory; Immunosuppressant | Rheumatoid arthritis | EP0979226-20000216; JP1999240873.19990907, US6100260-20000808; WO9847880-19981029 |
| 111 | 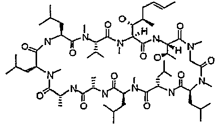 | FR-901459 | Fujisawa Pharmaceutical Co Ltd | Anti-inflammatory; Immunosuppressant | Inflammation | WO 2002032447 |
| 112 | 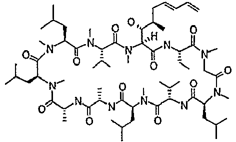 | ISAtx-247 | Isotechnika Inc | Immunosuppressant; Calcineurin inhibitor | Psoriasis; Rheumatoid arthritis; Autoimmune disease; Transplant rejection; Insulin dependent diabetes | WO-09918120 |
| 113 | 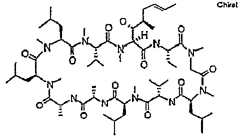 | ciclosporin (topical; HILT), SKyePharma | Hyal Pharmaceutical Corp | Immunosuppressant; Calcineurin modulator; Cytokine synthesis inhibitor | Psoriasis | WO9813024A2 |
| 114 | 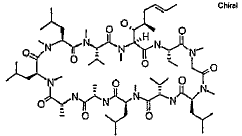 | DE-076 | Santen Pharmaceutical Co Ltd | Anti-inflammatory; Immunosuppressant; Calcineurin inhibitor | Keratoconjunctivitis | J Pharm Pharmacol. 49:835 |
| 115 | 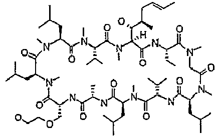 | oxeclosporin | Novartis AG | Immunosuppressant | Psoriasis; Asthma; Transplant rejection | AU9059727-19910124; EP414632-19910227; JP93218396-19910925 |
| 116 | 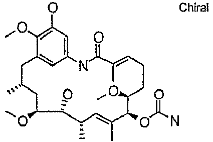 | reblastatin | Glaxo Wellcome plc | Immunosuppressant | Rheumatoid arthritis | JP97286779-19971104 WO 2003013430 WO 9314215 |
| 117 | 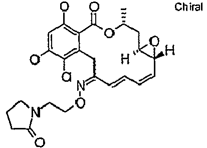 | radicicol derivatives, Kyowa | Kyowa Hakko Kogyo Co Ltd | Anticancer; Apoptosis stimulator; Immunosuppressant | Allergy; Autoimmune disease; Transplant rejection; Cancer | EP0889042-19990107; WO9818780-19980507 |
| 118 | 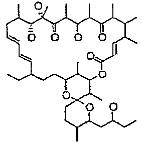 | oligomycin-F, Boehringer Mannheim | Roche Holding AG | Immunosuppressant | Transplant rejection | US6395711 |
| 119 | 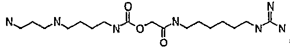 | tresperimus | Fournier Pharma | Immunosuppressant | Autoimmune disease; Transplant rejection | EP-00600762-940608; FR2698628-940603; US 5476870 |
| 120 | 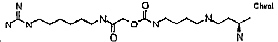 | anisperimus | Fournier Pharma | Immunosuppressant | Autoimmune disease | EP669316-950830; FR2716451-950825; FR2716452-9508251 JP96041007-960213; US 5637613 |
| 121 | 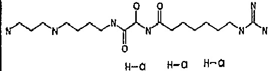 | gusperimus ; trihydrochloride | Nippon Kayaku Co Ltd | Immunosuppressant | Granulomatosis; Multiple sclerosis; Rheumatoid arthritis; Transplant rejection | BE-00894651; US 4518532 |
| 122 | 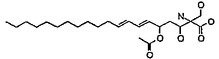 | WF-10917 | Fujisawa Pharmaceutical Co Ltd | Immunosuppressant | Psoriasis; Rheumatoid arthritis; Autoimmune disease; Transplant rejection; Graft versus host disease | WO9709298-970313 |
| 123 | 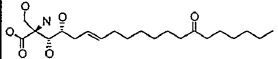 | ISP-1/myriocin analogs, Yoshitomi | Mitsubishi Pharma Corp | Immunosuppressant | Autoimmune disease; Graft versus host disease | EP-00436020; JP89104087-19890421; W09002727-19900322 |
| 124 | 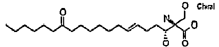 | Mycestericin E | Taito Co Ltd | Immunosuppressant | Immune disorder | US6720391 |
| 125 | 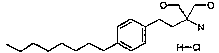 | FTY-720 | Welfide Corp | Anticancer; Apoptosis stimulator; Immunosuppressant | Inflammatory bowel disease; Multiple sclerosis; Myocarditis; Autoimmune disease; Transplant rejection; Cancer; Insulin dependent diabetes | US6121329; EP627406-941207;JP94509845-941102; W09408943-940428 |
| 128 | 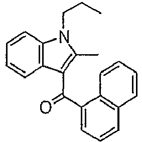 | JWH-015 | Clemson University | Cannabinoid CB2 agonist; Immunosuppressant | Transplant rejection | WO 2003049727; WO 2001028588 |
| 129 | 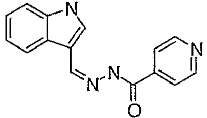 | TJU-103 | Jefferson Medical College | Immunosuppressant | Graft versus host disease | See above for synthesis |
| 130 | 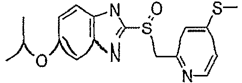 | TU-572 | Taisho Pharmaceutical Co Ltd | Protein tyrosine phosphatase inhibitor; Immunosuppressant | Immune disorder | Int Arch Allergy Immunol 126:318; See above for synthesis |
| 131 | 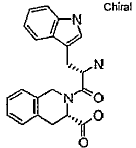 | dipeptidyl peptidase IV inhibitors, Tanabe | Tanabe Seiyaku Co Ltd | Immunosuppressant; Dipeptidyl peptidase IV inhibitor | Rheumatoid arthritis; Autoimmune disease; Diabetes mellitus | WO9818763-19980507 |
| 132 | 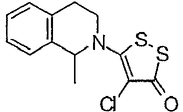 | RP-54745 | Rhone-Poulenc SA | Immunosuppressant | Rheumatoid arthritis | US4576954-19860318 |
| 133 | 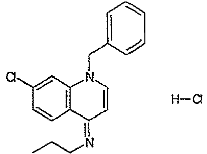 | Win-17317-3 | Sanofi Winthrop Inc | Potassium channel blocker; Immunosuppressant | Autoimmune disease; Transplant rejection | |
| 134 | 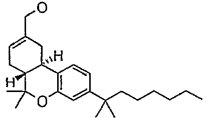 | JWH-051 | Clemson University | Immunosuppressant; Cannabinoid agonist | Transplant rejection | Eur J Pharmacol 339:53 |
| 135 | 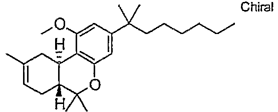 | L-759633 | Merck & Co Inc | Immunosuppressant; Cannabinoid agonist | Autoimmune disease | Br J Pharmacol 126:665 |
| 136 | 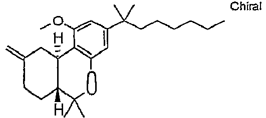 | L-759656 | Merck & Co Inc | Immunosuppressant; Cannabinoid agonist | Autoimmune disease | Br J Pharmacol 126:665 |
| 137 | 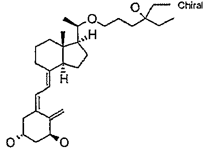 | lexacalcitol | LEO Pharma A/S | Anticancer; Immunosuppressant; Vitamin D3 agonist | Sarcoidosis; Psoriasis; Breast tumor; Skin tumor; Insulin dependent diabetes | WO9414453-19940707 |
| 138 | 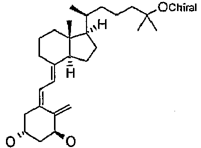 | MC-1288 | LEO Pharma A/S | Immunosuppressant; Vitamin D3 agonist | Sarcoidosis; Multiple sclerosis; Transplant rejection; Cancer | US6071897 |
| 139 | 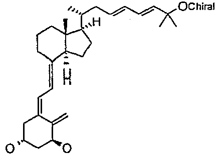 | Ro-63-2023 | F Hoffmann-La Roche Ltd | Immunosuppressant | Multiple sclerosis | WO9100855-19910124 |
| 140 | 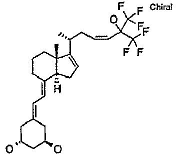 | Ro-26-2198 | Roche SpA | Cytokine release inhibitor; Immunosuppressant | Insulin dependent diabetes | US5428029-19950627 |
| 141 | 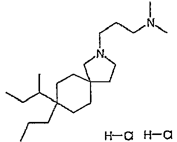 | SKF-105685 | SmithKline Beecham plc | Immunosuppressant | HIV-1 infection; Rheumatoid arthritis; Autoimmune disease; Transplant rejection | WO 92/02229 |
| 142 | 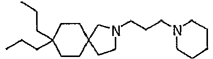 | SKF-106610 | SmithKline Beecham plc | Immunosuppressant | Rheumatoid arthritis; Autoimmune disease; Insulin dependent diabetes | WO9202229-920920 |
| 143 | 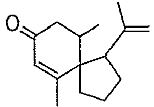 | TAN-1140 | Takeda Chemical Industries Ltd | Immunosuppressant | Immune disorder | JP90193940-19900731; See above procedure |
| 144 | 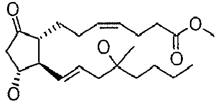 | enisoprost | GD Searle & Co | Prostanoid receptor agonist; Immunosuppressant | Peptic ulcer; Transplant rejection | US6255518; US5807895; US5218139 |
| 145 | 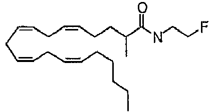 | O-689 | Organix Inc | Immunosuppressant; Cannabinoid agonist | Transplant rejection | Eur J Pharmacol. 355:113 See above synthesis |
| 146 | 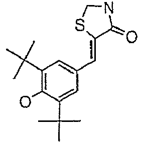 | LY-178002 | Eli Lilly & Co | Anti-inflammatory; Immunosuppressant; Antioxidant agent | Inflammation; Neurodegenerative disease | EP-00211670-19870225; JP87042977-19870224; AU8660972-19870212; US 5356917 |
| 147 | 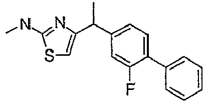 | SM-8849 | Sumitomo Pharmaceuticals Co Ltd | Immunosuppressant; Bone resorption inhibitor | Rheumatoid arthritis | EP248399-19871209; JP88152368-19880624; US4914112 |
| 148 | 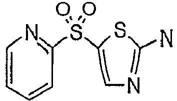 | FR-115092 | Fujisawa Pharmaceutical Co Ltd | Immunosuppressant | Thrombocytopenic purpura; Systemic lupus erythematosus; Thrombocytopenia; Autoimmune disease | EP-00412404-19910213; JP91068567-19910325; JP96048628-19960220; US 5256675 |
| 149 | 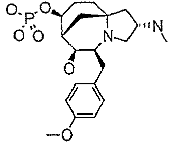 | FR-901483 | Fujisawa Pharmaceutical Co Ltd | Immunosuppressant | Transplant rejection; Graft versus host disease | WO9312125-930624 |
| 150 | 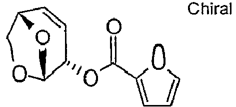 | MT-2221 | Ishihara Sangyo KK | Immunosuppressant | Rheumatoid arthritis | CA2087805-930807; EP560055-930915; JP94183965-940705; US 5380834 |
| 151 | 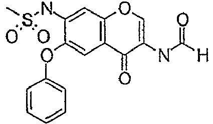 | iguratimod | Toyama Chemical Co Ltd | Immunosuppressant | Rheumatoid arthritis | FR2621585-19890414; GB2210879-19890621; JP90268178-19901101; US 4954518 |
| 152 | 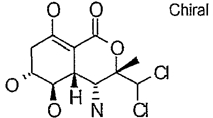 | bactobolamine | Meiji Seika Kaisha Ltd | Immunosuppressant | Autoimmune disease | Transplant Proc 28:1049 See above synthesis |
| 153 | 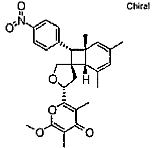 | SNF-4435 | Snow Brand Milk Products Co Ltd | Antibacterial; Immunomodulator; Immunosuppressant | Allergy; Autoimmune disease; Infection | WO9743434-19971120 |
| 154 | 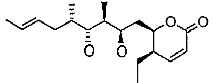 | NK-10958P | Nippon Kayaku Co Ltd | Antibacterial; Immunosuppressant | Transplant rejection | EP 560389, 930915; JP 93310726, 931122; US 5314911 |
| 155 | 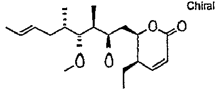 | PA-48153C | Shionogi & Co Ltd | Fungicide; Immunosuppressant | Fungal infection; Immune disorder; Arthritis; Autoimmune disease | EP560389-930915; JP93310726-931122; US 5314911 |
| 156 | 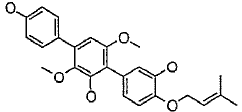 | terprenin derivatives, Shionogi | Shionogi & Co Ltd | Immunosuppressant | Allergy | WO9739999-19971030 |
| 157 | 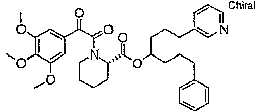 | neurophilins (neurological disease), Vertex/Schering | Vertex Pharmaceuticals, Inc | Antiparkinsonian; Immunosuppressant | Alzheimers disease; Multiple sclerosis; Parkinsons disease; Arthritis; Psoriasis; Autoimmune disease; Neurological disease; Diabetes mellitus | WO9929858A1 |
| 158 | 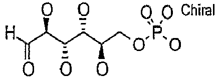 | carbohydrate-based therapeutics, Pharmaxis/Austr alian Nat Univ | The Australian National University | Anti-inflammatory; Immunosuppressant | Chronic obstructive pulmonary disease; Inflammation; Multiple sclerosis; Arthritis; Rheumatoid arthritis; Asthma; Cystic fibrosis; Diabetes mellitus | WO-09001938 |
| 159 | 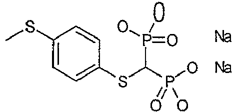 | TRK-530 | Toray Industries Inc | Non-steroidal anti-inflammatory; Immunosuppressant; Bone resorption inhibitor | Rheumatoid arthritis | US6670343; EP594857-19940504; WO9305052-19930318; WO9419359-19940901 |
| 160 | 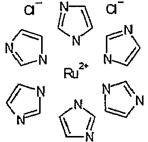 | ruthenium complexes, Procept | Paligent Inc | Immunosuppressant | Multiple sclerosis; Systemic lupus erythematosus; Autoimmune disease; Graft versus host disease | WO9613510A1; US5512687-960430; US5708022; US5512687-960430 |
| 161 | 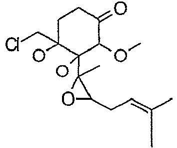 | PF-1131 | Meiji Seika Kaisha Ltd | Immunosuppressant | Asthma | JP95017957-950120 |
| 162 | 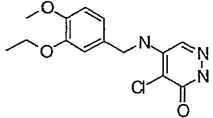 | NIP-520 | Nissan Chemical Industries Ltd | Immunosuppressant | Asthma | Jpn J Pharmacol 68:47 |
| 163 | 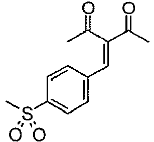 | OR-1384 | Orion Corp | Immunosuppressant | Inflammatory bowel disease; Asthma | US6201027; US5185370-930209 |
| 164 | 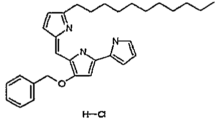 | PNU-156804 | Pharmacia & Upjohn Inc | Immunosuppressant | Multiple sclerosis; Rheumatoid arthritis; Autoimmune disease | WO9517381-950629 |
| 165 | 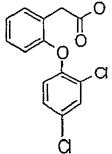 | fenclofenac | Reckitt & Colman plc | anti-inflammatory; Immunosuppressant | Inflammation; Arthritis | US4468469; WO9827972A2; WO9827972A3; US4845083; US4912136 |
| 166 | 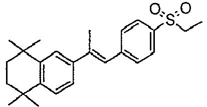 | etarotene | Roche Holding AG | Immunosuppressant | Neoplasm; Psoriasis; Dermatitis | EP058370-19820825; US 4396553 |
| 167 | 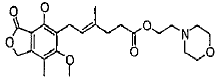 | mycophenolate mofetil | Roche Holding AG | Vasoprotectant; Anti-inflammatory; Immunosuppressant; IMP dehydrogenase inhibitor | Inflammatory bowel disease; Respiratory syncytial virus infection; Rheumatoid arthritis; Systemic lupus erythematosus; Thrombocytopenia; Restenosis; Autoimmune disease; Transplant rejection; Crohns disease | US4753935-19880628; US4786637-19881122; WO9412184-19940609; WO9426266-19941124; WO9507902-19950323; WO9509626-19950413 |
| 168 | 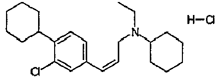 | SR-31747 | Sanofi-Synthelabo | Anticancer; IL-1 antagonist; Sigma opioid agonist; Immunosuppressant; IFN beta antagonist; IL-6 antagonist | Immune disorder; Prostate tumor; Psoriasis; Rheumatoid arthritis; Autoimmune disease; Breast tumor; Cancer | EP376850-19900704; FR2641276-19900706; US 5354781 |
| 169 | 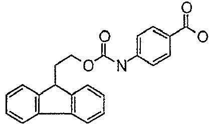 | NPC-16570 | Scios Inc | Immunosuppressant | Allergy; Allergic encephalitis | WO 96/15105 US 5519043 WO 93/11764 |
| 170 | 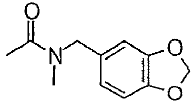 | SY-640 | Taisho Pharmaceutical Co Ltd | Immunosuppressant; Protectant | Hepatitis; Liver disease | US 6723743 |
| 171 | 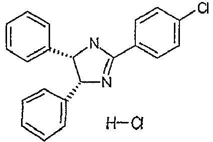 | TA-383 | Tanabe Seiyaku Co Ltd | IL-1 antagonist; Immunosuppressant; IFN beta antagonist; IL-6 antagonist | Rheumatoid arthritis | U96051441 |
| 172 | 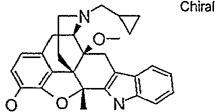 | HS-378 | University of Innsbruck | Opioid receptor modulator; Anti-inflammatory; Immunosuppressant | Arthritis; Transplant rejection | WO9531463 |
| 173 | 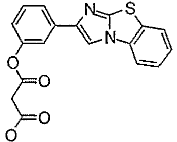 | YM-13650 | Yamanouchi Pharmaceutical Co Ltd | Immunosuppressant | Allergy; Transplant rejection | EP082712-19830629; US 4464384 |
| 174 | 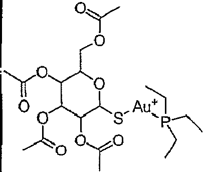 | Ridaura Ridauran SK&F-39162 | GlaxoSmithKline | Immunosuppressant Prostaglandin synthase inhibitor | Arthritis, rheumatoid | 3708579 |

Preferred analogs of compounds of Table 4 include those encompassed by formulas I, II, III, IV, V, VI, VII, VIII, IX, XI, XII, XIII, XIV, XV, XVI, XVII, XVIII, XIX, XX, XXI, XXII, XXIII, XXIV, XXV, XXVI, XXVII, XXVIII, XXIX, XXX, XXXI, XXXII, XXXIII, XXXIV, XXXV, XXXVI, XXXVII, XXXVIII, XXXIX, XL, KLI, XLII, XLIII, XLIV, XLV, XLVI, XLVII, XLVIII, XLIX, L, and LI.

### BIOLOGICAL EXAMPLES

### Example 174

Candidate small molecule immuno-potentiators can be identified *in vitro.* Compounds are screened *in vitro* for their ability to activate immune cells. One marker of such activation is the induction of cytokine production, for example TNF-α production. Apoptosis inducing small molecules may be identified having this activity. These small molecule immuno-potentiators have potential utility as adjuvants and immuno-therapeutics.

In an assay procedure (High Throughput Screening (HTS)) for small molecule immune potentiators (SMIPs), human peripheral blood mononuclear cells (PBMC), 500,000 per mL in RPMI 1640 medium with 10% FCS, were distributed in 96 well plates ( 100,000 per well) already containing 5µM of compound in DMSO. The PBMCs were incubated for 18 h at 37°C in 5% CO₂. Their ability to produce cytokines in response to the small molecule compounds is determined using a modified sandwich ELISA. Alternatively, Luminex Technology may be used.

Brie fly supernatants from the PBMC cultures were assayed for secreted TNF using a primary plate bound antibody for capture followed by a secondary biotinylated anti-TNF antibody forming a sandwich. The biotinylated second antibody was then detected using streptavidin-Europium and the amount of bound europium was determined by time resolved fluorescence. SMIP compounds were confirmed by their TNF inducing activity that was measured in the assay as increased Europium counts over cells incubated in RPMI medium alone. "Hits" were selected based on their TNF-inducing activity relative to an optimal dose of lipopolysaccaride LPS (1 µg/ml), a strong TNF inducer. The robustness of the assay and low backgrounds allowed for the routine selection of hits with ~10% of LPS activity that was normally between 5-10X background (cells alone). Selected hits are then subjected to confirmation for their ability to induce cytokines from multiple donors at decreasing concentrations. Those compounds with consistent activity at or below 5µM are considered confirmed for the purposes of this assay. The assay is readily modified for screening for compounds effective at higher or lower concentrations.

### Example 175

Each of Examples 2-67 elicited TNF-α production in human peripheral blood mononuclear cells. Many of the compounds showed activity at less than 20 µM with respect to pro duction of TNF-α. Many of these compounds showed activity at less than 5 µM with respect to production of TNF-α. Many of these compounds showed activity in the production of TNF-α at less than 1.5 µM.

For this reason, each of the R groups of any of the compounds listed in Table 3 are preferred. Additionally, because of the excellent activity of each of the compounds, and the mechanistic correlations, each of these compounds of Tables 3 and 4 are individually preferred and is preferred as a member of a group that includes any or all of the other compounds and each compound is preferred in methods of modulating immunopotentiation and in methods of treating biological conditions associated therewith, for example to be used as a vaccine adjuvant. Each of the compounds is also preferred for use in preparation of medicaments for vaccines, immunopotentiation, reducing tumor growth and in treating biological conditions mediated therefrom.

In addition to the procedure described above, methods of measuring other cytokines (e.g. IL1 -beta, IL-12, IL-6, IFN-gamma, IL-10 etc.) are well known in the art and can be used to find active SMIP compounds of the present invention.

Compounds may be useful that cause production of TNF-α at higher concentrations, such as 100µM, 200 µM or 300µM in the assays described herein. For example Loxoribine causes useful production of TNF-α at 300µM (see Pope et al Immunostimulatory Compound 7-Allyl-8-Oxoguanosine (Loxoribine) Induces a Distinct Subset of Murine Cytokines Cellular Immunology 162: 333-339 (1995)).

### Example 176

A combinatorial library is constructed (e.g., using a conventional mix/split synthesis on suitable resin bead supports) which comprises a number of sublibrary mixtures, each generally containing about 2 to 500, and preferably about 20 to 100 compounds each. It is preferable that the bead supports be high-loading beads (which provide >1 nmole of compound per bead). It is also preferable that the bead supports have a substantially uniform diameter. The use of a substantially uniform population of bead supports in the methods of the invention provide the added benefit that final reaction volumes of compounds cleaved from the bead supports will have substantially uniform compound concentrations. Thus, the bead supports preferably have a diameter variance of about 40%, preferably about 30%, more preferably about 20%, and most preferably about 5-10% or less. The actual number of individual compounds in each sublibrary is not important or limiting in the present invention, and the method can be practiced with any size sublibrary selected according to user preferences. Prior to cleavage of the compounds from the resin bead supports, each sublibrary is split into archive and screening samples, wherein the screening sample is generally comprised of roughly 10 to 30 percent of the entire sublibrary volume.

A small aliquot of the archive sample can be used in a statistical post-synthesis analysis, wherein the method and device of the present invention are used to deposit single beads in a suitable reaction vessel (preferably a multi-well plate or a fixed array of reaction vials) so that each bead can be chemically analyzed or screened separately. This statistical analysis can be used to determine the amount of, and/or identify different compounds present in the archive sample. The remainder of the archive sample is retained in bound form (uncleaved), but is treated to remove solvents, suitably dried, and then stored either as an intact archive sample, or in a plurality of replica samples which c an contain individual beads, small collections of beads, or the entire sublibrary pool of beads. Storing the archive sample in a dried, uncleaved form allows for indefinite archiving of the library with a reduced incidence of compound loss and/or decomposition.

The screening sample is distributed into reaction vessels (e.g., a multi-well plate or an array of reaction vials) to establish screening aliquots. The screening aliquots are then treated in a suitable cleavage step to remove and separate the bead supports from the cleaved compounds, and the cleaved compounds are screened in a typical primary screen for desired activity. For example, the cleaved compounds can be subjected to evaporation to remove solvents, lyophilized, labeled (if desired), and subjected to dissolution. Sublibraries which contain active components are then subjected to the following nonsynthetic deconvolution methodology.

The dried archive sample, which corresponds to a sublibrary identified as having activity in the above-described primary screen of the analysis sample, is then retrieved. The sample is reconstituted in a suitable solvent, preferably a solvent with a density of at least about 1.1 g/ml, and a suitable bead-sorting apparatus is used to distribute one bead per well in a multi-well reaction plate or reaction vessel array in multiple redundancy such that there is a greater than 95% probability that every compound in the sublibrary is represented (e.g., at a 5X redundancy). If desired, the bead-sorting apparatus is used to distribute any number of beads per well, such as where combinations of compounds are to be assessed for activity in the screening method.
After the desired number of beads have been distributed, the nonsynthetic deconvolution method of the invention is then carried out. As discussed above, each sublibrary generally contains about 20-100 compounds each, thus about 100-500 discrete beads can be distributed from the archive sample to provide a screening array with adequate compound representation. The compounds are cleaved from the bead supports using a suitable cleavage reagent, and the compounds reconstituted in a suitable reaction solvent (e.g., DMSO). Portions of the cleaved compounds are delivered into a further array which replicates the screening array. This replica array is then contacted with the cells, such as HPMCs, and immunologically active compounds are identified using the high throughput assay of the instant invention. A sampling of the reserved portion of the screening array (e.g., about 10%) is then removed for conventional chemical analytics (e.g., liquid chromatography such as HPLC, mass spectrometry (MS) and/or nitrogen (N₂) analyses) in order to provide for direct chemical identification and characterization of active compounds As can be seen, the above nonsynthetic deconvolution obviates the iterative deconvolution by resynthesis normally needed to identify single compounds responsible for biological and/or chemical activity in a mixture of compounds that were synthesized by a mix-and-split method. If desired, the individual compounds can be suitably labeled with a chemical tag (e.g., mass tags, enzymatic labels, or the like) to facilitate sample identification, however such labeling only provides marginal advantage in the present nonsynthetic deconvolution method, since MS data can easily be used as a "tag" to identify active sublibrary component.

**Table 5. Chemokine Species**

| Chemokine nomenclature^{a}(tab001cbb) | | |
|---|---|---|
| Systematic name | Original ligand name | Receptors |
| CXC chemokines | | |
| CXCL1 | GROα | CXCR2, CXCR |
| GXCL2 | GROβ | CXCR2 |
| CXCL3 | GROγ | CXCR2 |
| CXCL4 | PF4 | Unknown |
| CXCL5 | ENA-78 | CXCR2 |
| GXCL6 | GCP-2 | CXCR1, CXCR2 |
| CXCL7 | NAP-2 | CXCR2 |
| CXCL8 | IL-8 | CXCR1, CXCR2 |
| CXCL9 | Mlg | CXCR3 |
| CXCL10 | IP-10 | CXCR3 |
| CXCL11 | I-TAC | CXCR3 |
| CXCL12 | SDF-1α/β | CXCR4 |
| CXCL13 | BCA-1 | CXCR5 |
| CXCL14 | BRAK | Unknown |
| CXCL15 | Unknown | Unknown |
| CXCL16 | | CXCR6 |
| C chemokines | | |
| XCL1 | Lymphotactin/SCM-1α | XCR1 |
| XCL2 | SCM-1β | XCR1 |
| CX₃C chemokines | | |
| CX₃CL1 | Fractalkine | CX₃CR1 |
| CC chemokines | | |
| CCL1 | I-309 | CCR8 |
| CCL2 | MCP-1 | CCR2 |
| CCL3 | MIP-1α | CCR1, CCR5 |
| CCL3L1 | LD78β | CCR1, CCR5 |
| CCL4 | MIP-1β | CCR5 |
| CCL5 | RANTES | CCR1, CCR3, CCR5 |
| CCL6 | Unknown | Unknown |
| CCL7 | MCP3 | CCR1, CCR2, CCR3 |
| CCL8 | MCP-2 | CCR3, CCR5 |
| CCL9/CCL10 | Unknown | CCR1 |
| CCL11 | Eotaxin | CCR3 |
| CCL12 | Unknown | CCR2 |
| CCL13 | MCP-4 | CCR2, CCR3 |
| CCL14 | HCC-1 | CCR1, CCR5 |
| CCL15 | HCC-2/Lkn-1/MIP-1δ | CCR1, CCR |
| CCL16 | HCC-4/LEC/LCC-1 | CCR1, CCR2 |
| CCL17 | TARC | CCR4 |
| GGL18 | DC-CK1 | Unknown |
| CCL19 | MIP-3β/ELC | CCR7 |
| CCL20 | MIP-3α/LARC | CCR6 |
| CCL21 | 6Ckine/SLC | CCR7 |
| CCL22 | MDC | CCR4 |
| CCL23 | MPIF-1/CKb8 | CCR1 |
| CCL24 | Eotaxin-2 | CCR3 |
| CCL25 | TECK | CCR9 |
| CCL26 | Eotaxin-3 | CCR3 |
| CCL27 | CTACK | CCR10 |
| CCL28 | MEC | CCR3/CCR10 |
| ^{a} The new nomenclature for human cytokines is detailed, Adapted from Ref. 95. | | |
| | | *(for abbreviations see next page)* |

| Abbreviations for Table *5*(tab001cbb) | | |
|---|---|---|
| BCA-1, B-cell-attracting chemokine 1; CTACK, cutaneous T-cell-attracting chemokine; DC-CK1. dendritic-cell-derived CC chemokine 1; ELC, ERL-1-ligand chemokine; ENA-78, epithelial-cell-derived neutrophil attractant 78; GCP, granulocyte chemotactic protein; GRO, growth-related oncogene; HCC, haemofiltrate CC chemokine; IL, interleukin; IP-10. interferon-inducible protein 10; I-TAC, interferon-inducible T-cell alpha chemoattractant: LARC, liver- and activation-regulated chemokine; LEC, liver-expressed chemokine; LCC-1, liver-specific CC chemokine-1; Lkn-1, leukotactin; MCP, monocyte chemoattractant protein; MDC. macrophage-derived chemokine; MEC, mammary-enriched chemokine; Mig, monokine induced by interfero γ; MIP, macrophage inflammatory protein; MPIF, myeloid progenitor inhibitory factor; NAP, neutrophil-activating peptide; PF4, platelet factor 4; RANTES, 'regulated on activation, normally T-cell-expressed and-secreted'; SCM-1α/β, single C motif-1 α/β; SDF, stromal-cell-derived factor; SLC, secondary lymphoid tissue chemokine; TARC, thymus- and activation-regulated chemokine; TECK, thymus-expressed chemokine. | | |

**Table 6. Interleukin Species (IL 1-30)**

| Symbol | Name | Allases | Previous Symbol | Location | Locus Link | PMID1 | Accession ID |
|---|---|---|---|---|---|---|---|
| IL1A | interleukin 1, alpha | II1F1 | IL1 | 2q12-q21 | 3552 | | M28983 |
| IL1B | interleukin 1, beta | IL1F2 | | 2q13-q21 | 3553 | | M15330 |
| IL1F5 | interleukin 1 family, member 5 (delta) | FIL1, IL1HY1, IL-1RP3, IL1L1 | | 2q14 | 26525 | 10625660 | AF201830 |
| IL1F6 | interleukin 1 family, member 6 (epsilon) | FIL1 | | 2q14 | 27179 | 10625660 | AF201831 |
| IL1F7 | interleukin 1 family, member 7 (zeta) | FIL1, IL-1H4, IL-1RP1 | | 2q14 | 27178 | 10625660 | AF201832 |
| IL1F8 | interleukin 1 family, member 8 (eta) | FIL1, IL-1H2 | | 2q14 | 27177 | 10625660 | AF201833 |
| IL1F9 | interleukin 1 family, member 9 . | IL-1H1,IL-1RP2 | | 2q12-q21 | 56300 | 10860666 | AF200492 |
| IL1F10 | interleukin 1 family, member 10 (theta) | FKSG75, FIL1- theta, IL-1HY2 | | 2q13-14.1 | 84639 | 11747621 | AY026753 |
| IL1R1 | interleukin 1 receptor, type I | D2S1473 | IL1R, IL1RA | 2q12 | 3554 | 1833184 | M27492 |
| IL1R2 | interleukin 1 receptor, type II | | IL1RB | 2q12 | 7850 | 10191101 | X59770 |
| IL1RAP | interleukin 1 receptor accessory protein | IL-1RAcP, IL1R3 | | 3q28 | 3556 | 9479509 | AB006537 |
| IL1RAPL1 | interleukin 1 receptor accessory protein-like 1 | OPHN4, TIGIRR-2 | IL1RAPL | Xp22.1-p21.3 | 11141 | 10471494 | AJ243874 |
| IL1RAPL2 | interleukin 1 receptor accessory protein-like 2 | IL-1R9, TIGIRR-1, IL1RAPL-2, IL1R9 | | Xq22 | 26280 | 10757639 | AF181285 |
| IL1RL1 | interleukin 1 receptor-like 1 | ST2, FIT-1, ST2L, ST2V | | 2q12 | 9173 | 1482686 | D12764 |
| "ILIRL2 | interleukin 1 receptor-like 2 | IL1R-rp2 | | 2q12 | 8808 | 8898719 | U49065 |
| IL1RN | interleukin 1 receptor antagonist | IL1RA, ICIL-1 RA, IL1 F3 | | 2q14.2 | 3557 | 1386337 | M55646 |
| IL2 | interleukin 2 | | | 4q26-q27 | 3558 | 3260003 | U25676 |
| IL2RA | interleukin 2 receptor, alpha | | IL2R | 10p15-p14 | 3559 | 3925551 | X01057 |
| IL2RB | interleukin 2 receptor, beta | | | 22q13 | 3560 | | M26062 |
| IL2RG | interleukin 2 receptor, gamma (severe combined immunodeficiency) | | SCIDX1, IMD4 | Xq13 | 3561 | 1631559 | D11086 |
| IL3 | interleukin 3 (colony-stimulating factor, multiple) | | | 5q23-q31 | 3562 | | M14743 |
| IL3RA | interleukin 3 receptor, alpha (low affinity) | | | xp22.3;Yp13.3 | 3563 | 1833064 | M74782 |
| IL4 | interleukin 4 | | | 5q23-q31 | 3565 | | M23442 |
| IL4R | interleukin 4 receptor | | | 16p11.2-12.1 | 3566 | 1679753 | X52425 |
| IL5 | interleukin 5 (colony-stimulating factor, eosinophil) | | | 5q23-q31 | 3567 | | X04688 |
| IL5RA | interleukin 5 receptor, alpha | | IL5R | 3p26-p24 | 3568 | 1732409 | M96652 |
| IL6 | interleukin 6 (interferon, beta 2) | | IFNB2 | 7p21-p15 | 3569 | 3294161 | M18403 |
| IL6R | interleukin 6 receptor | | | 1 | 3570 | | X12830 |
| IL6RL1 | interleukin 6 receptor-like 1 | | | 9 | 3571 | 1889804 | |
| IL6ST | interleukin 6 signal transducer (gp130, oncostatin M receptor) | GP130 | | 5q11 | 3572 | 2261637 | M57230 |
| IL6ST2 | interleukin 6 signal transducer-2 | dJ738P15.3 | | 20p11.2-11.22 | | | AL035252 |
| IL6STP | interleukin 6 signal transducer (gp130, oncostatin M receptor) pseudogene | | | 17p11 | 3573 | | |
| IL7 | interleukin 7 | | | 8q12-q13 | 3574 | | J04156 |
| IL7R | interleukin 7 receptor | | | 5p13 | 3575 | 2317865 | M29696 |
| IL8 | interleukin 8 | SCYB8, LUCT, LECT, MDNCF, TSG-1 | | 4q13-q21 | 3576 | | Y00787 |
| IL8RA | interleukin 8 receptor, alpha | CXCR1, C-C CKR-1 | CMKAR1 | 2q35 | 3577 | 1303245 | U11870 |
| IL8RB | interleukin 8 receptor, beta | CXCR2, CMKAR2 | | 2q35 | 3579 | 1427896 | U11869 |
| IL8RBP | interleukin 8 receptor, beta pseudogene | | | 2q35 | 3580 | 1427896 | |
| IL9 | interleukin 9 | | | 5q31-q35 | 3578 | | S63356 |
| IL9R | interleukin 9 receptor | | | Xq28 orYq12 | 3581 | 1376929 | M84747 |
| IL9RP1 | interleukin 9 receptor pseudogene 1 | | | 9 q34 | 3582 | 8666384 | |
| IL9RP2 | interleukin 9 receptor pseudogene 2 | | | 10p15 | 3583 | 8666384 | |
| IL9RP3 | interleukin 9 receptor pseudogene 3 | | | 16 p13.3 | 3584 | 8666384 | L39062 |
| IL9RP4 | interleukin 9 receptor pseudogene 4 | | | 18 p11.3 | 3585 | 8666384 | |
| IL10 | interleukin 10 | CSIF, TGIF, IL10A | | 1q31-q32 | 3586 | 9162098 | U16720 |
| IL10RA | interleukin 10 receptor, alpha | HIL-10R | IL10R | 1 1q23 | 3587 | 8120391 | U00672 |
| IL10RB | interleukin 10 receptor, beta | CRF2-4 | CRFB4, D21S58, D21S66 | 21q22.1-q22.2 | 3588 | 83145776 | U08988 |
| IL11 | interleukin 11 | | | 19q13.3-q13.4 | 3589 | 1386338 | X58377 |
| IL11RA | interleukin 11 receptor, alpha | | | 9p13 | 3590 | 7670098 | Z38102 |
| IL11RB | interleukin 11 receptor, beta | | | reserved | 3591 | | |
| IL12A | interleukin 12A (natural killer cell stimulatory factor 1, cytotoxic lymphocyte maturation factor 1, p35) | CLMF | NKSF1 | 3p1 2-q13.2 | 3592 | 1673147 | M65271 |
| IL12B | interleukin 128 (natural killer cell stimulatory factor 2, cytotoxic lymphocyte maturation factor 2, p40) | CLMF | NKSF2 | 5q31 .1-q33.1 | 3593 | 1673147 | M65290 |
| IL12R81 | interleukin 12 receptor, beta 1 | | IL12RB | 19p13.1 | 3594 | 9284929 | U03187 |
| IL12RB2 | interleukin 12 receptor, beta 2 | | | 1p31.3-p31.2 | 3595 | 9284929 | U64198 |
| IL13 | interleukin 13 | P600 | | 5q31 | 3596 | | U31120 |
| IL13RA1 | interleukin 13 receptor, alpha 1 | IL-13Ra, NR4 | | Xq24 | 3597 | 8910586 | U62858 |
| IL13RA2 | interleukin 13 receptor, alpha 2 | IL-13R, IL13BP | | Xq1 3.1-q28 | 3598 | 8663118 | X95302 |
| IL14 | interleukin 14 | HMW-BCGF | | 1 p34.3 | 3599 | | L15344 |
| IL15 | interleukin 15 | | | 4q31 | 3600 | 8178155 | U14407 |
| IL15RA | interleukin 15 receptor, alpha | | | 10p15-10p14 | 3601 | 8530383 | U31628 |
| IL15RB | interleukin 15 receptor, beta | | | reserved | 3602 | | |
| IL16 | interleukin 16 (lymphocyte chemoattractant factor) | LCF | | 15q26.3 | 3603 | 9144227 | U82972 |
| IL17 | interleukin 17 (cytotoxic T-lymphocyte-associated serine esterase 8) | IL-17A | CTLA8 | 2q31 | 3605 | 8390535 | U32659 |
| IL17B | interleukin 17B | IL-178, ZCYTO7,IL-20 | | 5q32-34 | 27190 | 10639155 | AF184969 |
| IL17C | interleukin 17C | IL-17C, CX2, IL-21 | | 16q24 | 27189 | 10639155 | AF152099 |
| IL17D | interleukin 17D | IL-22, IL-27 | | 13q11 | 53342 | | AY078238 |
| IL17E | interleukin 17E | IL25, IL-25 | | 14q11.2 | 64806 | 11058597 | AF305200 |
| IL17F | interleukin 17F | IL-17F, ML-1, ML1, IL-24, IL-26 | | 6p12 | 112744 | | AF384857 |
| IL17R | interleukin 17 receptor | hIL-17R | | 22 | 23765 | 9367539 | U58917 |
| IL17RB | interleukin 17 receptor B | IL17RH1, EVI27, CRL4 | IL17BR | 3p21.1 | 55540 | 10749887 | AF212365 |
| IL17RC | interleukin 17 receptor C | IL17-RL | | 3p | | | BC006411 |
| IL17RD | interleukin 17 receptor D | SEF, IL17RLM, FLJ35755, IL-17RD | | 3p | | | AF494208 |
| IL17RE | interleukin 17 receptor E | FLJ23658 | | 3p | | | AF458069 |
| IL18 | interleukin 18 (interferon-gamma-inducing factor) | IGIF, IL1F4, IL-1g | | 11q22.2-q22.3 | 3606 | 7477296 | U90434 |
| IL18BP | interleukin 18 binding protein | | | 11q13 | 10068 | 10023777 | AF110798 |
| IL18R1 | interleukin 18 receptor 1 | IL1RRP, IL-1Rrp | | 2q12 | 8809 | 8626725 | U43672 |
| IL18RAP | interleukin 18 receptor accessory protein | AcPL | | 2p24.3-p24.1 | 8807 | 9792649 | AF077346 |
| IL19 | interleukin 19 | IL-19, MDA1, ZMDA1, IL-10C | | 1q32.2 | 29949 | | AF192498 |
| IL20 | interleukin 20 | ZCYTO10, IL10D | | 1q32 | 50604 | | AF224266 |
| IL20RA | interleukin 20 receptor, alpha | ZCYTOR7 | | 6q23 | 53832 | 10875937 | AF184971 |
| IL20RB | interleukin 20 receptor beta | | | | | | |
| IL21 | interleukin 21 | Za11 | | 4q26-q27 | 59067 | 11081504 | AF254069 |
| IL21R | interleukin 21 receptor | | | 16p11 | 50615 | 11081504 | AF254067 |
| IL22 | interleukin 22 | LTIF,IL-21, zcyto18, IL-TIF, IL-D110, TIFa, TIFIL-23 | | 12q15 | 50616 | 10954742 | AF279437 |
| IL22RA1 | Interleukin 22 receptor alpha 1 | CRF2-9 | IL22R | 1p86.11 | 58985 | 10875937 | AF286095 |
| IL22RA2 | interleukin 22 receptor alpha 2 | CRF2-S1, IL-22BP | | 6q24.1-q24.2 | | | |
| IL23A | interleukin 23, alpha subunit p19 | SGRF, IL23P19, IL-23 | | 12 | 51561 | 11114383 | AB030000 |
| IL24 | interleukin 24 | mda-7, IL10B, Mob-5, C49A, FISP | ST16 | 1q32 | 11009 | 8545104 | U16261 |
| IL25 | This symbol will not be used | | | | | | |
| IL26 | interleukin 26 | AK155 | | 12q15 | 55801 | 10729163 | AJ251549 |
| IL27 | This symbol will not be used | | | | | | |
| IL28A | interleukin 28A (interferon, lambda 2) | IL-28A, IFNL2 | | 19q13.13 | | | AY129148 |
| IL28B | interleukin 28B (interferon, lambda 3) | IL-28B, IFNL3 | | 19q13.13 | | | AY129149 |
| IL28RA | interleukin 28 receptor, alpha (interferon, lambda receptor) | CRF2/12, IFNLR | | 1p36.11 | | | AY129153 |
| IL29 | interleukin 29 (interferon, lambda 1) | IL-29, IFNL1 | | 19q13.13 | | | AY129150 |
| IL30 | interleukin 30 | IL-27, p28 | | 16p11 | 246778 | 12121660 | AY099296 |

While the invention has been described with respect to specific examples including presently preferred modes of carrying out the invention, those skilled in the art will appreciate that there are numerous variations and permutations of the above described systems and techniques that fall within the spirit and scope of the invention.
Each of the following U.S. Provisional Applications is incorporated herein by reference in their entirety: 60/599,717, filed August 5, 2004; 60/599,592, filed August 5, 2004; 60/600,850, filed August 11, 2004; 60/603,001, filed August 19,1 2004; 60,603,867, filed August 23, 2004; 60/612,070, filed September 21, 2004; 60/582,654), filed June 24, 2004; 60/614,963, filed September 21, 2004; and 60/590,459, filed July 22, 2004.

### INCORPORATION BY REFERENCE

The contents of all of the patents, patent applications and journal articles cited throughout this document are incorporated by reference as if set forth fully herein.

### Additional Embodiments

The invention comprises the following embodiments:
1. A SMIP of formula (IV): 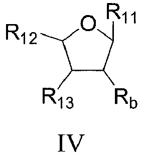 wherein,
   R₁₁ is selected from the group consisting of hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted hydroxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted arylamino, substituted and unsubstituted heteroarylamino, substituted and unsubstituted heterocyclylamino, and substituted and unsubstituted carbocyclylamino;
   R₁₂ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
   R₁₃ is selected from the group consisting of H, hydroxyl, alkoxy, substituted and unsubstituted alkyl, substituted and unsubstituted aryl, substituted and unsubstituted heterocyclyl and substituted and unsubstituted heteroaryl; or
   R₁₂ and R₁₃ are bound together to form a substituted or unsubstituted heterocyclyl group; and
   R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; for use in eliciting an immune response in a patient.
2. The SMIP according to embodiment 1 wherein R₁₂ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl; R₁₃ is selected from the group consisting of H, substituted and unsubstituted alkyl, substituted and unsubstituted aryl, substituted and unsubstituted heterocyclyl and substituted and unsubstituted heteroaryl; and R₁₁ is further defined as structure of formula (R₁₁ₐ): 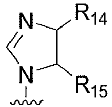 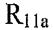 wherein,
   R₁₄ and R₁₅ are independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; or
   R₁₄ and R₁₅ are taken together to form a substituted or unsubstituted heteroaryl group.
3. The SMIP according to embodiment 2 wherein R₁₄ and R₁₅ are taken together to form a substituted heteroaryl group such that R₁₁ₐ is a DNA base.
4. The SMIP according to embodiment 3 wherein said DNA base is adenine.
5. The SMIP according to embodiment 2 wherein R₁₄ is aminocarbonyl.
6. The SMIP according to embodiment 2 wherein R₁₄ is hydroxy.
7. The SMIP according to embodiment 1 wherein R₁₂ and R₁₃ are bound together to form a heterocyclyl group as shown in Figure (IVₐ): 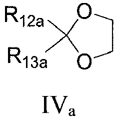 wherein,
   R₁₂ₐ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
   R₁₃ₐ is selected from the group consisting of H, substituted and unsubstituted alkyl, substituted and unsubstituted aryl, substituted and unsubstituted heterocyclyl and substituted and unsubstituted heteroaryl.
8. The SMIP as in any of embodiments 1-7, wherein said SMIP is administered in a dose capable of increasing TNF-α levels.
9. The SMIP as in any of embodiments 1-7, wherein said SMIP modulates activity of at least one target selected from the group consisting of glucocortocoid receptors, DNA alkylation, calcineurin, JNK, p38 kinase, cyclin kinase cascade, PDE IV, IMPDH, DHOD, lck, and thymidylate synthase.
10. The SMIP as in any of embodiments 1-7, wherein said immune response involves production of cytokines.
11. The SMIP as in any of embodiments 1-7, wherein said immune response involves increased production of TNF-α.
12. The SMIP as in any of embodiments 1-7, wherein the patient is suffering from a viral infection.
13. The SMIP according to embodiment 12 wherein said viral infection is HCV.
14. The SMIP as in any of embodiments 1-7, wherein said patient is suffering from increased cellular proliferation or cancer.
15. The SMIP as in any of embodiments 1-7, wherein said patient is suffering from allergic diseases.
16. The SMIP as in any of embodiments 1-7, wherein said patient is suffering from asthma.
17. The SMIP according to any of embodiments 12, 13 or 16 wherein said SMIP is co-administered with another agent.
18. The SMIP as in embodiment 17 wherein the other agent is a vaccine.
19. The SMIP according to embodiment 13 wherein said SMIP is co-administered with another agent.
20. The SMIP according to embodiment 19 wherein the other agent is a vaccine.
21. The SMIP according to embodiment 14 wherein said SMIP is co-administered with another agent.
22. The SMIP according to embodiment 15 wherein said SMIP is co-administered with another agent.
23. The SMIP according to embodiment 16 wherein said SMIP is co-administered with another agent.
24. The SMIP as in any of embodiments 1-7 wherein said SMIP is co-administered with another agent.
25. SMIP according to embodiment 16 wherein the other agent is a vaccine
26. The SMIP as in any of embodiments 1-7 wherein said SMIP, present at a concentration less than 20uM, induces production of TNF-α.
27. A high throughput assay for identifying small molecule immuno modulators, said assay comprising:
   a) contacting a plurality of test compounds with cells to form one or more test solution(s);
   b) incubating said test solution for at least 30 minutes;
   c) measuring for an increased level of one or more immunological markers in said test solution;
   wherein immunomodulation by one or more test compounds present in said plurality of test compounds causes an increase in the amount of said immunological markers in said test solution.
28. The high throughput assay of embodiment 27 wherein said small molecule immuno- modulators are SMIPs and said immunomodulation is immunopotentiation.
29. The high throughput assay of embodiment 28, further comprising the step of comparing said amount of immunological markers in said test solution with an unstimulated solution, devoid of any test compounds.
30. The high throughput assay of embodiment 29, wherein said unstimulated solution is run in parallel with said test solution.
31. The high throughput assay of embodiment 28 further comprising the step of comparing said amount of immunological markers in said test solution with a stimulated solution containing a known immunopotentiating agent.
32. The high throughput assay of embodiment 31, wherein said stimulated solution is run in parallel with said test solution.
33. The high throughput assay of embodiment 31, wherein said immunopotentiating agent is selected from the group consisting of LPS, CpG, resiquimod, Poly I:C (dsRNA), Pam3-Cys, MPL, and anti-CD3.
34. The high throughput assay of embodiment 28, wherein said immunological markers are cytokines.
35. The high throughput assay of embodiment 28, wherein said immunological markers are chemokines.
36. The high throughput assay of embodiment 28, wherein said immunological markers are growth factors.
37. The high throughput assay of embodiment 28, wherein said immunological markers are both cytokines and chemokines.
38. The high throughput assay of embodiment 28, wherein said immunological markers are both cytokines and growth factors.
39. The high throughput assay of embodiment 28, wherein said immunological markers are both chemokines and growth factors.
40. The high throughput assay of embodiment 28, wherein said immunological markers are cytokines, chemokines and growth factors.
41. The high throughput assay of embodiment 26 wherein said immunological marker is TGF-beta, said small molecule immunomodulators are SMIS, and said immunomodulation is immunosuppression.

## Claims

1. A SMIP compound of formula (XIX): 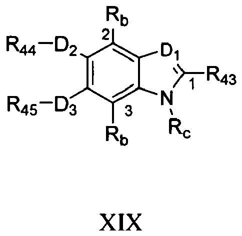 D₁ is -N= or -C(R₄₆)=;
D₂ and D₃ are independently selected from the group consisting of a covalent bond, -O-, -S-, -NH-, and -NR_{d}-;
R₄₃ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted aralkylsulfonyl, substituted and unsubstituted heteroaralkylsulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R₄₄ and R₄₅ are independently selected from the group consisting of H, halogen, cyano, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl groups;
R₄₆ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, CH=N-N-CO-Rₐ, -CH₂CHRₐCOR_{b}, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl;
R_{c} is selected from the group consisting of H, substituted and unsubstituted alkyl, substituted and unsubstituted aryl, substituted and unsubstituted heterocyclyl, and substituted and unsubstituted heteroaryl;
R_{d} is selected from the group consisting of H, carboxylic acid, substituted and unsubstituted alkyl, substituted and unsubstituted alkenyl, substituted and unsubstituted alkynyl, substituted and unsubstituted alkoxycarbonyl, substituted and unsubstituted aminocarbonyl, substituted and unsubstituted sulfonyl, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is 1, 2, or 3;
with the proviso that if R₄₃ is amino, alkylamino, carbonylamino, or alkylcarbonylamino, then both D₂ and D₃ are covalent bonds;
a SMIP compound of formula (II): 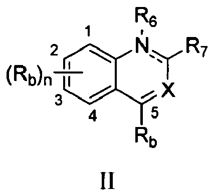 wherein,
a dotted line represents an optional placement of an additional bond;
R₆ is H, substituted alkyl or unsubstituted alkyl; R₇ is =O; and the dotted line is absent; or
R₆ is absent; R₇ is selected from the group consisting of H, hydroxy, amino, nitro, cyano, carboxylic acid, substituted and unsubstituted alkyl, substituted and unsubstituted alkenyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, and substituted and unsubstituted carbocyclylalkyl; and the dotted line represents the placement of an additional bond;
X is =N- or =C(R₈)-, wherein R₈ is selected from the group consisting of H, hydroxy, amino, nitro, cyano, carboxylic acid, substituted and unsubstituted alkyl, substituted and unsubstituted alkenyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, and substituted and unsubstituted carbocyclylalkyl;
R_{b} is independently selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl; and
n is 1, 2, or 3; or
a SMIP compound of formula (IV): 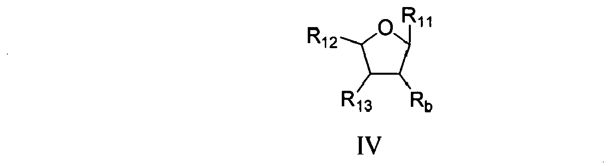 wherein,
R₁₁ is selected from the group consisting of hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted hydroxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted arylamino, substituted and unsubstituted heteroarylamino, substituted and unsubstituted heterocyclylamino, and substituted and unsubstituted carbocyclylamino;
R₁₂ is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, and substituted and unsubstituted alkoxyalkyl; and
R₁₃ is selected from the group consisting of H, hydroxyl, alkoxy, substituted and unsubstituted alkyl, substituted and unsubstituted aryl, substituted and unsubstituted heterocyclyl and substituted and unsubstituted heteroaryl; or R₁₂ and R₁₃ are bound together to form a substituted or unsubstituted heterocyclyl group; and
R_{b} is selected from the group consisting of H, halogen, hydroxy, amino, nitro, cyano, carboxylic acid, substituted and unsubstituted alkyl, substituted and unsubstituted alkenyl, substituted and unsubstituted alkynyl, substituted and unsubstituted alkylamino, substituted and unsubstituted carbonyloxy, substituted and unsubstituted alkoxycarbonyl, substituted and unsubstituted aminocarbonyl, substituted and unsubstituted carbonylamino, substituted and unsubstituted sulfonyl, substituted and unsubstituted alkoxy, substituted and unsubstituted alkoxyalkyl, substituted and unsubstituted carbocyclyl, substituted and unsubstituted heterocyclyl, substituted and unsubstituted aryl, substituted and unsubstituted heteroaryl, substituted and unsubstituted aralkyl, substituted and unsubstituted heteroaralkyl, substituted and unsubstituted heterocyclylalkyl, substituted and unsubstituted carbocyclylalkyl, substituted and unsubstituted aralkenyl, substituted and unsubstituted heteroaralkenyl, substituted and unsubstituted heterocyclylalkenyl, and substituted and unsubstituted carbocyclylalkenyl
for use in eliciting an immune response in a patient.

2. The SMIP, compound as claimed in claim 1, wherein the SMIP compound is of formula (XIX).

3. The SMIP compound as claimed in claim 1, wherein the SMIP compound is of formula (II).

4. The SMIP compound as claimed in claim 1, wherein the SMIP compound is of formula (IV).

5. The SMIP compound as claimed in any one of claims 1 to 4, wherein the use comprises the simultaneous separate or sequential administration of the SMIP compound and an antigen.

6. An immunogenic composition comprising a SMIP compound of formula (XIX), (II), or (IV) as defined in claim 1, and an antigen.

7. The immunogenic composition of claim 6, wherein the SMIP compound is of formula (XIX).

8. The immunogenic composition of claim 6, wherein the SMIP compound is of formula (II).

9. The immunogenic composition of claim 6, wherein the SMIP compound is of formula (IV).

10. The use of a SMIP compound of formula (XIX), (II), or (IV) as defined in claim 1, for the manufacture of a medicament for eliciting an immune response in a patient.

11. The use of claim 10, wherein the SMIP compound is of formula (XIX).

12. The use of claim 10, wherein the SMIP compound is of formula (II).

13. The use of claim 10, wherein the SMIP compound is of formula (IV).

14. The use of any one of claims 10 to 13, wherein the medicament is adapted for co-administration with an antigen.
